# EUROPEAN PATENT APPLICATION

(11) **EP 4 778 575 A2**
(43) Date of publication of application: **22.07.2026**
(21) Application number: 26161618.9
(22) Date of filing: 04.02.2021
(51) Int. Cl.: A61P 7/04

(54) **TREATMENT OF MENORRHAGIA IN PATIENTS WITH SEVERE VON WILLEBRAND DISEASE BY ADMINISTRATION OF RECOMBINANT VWF**

(30) Priority: 04.02.2020 US 202062969998 P
(62) Divisional of application: 21708526.5
(71) Applicant: Takeda Pharmaceutical Company Limited, Osaka-shi, Osaka 541-0045 (JP)
(72) Inventor: Ploder, Bettina, 1220 Vienna (AT); Truong-Berthoz, Françoise, 8152 Glattpark (Opfikon) (CH)
(74) Representative: Hoffmann Eitle

(57) **Abstract**

The present invention relates to a method for treatment of menorrhagia in a subject with von Willebrand Disease (VWD) comprising administering a therapeutic amount of recombinant von Willebrand Factor (rVWF) to the subject.

## Description

### CROSS-REFERENCE TO RELATED APPLICATIONS

This application claims priority to U.S. Provisional Patent Application No. 62/969,998, filed on February 4, 2020, which is hereby incorporated by reference in its entirety.

### REFERENCE TO SEQUENCE LISTING

The Sequence Listing text copy submitted herewith was created on February 3, 2021, is entitled 008073-5215-WO_Sequence_Listing.txt, is 53,961 bytes in size and is herein incorporated by reference in its entirety.

### BACKGROUND OF THE INVENTION

Coagulation diseases, such as von Willebrand Disease (VWD), generally result from a deficiency in the coagulation cascade. von Willebrand Disease (VWD) refers to the group of diseases caused by a deficiency of von Willebrand factor. Von Willebrand factor (VWF) helps blood platelets clump together and stick to the blood vessel wall, which is necessary for normal blood clotting.

von Willebrand disease (VWD) is the most common inherited bleeding disorder, with an estimated prevalence rate of 1% (Veyradier A, et al., Medicine (Baltimore). 2016, 95(11):e3038). However, excluding milder forms of the disease, only about 1/10,000 patients actually require treatment. Current treatment for these coagulopathies includes a replacement therapy using pharmaceutical preparations comprising the normal coagulation factor.

VWF is a glycoprotein circulating in plasma as a series of multimers ranging in size from about 500 to 20,000 kD. The full length of cDNA of VWF has been cloned; the propolypeptide corresponds to amino acid residues 23 to 764 of the full length prepro-VWF (Eikenboom et al (1995) Haemophilia 1, 77 90). Multimeric forms of VWF are composed of 250 kD polypeptide subunits linked together by disulfide bonds. VWF mediates the initial platelet adhesion to the sub-endothelium of the damaged vessel wall, with the larger multimers exhibiting enhanced hemostatic activity. Multimerized VWF binds to the platelet surface glycoprotein Gp1bα, through an interaction in the A1 domain of VWF, facilitating platelet adhesion. Other sites on VWF mediate binding to the blood vessel wall. Thus, VWF forms a bridge between the platelet and the vessel wall that is essential to platelet adhesion and primary hemostasis under conditions of high shear stress. Normally, endothelial cells secrete large polymeric forms of VWF and those forms of VWF that have a lower molecular weight arise from proteolytic cleavage. The multimers of exceptionally large molecular masses are stored in the Weibel-Pallade bodies of the endothelial cells and liberated upon stimulation by agonists such as thrombin and histamine.

For patients with VWD, it is recommended that they be treated with VWF replacement given the need for prolonged hemostasis, particularly in major surgery (Mannucci PM and Franchini M., Haemophilia, 2017, 23(2):182-187; National Institutes of Health. National Heart, Lung, and Blood Institute. The Diagnosis, Evaluation, and Management of von Willebrand Disease NIH Publication No. 08-5832; December, 2007). Plasma-derived VWF (pdVWF) therapies contain factor VIII (FVIII) and have the potential for FVIII accumulation with repeated dosing. The recombinant VWF (rVWF) concentrate (also known as VONVENDI^{®}, VEYVONDI^{®}, vonicog alfa, Takeda Pharmaceutical Company Limited) provides improved clinical benefits compared to plasma-derived VWF therapies (Turecek PL, et al. Hamostaseologie. 2009;29(suppl 1):S32-38; Mannucci PM, et al. Blood, 2013;122(5):648-657; Gill JC, et al. Blood, 2015;126(17):2038-2046).

The most common symptom in women with VWD is heavy menstrual bleeding (HMB) or menorrhagia, which affects up to 80% of women with VWD, with 20% of women with moderate or severe VWD requiring hysterectomy predominantly because of HMB. Menorrhagia/HMB is commonly associated with symptomatic iron deficiency anemia, psychological stress, and reduced quality of life. It can adversely affect work, school, and daily activities, and result in increased health care costs.

Lack of diagnosis and effective treatment for menorrhagia is an unmet health need in women with VWD. rVWF has demonstrated hemostatic efficacy and a positive benefit-risk profile when used for the on-demand treatment of bleeding episodes (BEs) in adult patients with severe VWD. However, limited information exists specifically on the management of menorrhagia with rVWF. Accordingly, there is a need for improved methods for treating women with severe VWD using rVWF.

### BRIEF SUMMARY OF THE INVENTION

The present disclosure provides a method for treating a menorrhagia bleeding episode in a subject with severe von Willebrand Disease (VWD) comprising administering to the subject at least one dose of recombinant von Willebrand Factor (rVWF) ranging from about 30 IU/kg to about 90 IU/kg.

In some embodiments, the menorrhagia bleeding episode is selected from the group consisting of a minor menorrhagia bleeding episode and a major menorrhagia bleeding episode.

In some embodiments, the VWD is selected from a group consisting of Type 1 VWD, Type 2 VWD, and Type 3 VWD.

In some embodiments, a first dose of 30-60 IU/kg rVWF is administered to the subject and the menorrhagia bleeding episode is a minor menorrhagia bleeding episode. In some embodiments, the method further comprises administering to the subject a second dose of 30-60 IU/kg rVWF.

In some embodiments, a first dose of 40-50 IU/kg rVWF is administered to the subject, and the menorrhagia bleeding episode is a minor menorrhagia bleeding episode. In some embodiments, the method further comprises administering to the subject a second dose of 40-50 IU/kg rVWF.

In some embodiments, the VWD is Type 1 VWD. In some embodiments, the VWD is selected from the group consisting of Type 2A, Type 2B, Type 2N, Type 2M VWD, and Type 3 VWD.

In some embodiments, the first dose of 40-90 IU/kg rVWF is administered to the subject, and the menorrhagia bleeding episode is a major menorrhagia bleeding episode. In some embodiments, the VWD is Type 1 VWD. In some embodiments, the VWD is selected from the group consisting of Type 2A VWD, Type 2B VWD, Type 2N VWD, Type 2M VWD, and Type 3 VWD.

In some embodiments, the first dose of 40-90 IU/kg rVWF is administered to the subject and the VWD is Type 1 VWD. In some embodiments, the first dose of 50-90 IU/kg VWF is administered to the subject and the VWD is selected from the group consisting of Type 2A VWD, Type 2B VWD, Type 2N VWD, Type 2M VWD, and Type 3 VWD.

In some embodiments, the method described further comprises administering to the subject a plurality of doses comprising a first dose of 40-90 IU/kg rVWF. In some embodiments, each dose is administered every 8 to 12 hours for about 3 days. In some embodiments, the method further comprises administering to the subject a plurality of doses comprising the first dose of 40-90 IU/kg rVWF and a plurality of second doses, wherein each of the second doses is administered every 8 to 12 hours for about 3 days.

In some embodiments, the method further comprises administering to the subject a plurality of doses comprising the first dose of 40-90 IU/kg rVWF and a plurality of second doses, wherein each of the second doses is 40-60 IU/kg rVWF and administered every 8 to 12 hours for about 3 days.

In some embodiments, the method further comprises administering to the subject a plurality of doses comprising the first dose of 40-90 IU/kg rVWF and a second dose of 40-60 IU/kg rVWF. In some embodiments, the method further comprises administering to the subject a third dose of 30-70 IU/kg rVWF daily for the remaining duration of the bleeding episode. In some embodiments, the total duration of the method is no more than about 7 days.

In some embodiments, a first dose of rVWF administered to the subject is 50-80 IU/kg rVWF, and the menorrhagia bleeding episode is a major menorrhagia bleeding episode.

In some embodiments, the VWD is Type 1 VWD. In some embodiments, the method further comprises administering to the subject a plurality of doses of rVWF. In some embodiments, the method described further comprises administering to the subject a plurality of doses comprising the first dose of 50-80 IU/kg rVWF and a plurality of second doses, wherein each of the second doses is administered every 8 to 12 hours for about 3 days. In some embodiments, the method further comprises administering to the subject a plurality of doses comprising the first dose of 50-80 IU/kg rVWF and a plurality of second doses, wherein each of the second doses is 40-60 IU/kg rVWF and administered every 8 to 12 hours for about 3 days. In some embodiments, the method further comprises administering to the subject a third dose of 30-70 IU/kg rVWF daily for the remaining duration of the bleeding episode. In some embodiments, the total duration of the method is no more than about 7 days.

In some embodiments, the first dose of rVWF administered to the subject is 60-80 IU/kg, and the VWD is selected from the group consisting of Type 2A VWD, Type 2B VWD, Type 2N VWD, Type 2M VWD, and Type 3 VWD. In some embodiments, the method further comprises administering to the subject a plurality of doses of rVWF. In some embodiments, the method further comprises administering to the subject a plurality of doses comprising the first dose of 60-80 IU/kg rVWF and a plurality of second doses, wherein each of the second doses is administered every 8 to 12 hours for about 3 days. In some embodiments, the method further comprises administering to the subject a plurality of doses comprising the first dose of 60-80 IU/kg rVWF and a plurality of second doses, wherein each of the second doses is 40-80 IU/kg rVWF and administered every 8 to 12 hours for about 3 days. In some embodiments, the method further comprises administering to the subject a plurality of doses comprising a first dose of 60-80 IU/kg rVWF and a second dose of 40-80 IU/kg rVWF. In some embodiments, the method further comprises administering to the subject a third dose of 40-60 IU/kg rVWF daily for the remaining duration of the bleeding episode. In some embodiments, the total duration of the method is no more than about 7 days.

In some embodiments, the method further comprises administering recombinant Factor VIII (rFVIII) to the subject. In some embodiments, the rFVIII is administered concomitantly or sequentially with one or more doses of rVWF.

In some embodiments, the rFVIII is administered concomitantly or sequentially with the first dose and/or the second dose of rVWF. In some embodiments, the rFVIII is administered concomitantly or sequentially with each dose of rVWF. In some embodiments, the rFVIII is administered to the subject at a dose of about 20 IU/kg to about 50 IU/kg.

In some embodiments, the rFVIII is administered to the subject such that said rVWF to FVIII ratio is selected from the group consisting of about 1.5:0.8; about 1.3:1; about 1.1:0.8; about 1.5:1; and about 1.1:1.2.

In one aspect, provided is a method for treating a major menorrhagia bleeding episode in a subject with severe Type 1 von Willebrand Disease (VWD) comprising: (a) administering a first dose of 50-75 IU/kg rVWF to the subject; (b) administering a plurality of second doses of 40-60 IU/kg rVWF to the subject, wherein each of the doses is administered every 8-12 hours for 3 days such that a trough level of VWF:RCo is maintained at at least 0.30 IU/mL or a trough level of VWF:GPIbM or VWF:GPIbR is maintained that is equivalent to the trough level of VWF:RCo of at least 0.30 IU/mL; and (c) administering a third dose of 40-60 IU/kg rVWF to the subject once a day for the remaining duration of the bleeding episode; wherein the total duration of steps (a)-(c) is no more than about 7 days.

In some embodiments, the trough level of VWF:RCo is maintained at at least 0.40 IU/mL or the trough level of VWF:GPIbM or VWF:GPIbR is maintained that is equivalent to the trough level of VWF:RCo of at least 0.40 IU/mL.

In some embodiments, the trough level of VWF:RCo is maintained at at least 0.50 IU/mL or the trough level of VWF:GPIbM or VWF:GPIbR is maintained that is equivalent to the trough level of VWF:RCo of at least 0.50 IU/mL.

In some embodiments, the total duration of steps (a)-(c) is no more than 6 days. In some embodiments, the total duration of steps (a)-(c) is no more than 5 days. In some embodiments, the total duration of steps (a)-(c) is no more than 4 days.

In some aspects, provided is a method for treating a major menorrhagia bleeding episode in a subject with severe Type 2 or Type 3 von Willebrand Disease (VWD) comprising: (a) administering a first dose of 60-80 IU/kg rVWF to the subject; (b) administering a plurality of second doses of 40-60 IU/kg rVWF to the subject, wherein each of the doses is administered every 8-12 hours for 3 days such that a trough level of VWF:RCo is maintained at at least 0.30 IU/mL or a trough level of VWF:GPIbM or VWF:GPIbR is maintained that is equivalent to the trough level of VWF:RCo of at least 0.30 IU/mL; and (c) administering a third dose of 40-60 IU/kg rVWF to the subject once a day for the remaining duration of the bleeding episode; wherein the total duration of steps (a)-(c) is no more than about 7 days.

In some embodiments, the trough level of VWF:RCo is maintained at at least 0.40 IU/mL or the trough level of VWF:GPIbM or VWF:GPIbR that is equivalent to the trough level of VWF:RCo of at least 0.40 IU/mL is maintained. In some embodiments, the trough level of VWF:RCo is maintained at at least 0.50 IU/mL or the trough level of VWF:GPIbM or VWF:GPIbR is maintained that is equivalent to the trough level of VWF:RCo of at least 0.50 IU/mL.

In some embodiments, the total duration of steps (a)-(c) is no more than 6 days. In some embodiments, the total duration of steps (a)-(c) is no more than 5 days. In some embodiments, the total duration of steps (a)-(c) is no more than 4 days.

In some embodiments, the method further comprises administering recombinant Factor VIII (rFVIII) to the subject. In some embodiments, the rFVIII is administered concomitantly or sequentially with the at least one dose of rVWF. In some embodiments, the rFVIII is administered concomitantly or sequentially with the first dose and/or the plurality of second doses of rVWF. In some embodiments, the rFVIII is administered concomitantly or sequentially with both the first dose and the plurality of second doses of rVWF. In some embodiments, the dose of rFVIII administered to v subject is about 20 IU/kg to about 50 IU/kg.

In some embodiments, the rFVIII is administered to the subject such that the rVWF to FVIII ratio is selected from the group consisting of about 1.5:0.8; about 1.3:1; about 1.1:0.8; about 1.5:1; and about 1.1:1.2. In some embodiments, the rVWF to FVIII ratio is about 1.5:0.8. In some embodiments, the rVWF to FVIII ratio is about 1.3:1. In some embodiments, the rVWF to FVIII ratio is about 1.1:0.8. In some embodiments, the rVWF to FVIII ratio is about 1.5:1. In some embodiments, the rVWF to FVIII ratio is about 1.1:1.2.

In some embodiments, the rVWF has a specific activity of between about 20 mU/µg to about 150 mU/µg. In some embodiments, the rVWF has a specific activity of between about 20 IU/mg protein to about 150 IU/mg protein. In some embodiments, the rVWF has a specific activity of between about 20 IU rVWF:RCo/mg protein to about 150 IU rVWF:RCo /mg protein, or equivalents thereof as determined using another VWF activity measurement.

In some embodiments, the rFVIII and rVWF is administered to the subject such that rFVIII procoagulant activity (IU rFVIII:C) to rVWF Ristocetin cofactor activity (IU rVWF:RCo) is at a ratio of between about 3:1 and about 1:5.

Also, provided herein is a method for treating a menorrhagia bleeding episode in a subject with severe von Willebrand Disease (VWD) comprising administering to the subject at least one dose of recombinant von Willebrand Factor (rVWF) ranging from about 30 IU/kg to about 90 IU/kg, wherein the duration and/or the severity of the bleeding episode suffered by the subject is reduced compared to the duration and/or the severity of the bleeding episode suffered by the subject when the rVWF is not administered.

In some embodiments of any of the methods described, one dose of rVWF is administered over the duration of the bleeding episode. In some embodiments, two doses of rVWF are administered over the duration of the bleeding episode. In some embodiments, three doses of rVWF are administered over the duration of the bleeding episode. In many embodiments, four doses of rVWF are administered over the duration of the bleeding episode. In other embodiments, five or more doses of rVWF are administered over the duration of the bleeding episode.

In some embodiments, a range of about 20 IU/kg to about 200 IU/kg of rVWF is administered to the subject over the duration of the bleeding episode. In many embodiments, a range of about 40 IU/kg to about 200 IU/kg of rVWF is administered to the subject over the duration of the bleeding episode. In some embodiments, a range of about 20 IU/kg to about 100 IU/kg of rFVIII is administered to the subject over the duration of the bleeding episode.

In many embodiments, the rVWF outlined herein has an amino acid sequence having at least 90% identity to the sequence of SEQ ID NO:3. In some embodiments, the rVWF has an amino acid sequence having at least 80%, 85%, 90%, 91%, 92%, 93%, 94%, 95%, 96%, 97%, 98%, 99%, or 100% identity to the sequence of SEQ ID NO:3. In many embodiments, the rVWF has an amino acid sequence having at least 90% identity to the sequence of SEQ ID NO:3 and the amino acid sequence is encoded by a nucleic acid sequence having at least 80% sequence identity to SEQ ID NO:1 or a fragment thereof.

In many embodiments, the rVWF described is a fragment of an rVWF, wherein the rVWF has an amino acid sequence having at least 90%, identity to the sequence of SEQ ID NO:3. In some embodiments, the rVWF is a fragment of an rVWF, wherein the rVWF has an amino acid sequence having at least 80%, 85%, 90%, 91%, 92%, 93%, 94%, 95%, 96%, 97%, 98%, 99%, or 100% identity to the sequence of SEQ ID NO:3.

In some embodiments, the rVWF is chemically modified using one or more techniques from the group consisting of ubiquitination, glycosylation, conjugation to therapeutic or diagnostic agents, labeling, covalent polymer attachment, introduction of non-hydrolyzable bonds, and insertion or substitution by chemical synthesis of one or more amino acids.

### BRIEF DESCRIPTION OF THE DRAWINGS

Figure 1 depicts the overall process of a Phase 3 open-label study of rVWF in patients with severe VWD.
Figure 2 depicts the rVWF dosing recommendations for treatment of bleeding events (BEs) during the Phase 3 open-label study of rVWF. The rVWF dosage was measured as IU VWF:RCo/kg body weight.
Figure 3 depicts the overall criteria used by investigators in rating the clinical efficacy of rVWF treatment for BEs.
Figure 4 depicts the demographic characteristics of patients with menorrhagia that were treated with rVWF in the study.
Figure 5 depicts the percentage of menorrhagia episodes that were mild, moderate or severe, as a percent of a total number of 45 menorrhagia episodes treated with rVWF only (*e.g*., not plasma-derived VWF).
Figure 6 shows the summary of rVWF treatment for 45 menorrhagia episodes.
Figure 7 shows the clinical efficacy ratings for rVWF treatment determined by investigators. The ratings were based on a total number of 45 menorrhagia episodes that were treated with rVWF only (*i.e*., not plasma-derived VWF).
Figure 8 shows the adverse events (AEs) that were reported during the study of menorrhagia patients under medications concomitant to rVWF. Each AE was reported by 1 patient.

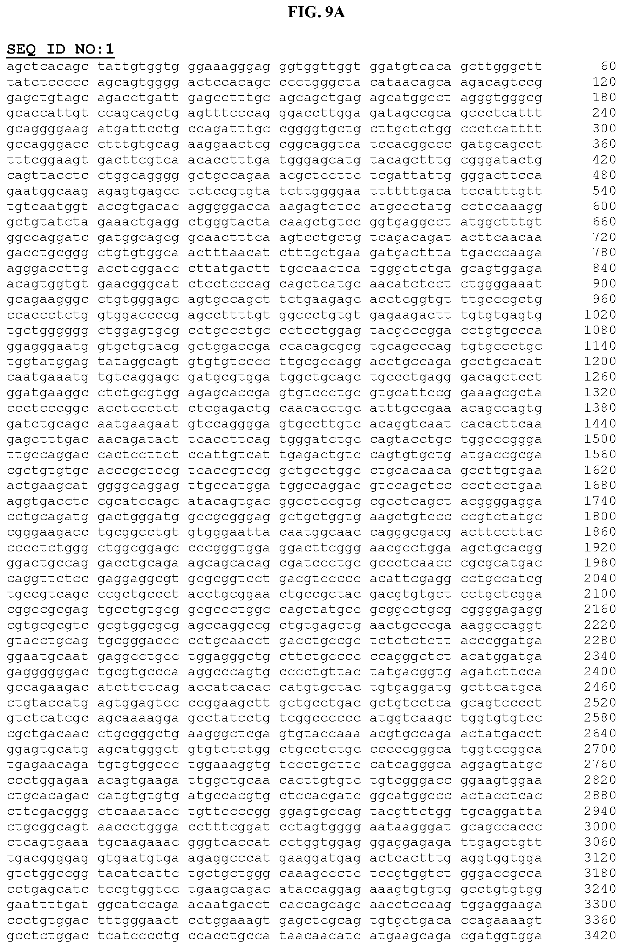

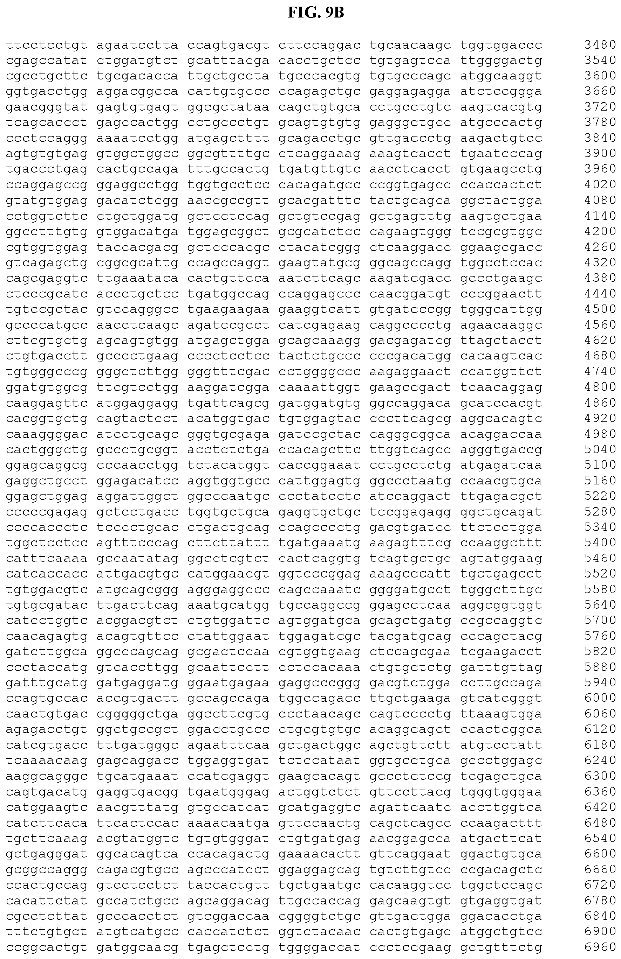

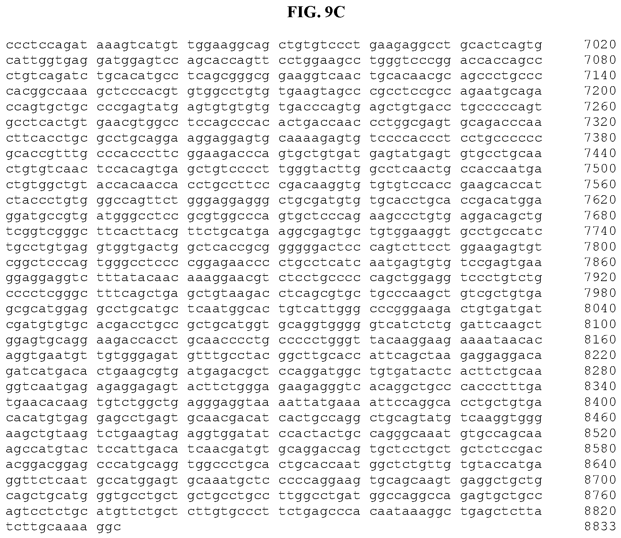

Figure 9A-C depicts the nucleic acid sequence of prepro-VWF.

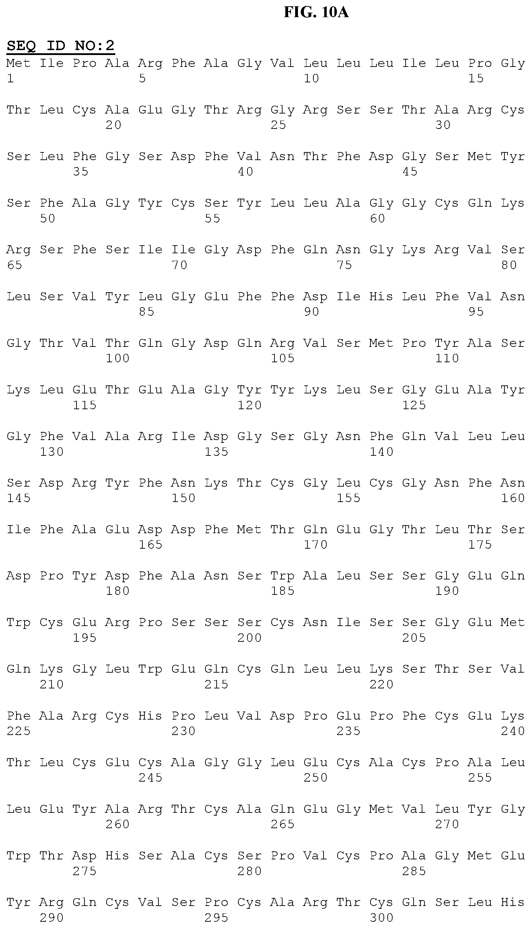

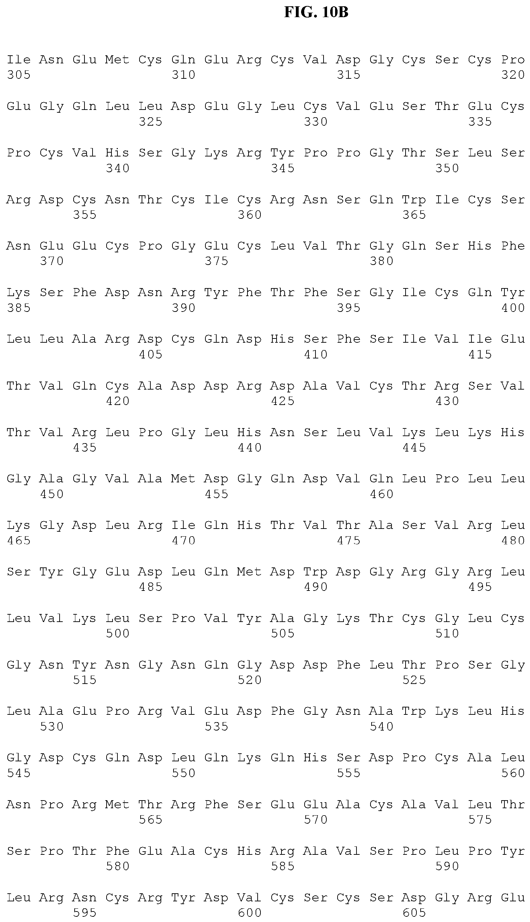

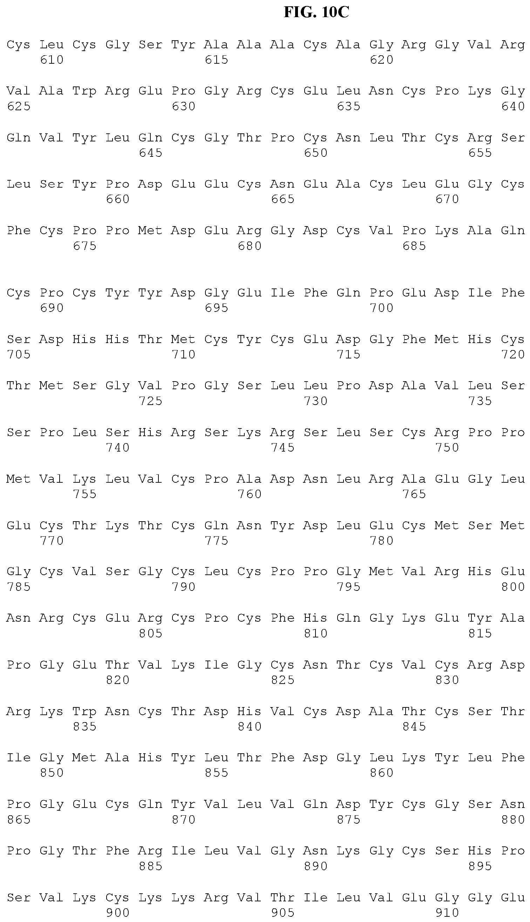

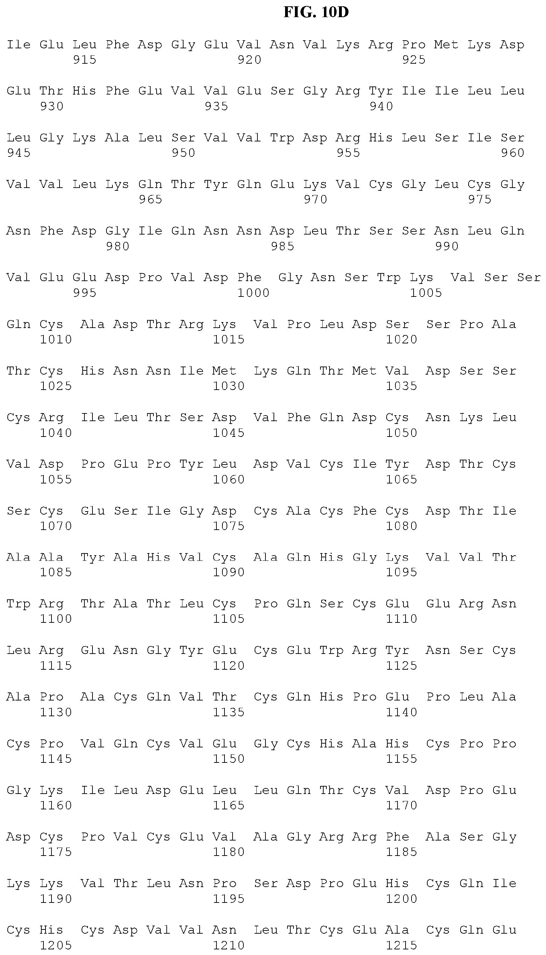

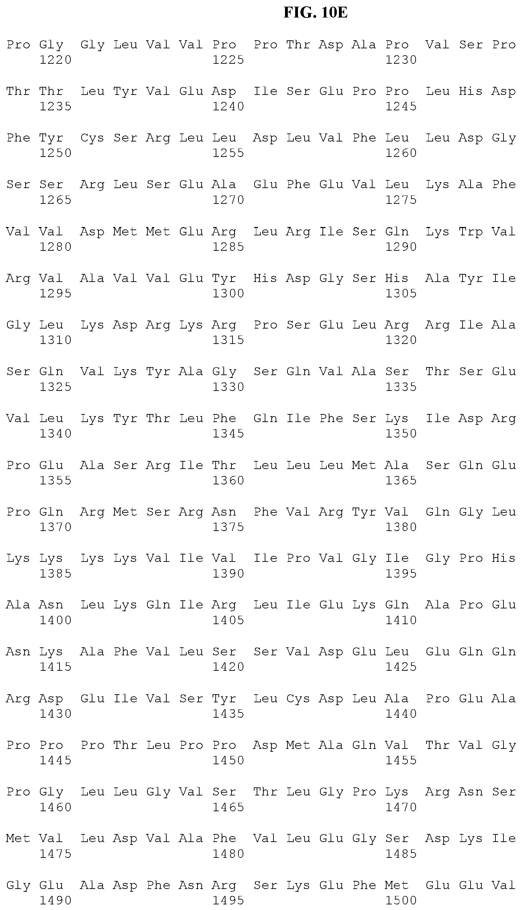

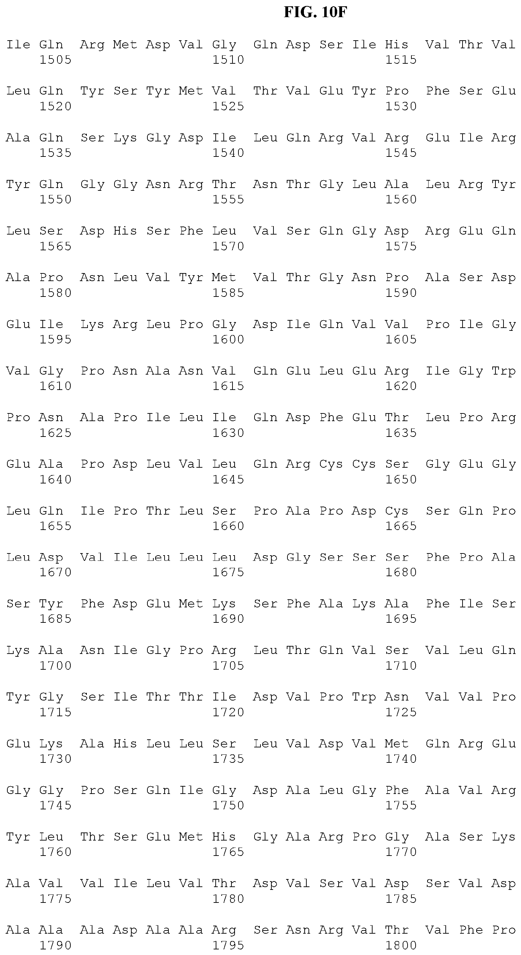

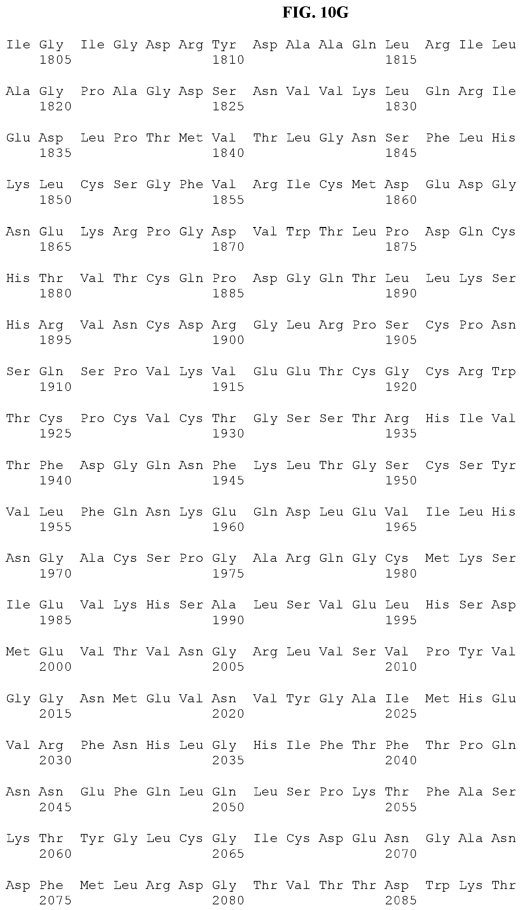

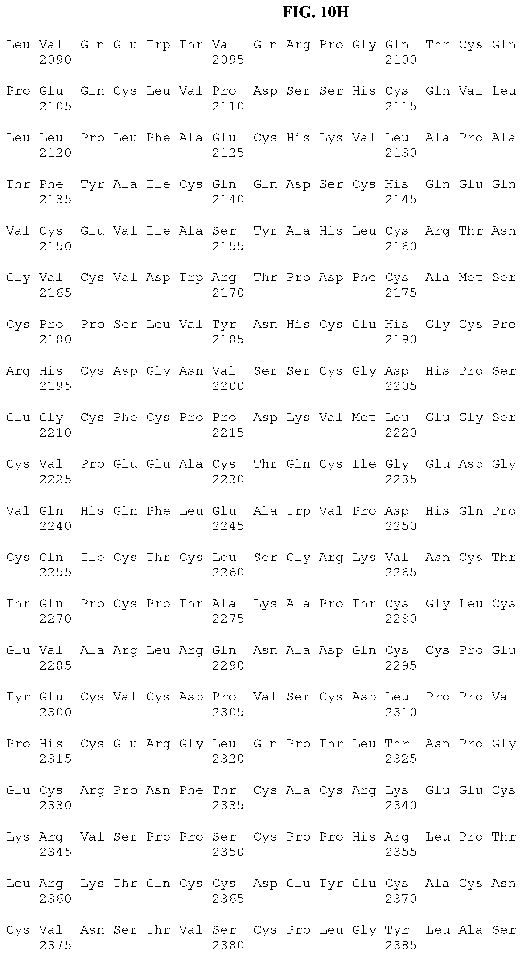

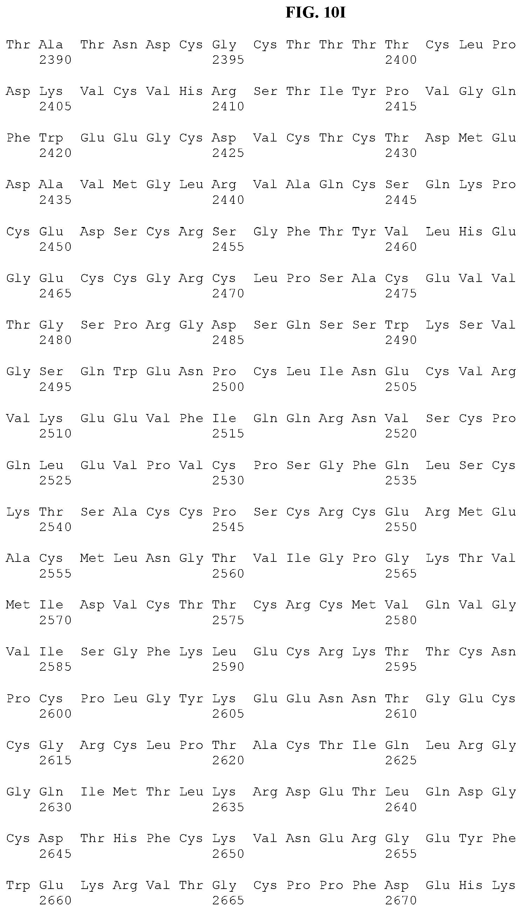

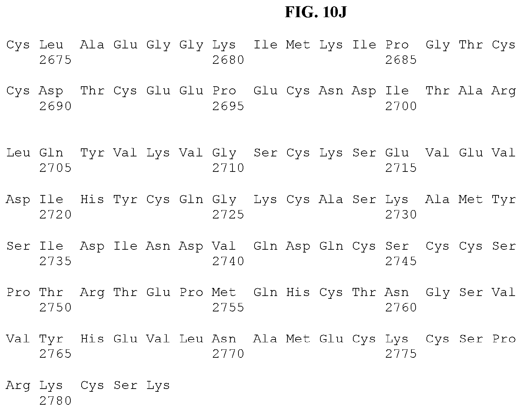

Figure 10A-J depicts the amino acid sequence of prepro-VWF.

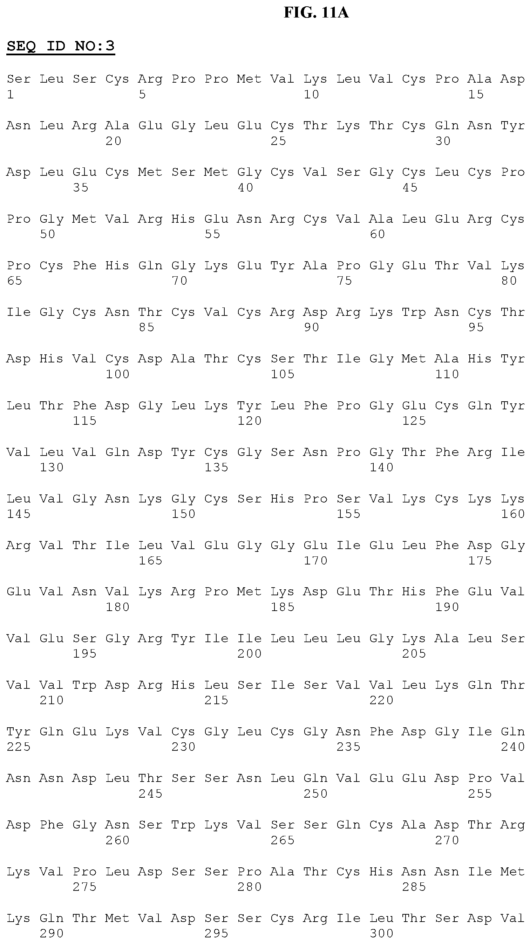

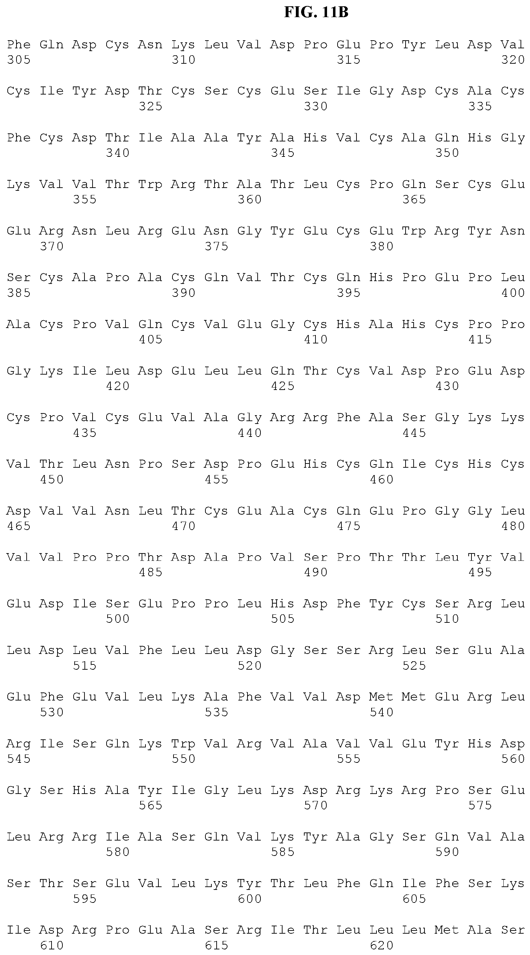

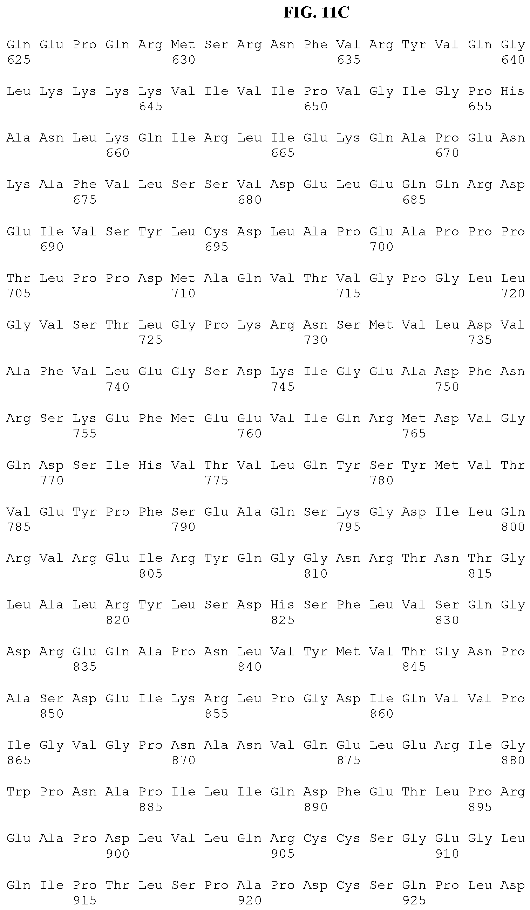

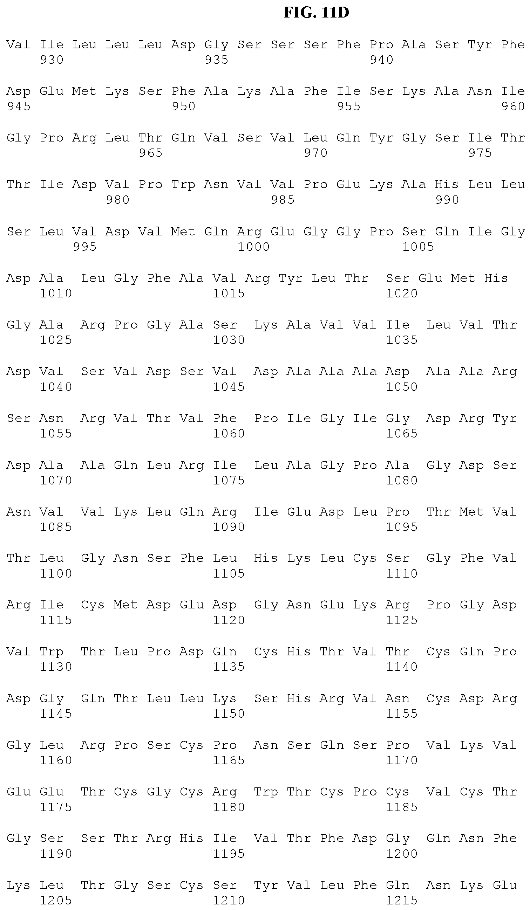

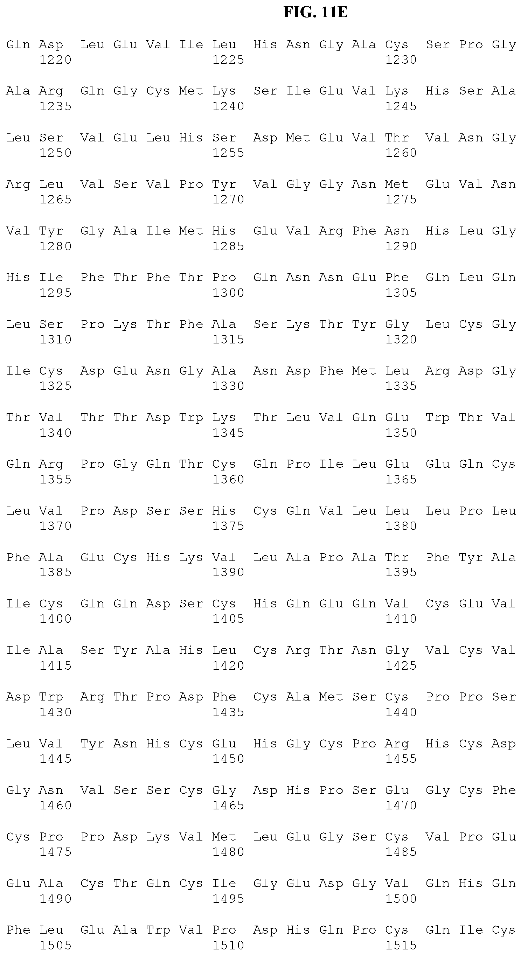

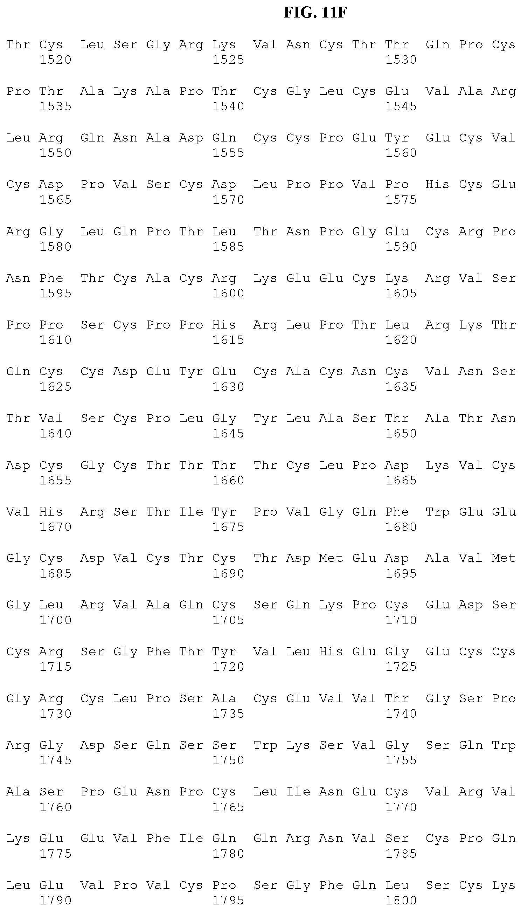

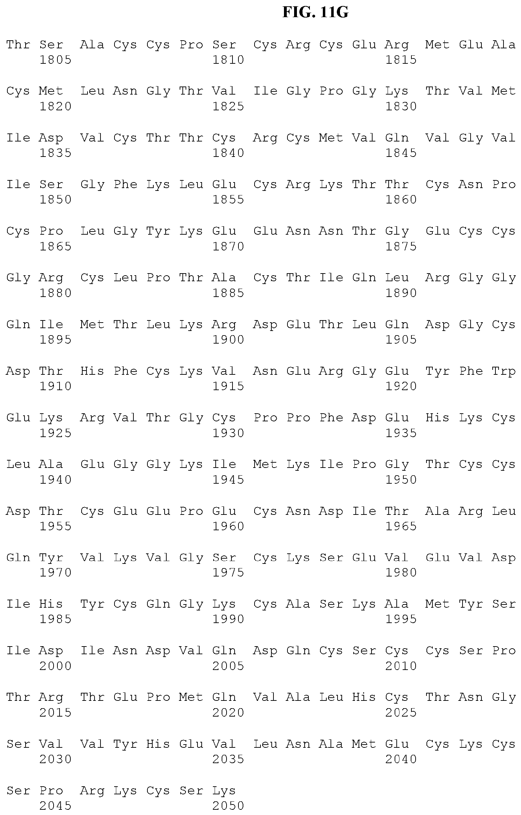

Figure 11 depicts the amino acid sequence of mature VWF (corresponding to amino acid residues 764-2813 of the full length prepro-VWF).

### DETAILED DESCRIPTION OF THE INVENTION

### Introduction

The present invention provides methods for treatment for menorrhagia in women with von Willebrand Disease (VWD). Such treatment comprises administration of recombinant von Willebrand factor (rVWF). Administration of rVWF according to the present invention and for on-demand treatment of bleeding episodes (BEs) in adult patients with severe VWD provides for hemostatic efficacy and a positive benefit-risk profile for the patients.

### Definitions

As used herein and in the appended claims, the singular forms "a," "an," and "the" include plural referents unless the context clearly dictates otherwise. Thus, for example, reference to "an antibody" includes a plurality of such antibodies and reference to "a host cell" includes reference to one or more host cells and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Before the invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

As used herein. "rVWF" refers to recombinant VWF.

As used herein, "rFVIII" refers to recombinant FVIII.

The term "recombinant" when used with reference, *e.g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

As used herein, "recombinant VWF" includes VWF obtained via recombinant DNA technology. In certain embodiments, VWF proteins of the invention can comprise a construct, for example, prepared as in WO 1986/06096 and US 8,597,910, the contents of which are incorporated herein by reference with respect to the methods of producing recombinant VWF. The VWF in the present disclosure can include all potential forms, including the monomeric and multimeric forms. It should also be understood that the present invention encompasses different forms of VWF to be used in combination. For example, the VWF of the present disclosure may include different multimers, different derivatives and both biologically active derivatives and derivatives not biologically active.

In the context of the present disclosure, the recombinant VWF embraces any member of the VWF family from, for example, a mammal such as a primate, human, monkey, rabbit, pig, rodent, mouse, rat, hamster, gerbil, canine, feline, and biologically active derivatives thereof. Mutant and variant VWF proteins having activity are also embraced, as are functional fragments and fusion proteins of the VWF proteins. Furthermore, the VWF of the invention may further comprise tags that facilitate purification, detection, or both. The VWF described herein may further be modified with a therapeutic moiety or a moiety suitable imaging in vitro or in vivo.

As used herein, "plasma-derived VWF (pdVWF)" includes all forms of the protein found in blood including the mature VWF obtained from a mammal having the property of in vivo-stabilizing, *e.g*., binding, of at least one FVIII molecule.

The term "highly multimeric VWF" or "high molecular weight VWF" refers to VWF comprising at least 10 subunits, or 12, 14, or 16 subunits, to about 20, 22, 24, or 26 subunits or more. The term "subunit" refers to a monomer of VWF. As is known in the art, it is generally dimers of VWF that polymerize to form the larger order multimers (see Turecek et al., Semin. Thromb. Hemost. 2010, 36(5): 510-521 which is hereby incorporated by reference in its entirety for all purposes and in particular for all teachings regarding multimer analysis of VWF).

As used herein, the term "factor VIII" or "FVIII" refers to any form of factor VIII molecule with the typical characteristics of blood coagulation factor VIII, whether endogenous to a patient, derived from blood plasma, or produced through the use of recombinant DNA techniques, and including all modified forms of factor VIII. Factor VIII (FVIII) exists naturally and in therapeutic preparations as a heterogeneous distribution of polypeptides arising from a single gene product (see, *e.g*., Andersson et al., Proc. Natl. Acad. Sci. USA, 83:2979-2983 (1986)). Commercially available examples of therapeutic preparations containing Factor VIII include those sold under the trade names of HEMOFIL M^{®}, ADVATE^{®}, and RECOMBINATE^{®} (Takeda Pharmaceutical Company Limited, Lexington, MA).

As used herein, "plasma FVIII activity" and "in vivo FVIII activity" are used interchangeably. The in vivo FVIII activity measured using standard assays may be endogenous FVIII activity, the activity of a therapeutically administered FVIII (recombinant or plasma derived), or both endogenous and administered FVIII activity. Similarly, "plasma FVIII" refers to endogenous FVIII or administered recombinant or plasma derived FVIII.

As used herein "von Willebrand Disease" refers to the group of diseases caused by a deficiency of von Willebrand factor. Von Willebrand factor helps blood platelets clump together and stick to the blood vessel wall, which is necessary for normal blood clotting. As described in further detail herein, there are several types of Von Willebrand disease including type 1, 2A, 2B, 2M, 2N, and 3.

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. VWF is the predominant species present in a preparation is substantially purified. The term "purified" in some embodiments denotes that a nucleic acid or protein gives rise to essentially one band in an electrophoretic gel. In other embodiments, it means that the nucleic acid or protein is at least 50% pure, more preferably at least 60%, 65%, 70%, 75%, 80%, 85%, 90%, 95%, 96%, 97%, 98%, 99% or more pure. "Purify" or "purification" in other embodiments means removing at least one contaminant from the composition to be purified. In this sense, purification does not require that the purified compound be homogenous, *e.g*., 100% pure.

As used herein, "administering" (and all grammatical equivalents) includes intravenous administration, intramuscular administration, subcutaneous administration, oral administration, administration as a suppository, topical contact, intraperitoneal, intralesional, or intranasal administration, or the implantation of a slow-release device, *e.g.,* a mini-osmotic pump, to a subject. Administration is by any route including parenteral, and transmucosal (*e.g*., oral, nasal, vaginal, rectal, or transdermal). Parenteral administration includes, *e.g*., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, etc.

The terms "therapeutically effective amount or dose" or "therapeutically sufficient amount or dose" or "effective or sufficient amount or dose" refer to a dose that produces therapeutic effects for which it is administered. For example, a therapeutically effective amount of a drug useful for treating hemophilia can be the amount that is capable of preventing or relieving one or more symptoms associated with hemophilia. The exact dose will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

As used herein, the terms "patient" and "subject" are used interchangeably and refer to a mammal (preferably human) that has a disease or has the potential of contracting a disease.

As used herein, the term "about" denotes an approximate range of plus or minus 10% from a specified value. For instance, the language "about 20%" encompasses a range of 18-22%.

As used herein, the term "half-life" refers to the period of time it takes for the amount of a substance undergoing decay (or clearance from a sample or from a patient) to decrease by half.

As used herein, the term "trough level" refers to the concentration of a drug in plasma just before the next dose, or the minimum drug concentration between two doses. For example, for a regimen that is administered every 12 hours, the trough level will generally occur 12 hours after the last dose. The trough levels are important as it is indicated that trough levels can correlate with clinical outcome, *e.g.,* low trough levels can cause resistance to develop which can negatively affect the efficacy of the treatment regimen. For example, the trough level can be maintained at ≥30% of plasma levels, *e.g.,* low trough levels would be indicated by a level of <30%. For example, the trough level can be maintained at ≥35% of plasma levels, *e.g.,* low trough levels would be indicated by a level of <35%. For example, the trough level can be maintained at ≥40% of plasma levels, *e.g.,* low trough levels would be indicated by a level of <40%. For example, the trough level can be maintained at ≥45% of plasma levels, e.g., low trough levels would be indicated by a level of <45%. For example, the trough level can be maintained at ≥50% of plasma levels, *e.g.,* low trough levels would be indicated by a level of <50%. In some instances, recommended target trough plasma levels and minimum duration of treatment include a VWF:RCo target trough plasma level of ≥30 IU/dL (minor) and >50 IU/dL (major). In some instances, recommended target trough plasma levels and minimum duration of treatment include a VWF:RCo target trough plasma level of ≥35 IU/dL (minor) and >55 IU/dL (major). In some instances, recommended target trough plasma levels and minimum duration of treatment include a VWF:RCo target trough plasma level of ≥40 IU/dL (minor) and >65 IU/dL (major). In some instances, recommended target trough plasma levels and minimum duration of treatment include a VWF:RCo target trough plasma level of ≥45 IU/dL (minor) and >70 IU/dL (major).

The term "menorrhagia", as used herein, refers to a menstrual bleeding episode (a periodic uterine bleeding event) in which menstrual blood loss is about 80 mL or greater per cycle. In some cases, menorrhagia includes heavy menstrual bleeding lasting for more than 7 days.

### I. Administration of rVWF for Methods of Treating Menorrhagia in Patient with Severe VWD

Provided herein is a method of treating menorrhagia (*i.e*., abnormal uterine bleeding) in subjects with severe VWD. One of the advantages of administering rVWF to such subjects is that the higher specific activity of rVWF as compared to pdVWF allows flexibility in the amount of rVWF administered and the number of times the subject is re-dosed. As will be appreciated and as is discussed in further detail herein, the co-administered FVIII may be recombinant or plasma derived.

In some aspects, rVWF is administered to a subject at a range from 30-100 IU/kg, *e.g*., 30, 40, 45, 50, 55, 60, 65, 70, 75, 80, 90, 100, 30-60, 30-70, 40-100, 40-80, 50-80, 60-80, 70-80, 40-50, 40-60, 40-70, 40-50, 50-60, 50-75, 60-70, 70-80, 40-90, 50-90, 60-90, 70-90, 80-90, or 90-100 IU/kg. In some embodiments, rVWF is administered at least once during a menorrhagia bleeding episode. In other embodiments, rVWF is administered two or more times, *e.g.,* 2, 3, 4, 5, or more times, during a bleeding episode. In some instances, the subject is administered one or more infusions of rVWF. Each infusion can include a range from about 30-90 IU/kg rVWF, *e.g.,* 30, 40, 45, 50, 55, 60, 65, 70, 75, 80, 90, 30-60, 30-70, 60-70, 40-50, 40-60, 40-70, 40-75, 50-75, 60-75, 40-80, 50-75, 50-80, 60-80, 70-80, 40-85, 50-85, 60-85, 70-85, 45-50, 45-60, 45-70, 50-60, 40-90, 50-90, 60-90, 70-90, or 80-90, IU/kg rVWF. In some embodiments, the infusions can be substantially equal in amount of rVWF. For instance, a first infusion and a second infusion (*i.e*., a first dose and a second dose) can be substantially equal in amount of rVWF. In some embodiments, a second infusion and a third infusion (*i.e.,* a second dose and a third dose) can be substantially equal in amount of rVWF. In some embodiments, the total dose of rVWF administered to the subject per bleeding episode is about 30-150 IU/kg, *e.g*., 40-50, 40-90, 40-150, 40-125, 40-100, 40-90, 40-75, 50-150, 50-100, 75-150, or 100-150 IU/kg.

In some embodiments, for minor and moderate menorrhagia bleeding events only 1-2 infusions more than estimated were required to control that bleeding episode and no additional VWF-containing product was required. In some embodiments, for major bleeding events <1.5 times more infusions than estimated were required to control that bleeding episode and no additional VWF-containing product was required. In some embodiments, minor, moderate, and major bleeding events the actual number of infusions was less than or equal to the estimated number required to treat the bleeding event, and no additional VWF-containing product was required.

In some embodiments, rVWF is administered at least once a day, at least twice a day, every 8-12 hours, and the like. In some instances, rVWF is administered for a total of 1 day, 2 days, 3 days, 4 days, 5 days, 6 days, 7 days, 8 days, 9 days, 10 days, 11 days, 12 days, 13 days, 14 days, and the like. In some embodiments, the rVWF is administered every 8 hours, every 9 hours, every 10 hours, every 11 hours, or every 12 hours. In some embodiments, rVWF is administered every 8 to 12 hours for about 3 days to about 7 days.

In some embodiments, the subject with severe VWD has Type 1 VWD. In some embodiments, the subject with severe VWD has Type 2 VWD. In some further embodiments, the subject with severe VWD has Type 2A VWD. In some further embodiments, the subject with severe VWD has Type 2B VWD. In some further embodiments, the subject with severe VWD has Type 2M VWD. In some further embodiments, the subject with severe VWD has Type 2N VWD. In other embodiments, the subject with severe VWD has Type 3 VWD. In other embodiments, the subject with severe VWD has severe non-Type 3 VWD.

In some embodiments, the subject is diagnosed with Type 3 VWD. In some embodiments, the subject is diagnosed with Type 3 VWD and has a baseline VWF antigen level of ≤ 3 IU/dL. In some embodiments, the subject has a baseline VWF antigen level ranging from 3 IU/dL or less. In some embodiments, the subject is diagnosed with severe non-Type 3 VWD. In some embodiments, the subject is diagnosed with Type 1 or Type 2A VWD. In some embodiments, the subject has a baseline VWF ristocetin cofactor activity (VWF:RCo) of < 20 IU/dL or 20%. In some embodiments, the subject has a baseline VWF:RCo ranging from < 20 IU/dL or less (*i.e*., < 0.2 IU/mL or 20% or less). In some embodiments, the subject has a baseline VWF:GPIbM or VWF:GPIbR that is comparable or equivalent to a baseline VWF:RCo ranging from < 20 IU/dL or less (*i.e.*, < 0.2 IU/mL or 20% or less). In some embodiments, the subject is diagnosed with Type 2B VWD. In some embodiments, the subject has Type 2B VWD as diagnosed by genotyping. In some embodiments, the subject is diagnosed with Type 2N VWD. In some embodiments, the subject has a baseline FVIII activity (FVIII:C) of < 10% or ranging from 10% or less. In some embodiments, the subject diagnosed with Type 2N VWD and has a baseline FVIII activity (FVIII:C) of < 10%. In some embodiments, the subject is diagnosed with Type 2M VWD.

In some embodiments, the subject is diagnosed with VWD according to ASH ISTH NHF WFH 2021 guidelines (James et al., Blood Advances, 2021, 5(1):280-300), the contents of which including figures and tables are herein incorporated by reference. In some embodiments, the subject has Type 1 VWD as diagnosed by VWF antigen activity (VWF:Ag), platelet-dependent VWF activity (*e.g*., VWF:GPIbM and VWF:GPIbR), and/or FVIII coagulant activity (FVIIlI:C) such that VWF levels are <30 (which refers to VWF levels of <0.30 IU/mL) and the platelet-dependent VWF activity/VWF:Ag ratio is >0.7. In some instances, a subject with Type 1 VWD has a VWF level of <0.30 IU/mL regardless of bleeding profile, as determined by a clinician. In some instances, a subject with Type 1 VWD has a VWF level of <0.60 IU/mL and an abnormal bleeding profile, as determined by a clinician. In some embodiments, VWF levels refer to VWF:Ag. In some embodiments, VWF levels refer to platelet-dependent VWF activity (*e.g*., VWF:GPIbM or VWF:GPIbR). In some embodiments, VWF levels refer to VWF:Ag and/or to platelet-dependent VWF activity (*e.g*., VWF:GPIbM or VWF:GPIbR). In some embodiments, the subject has Type 1C VWD as diagnosed by VWF antigen activity (VWF:Ag), platelet-dependent VWF activity (*e.g.*, VWF glycoprotein IbM (VWF:GPIbM) and VWF glycoprotein IbR (VWF:GPIbR)), FVIII coagulant activity (FVIIlI:C), and/or desmopressin (DDAVP) trial such that VWF levels are <30 (which refers to VWF levels of <0.30 IU/mL) and the platelet-dependent VWF activity/VWF:Ag ratio is >0.7. In some embodiments, a DDAVP trial includes a 1-hour and 4-hour postinfusion blood work to confirm enhanced VWF clearance in subjects with Type 1C. In some embodiments, the subject has Type 2 VWD as diagnosed by VWF:Ag, platelet-dependent VWF activity, and FVIII:C assessment such that VWF levels are <30 VWF levels of <0.30 IU/mL and the platelet-dependent VWF activity/VWF:Ag ratio is <0.7. In some embodiments, the subject has Type 2A or 2B VWD as diagnosed by VWF:Ag activity, platelet-dependent VWF activity, FVIII:C activity, ratio of VWF collagen binding to antigen (VWF:CB/Ag) assay, VWF multimer analysis, and/or genotyping such that VWF levels are < 30 VWF levels of <0.30 IU/mL, the platelet-dependent VWF activity/VWF:Ag ratio is < 0.7, and an abnormal VWF multimer profile. In some instances, the subject has Type 2B as further confirmed by genetic testing. In some embodiments, the subject has Type 2M VWD as diagnosed by VWF:Ag activity, platelet-dependent VWF activity, FVIII:C activity, ratio of VWF collagen binding to antigen (VWF:CB/Ag) assay, VWF multimer analysis, and/or genotyping such that VWF levels are <30 (VWF levels of <0.30 IU/mL), the platelet-dependent VWF activity/VWF:Ag ratio is <0.7, and a normal VWF multimer profile. In some embodiments, the subject has Type 2N VWD as diagnosed by VWF FVIII binding (VWF:FVIIIB) and/or genetic testing. In some instances, the subject has Type 2N VWD is also diagnosed based on FVIII < VWF levels or abnormal levels. In some cases, the genetic testing detects the presence of one or more Type 2N VWD gene variants. Methods for assessing VWF assays including assays for VWF glycoprotein IB binding, VWF collagen binding, VWF multimers, DDAVP challenge trials are known to those skilled in the art and are described in, for example, Patzke and Favaloro, Methods Mol Biol, 2017, 1646:453-460; Higgins and Goodwin, Am J Hematol, 2019, 94:496-503; Stufano et al., Haemophilia, 2020, 26(2):298-305; Ni et al., Int J Lab Hematol, 2013, 35(2):170-176; Michiels et al., Clinical and Applied Thrombosis/Hemostatis, 2017, 23(6):518-531; Mohammed and Favaloro, Methods Mol Biol, 2017, 1646:461-472.

In some embodiments, the subject has been administered plasma-derived VWF (pdVWF) to treat one or more bleeding episodes in the past 12 months. In some embodiments, the subject has been administered pdVWF in the past 12 months. In some embodiments, the subject has been administered pdVWF to treat 1, 2, 3, 4, 5, 6, 7, 8, 9, 10, 11, 12 or more bleeding episodes in the past 12 months. In some embodiments, the subject has been administered pdVWF in the past 12 months before initial administration of rVWF. In other words, the subject was administered pdVWF within the past 12 months prior to receiving rVWF to treat a menorrhagia bleeding episode.

In some embodiments, the subject is administered a dose of rVWF such that subject's VWF:RCo activity ranges from 0.6 IU/mL (*i.e.,* 60 IU/dL) or greater after administration. In some embodiments, a dose of rVWF is administered such that subject's VWF:RCo activity is greater than 0.6 IU/mL (*i.e*., 60 IU/dL) after administration. In some embodiments, the subject is administered a dose of rVWF such that after administration the subject's VWF:GPIbM or VWF:GPIbR activity is equivalent or comparable to a VWF:RCo activity ranging from 0.6 IU/mL (*i.e*., 60 IU/dL) or greater.

In some embodiments, the subject is administered a dose of rVWF such that subject's FVIII:C activity ranges from 0.4 IU/mL (*i.e*., 40 IU/dL) or greater after administration. In some embodiments, a dose of rVWF is administered such that subject's FVIII:C activity is greater than 0.4 IU/mL (*i.e*., 40 IU/dL) after administration.

In some embodiments, the subject is administered a dose of rVWF such that the subject's VWF:RCo activity ranges from 0.6 IU/mL (*i.e*., 60 IU/dL or 60% or a normal level) or greater (or the subject's VWF:GPIbM or VWF:GPIbR activity is equivalent or comparable to the VWF:RCo activity ranging from 0.6 IU/mL (*i.e*., 60 IU/dL) or greater) and FVIII:C activity ranges from 0.4 IU/mL (*i.e*., 40 IU/dL or 40% of a normal level) or greater after administration. In some embodiments, the dose of rVWF is administered such that the subject's VWF:RCo activity is greater than 0.6 IU/mL (*i.e*., 60 IU/dL or 60% of a normal level) or greater (or the subject's VWF:GPIbM or VWF:GPIbR activity is equivalent or comparable to the VWF:RCo activity ranging from 0.6 IU/mL (*i.e*., 60 IU/dL or 60% of a normal level) or greater) and FVIII:C activity is greater than 0.4 IU/mL (*i.e*., 40% of a normal level) after administration.

One skilled in the art recognizes that trough levels of VWF encompassed herein can be measured according to VWF:RCo as well as VWF:GPIbM, VWF:GPIbR, and other known methods. As such, although the VWF trough levels are stated in terms of VWF:RCo, it should be understood that any of the embodiments herein encompass trough levels of VWF measured by VWF:GPIbM or VWF:GPIbR equivalent to the stated trough level measured by VWF:RCo. In some embodiments, the subject is administered a dose of rVWF such that the subject's trough level of VWF:RCo is at least 30% (*i.e*., 30 IU/dL or 0.3 IU/mL). In some embodiments, the subject is administered a dose of rVWF such that subject's trough level of VWF:RCo is at least 35% (*i.e*., 35 IU/dL or 0.35 IU/mL). In some embodiments, the subject is administered a dose of rVWF such that subject's trough level of VWF:RCo is at least 40% (*i.e*., 40 IU/dL or 0.4 IU/mL). In some embodiments, the subject is administered a dose of rVWF such that subject's trough level of VWF:RCo is at least 45% *(i.e.,* 45 IU/dL or 0.45 IU/mL). In some embodiments, the subject is administered a dose of rVWF such that subject's trough level of VWF:RCo is at least 50% *(i.e.,* 50 IU/dL or 0.5 IU/mL). In some embodiments, the subject is administered a dose of rVWF such that subject's trough level of VWF:RCo is at least 55% *(i.e.,* 55 IU/dL or 0.55 IU/mL). In some embodiments, the subject is administered a dose of rVWF such that subject's trough level of VWF:RCo is at least 60% *(i.e.,* 60 IU/dL or 0.6 IU/mL). In some embodiments, the subject is administered a dose of rVWF such that subject's trough level of VWF:RCo is at least 65% *(i.e.,* 65 IU/dL or 0.65 IU/mL). In some embodiments, the subject is administered a dose of rVWF such that subject's trough level of VWF:RCo is at least 70% (*i.e*., 70 IU/dL or 0.70 IU/mL). In some embodiments, the subject is administered a dose of rVWF such that subject's trough level of VWF:RCo is at least 75% (*i.e*., 75 IU/dL or 0.75 IU/mL).

In some embodiments, administration of rVWF maintains the trough level of VWF:RCo greater than 0.6 IU/mL or 60 IU/dL. In some embodiments, administration of rVWF maintains a trough level of VWF:GPIbM or VWF:GPIbR that is substantially equivalent to a trough level of VWF:RCo greater than 0.6 IU/mL or 60 IU/dL. In some embodiments, administration of rVWF maintains the trough level of VWF:RCo greater than 0.5 IU/mL or 50 IU/dL. In some embodiments, administration of rVWF maintains a trough level of VWF:GPIbM or VWF:GPIbR that is substantially equivalent to a trough level of VWF:RCo greater than 0.5 IU/mL or 50 IU/dL. In some embodiments, administration of rVWF maintains the trough level of VWF:RCo greater than 0.4 IU/mL or 40 IU/dL. In some embodiments, administration of rVWF maintains a trough level of VWF:GPIbM or VWF:GPIbR that is substantially equivalent to a trough level of VWF:RCo greater than 0.4 IU/mL or 40 IU/dL. In some embodiments, administration of rVWF maintains the trough level of VWF:RCo greater than 0.3 IU/mL or 30 IU/dL. In some embodiments, administration of rVWF maintains a trough level of VWF:GPIbM or VWF:GPIbR that is substantially equivalent to a trough level of VWF:RCo greater than 0.3 IU/mL or 30 IU/dL.

In some embodiments, if the subject is experiencing a severe menorrhagia episode, one or more doses of rVWF are administered to maintain the trough level of VWF:RCo greater than 0.5 IU/mL for about 1-7 days or more. In some embodiments, the trough level of VWF:RCo of greater than 0.5 IU/mL is maintained for about 1-2 days, about 1-3 days, about 2-5 days, about 1-5 days, about 1-7 days, about 2-7 days, about 3-7 days, about 4-7 days, about 5-7 days, about 6-7 days, or more. In some embodiments, the trough level of VWF:RCo of greater than 0.5 IU/mL for 1 week, 2 weeks, 3 weeks, or more until resolution of the bleeding episode. In some embodiments, if the subject is experiencing a severe menorrhagia episode, one or more doses of rVWF are administered for about 1-7 days or more to maintain a trough level of VWF:GPIbM or VWF:GPIbR that is substantially equivalent to a trough level of VWF:RCo greater than 0.5 IU/mL. In some embodiments, a trough level of VWF:GPIbM or VWF:GPIbR that is substantially equivalent to a trough level of VWF:RCo of greater than 0.5 IU/mL is maintained for about 1-2 days, about 1-3 days, about 2-5 days, about 1-5 days, about 1-7 days, about 2-7 days, about 3-7 days, about 4-7 days, about 5-7 days, about 6-7 days, or more. In some embodiments, the trough level of VWF:GPIbM or VWF:GPIbR that is substantially equivalent to the trough level of VWF:RCo of greater than 0.5 IU/mL is maintained for 1 week, 2 weeks, 3 weeks, or more until resolution of the bleeding episode.

In some embodiments, if the subject is experiencing a mild to moderate menorrhagia episode, one or more doses of rVWF are administered to maintain the trough level of VWF:RCo greater than 0.3 IU/mL for about 1-7 days or more. In some embodiments, the trough level of VWF:RCo of greater than 0.3 IU/mL is maintained for about 1-2 days, about 1-3 days, about 2-5 days, about 1-5 days, about 1-7 days, about 2-7 days, about 3-7 days, about 4-7 days, about 5-7 days, about 6-7 days, or more. In some embodiments, the trough level of VWF:RCo of greater than 0.5 IU/mL is maintained for 1 week, 2 weeks, 3 weeks, or more until resolution of the bleeding episode. In some embodiments, if the subject is experiencing a mild to moderate menorrhagia episode, one or more doses of rVWF are administered for about 1-7 days or more to maintain a trough level of VWF:GPIbM or VWF:GPIbR that is substantially equivalent to a trough level of VWF:RCo greater than 0.3 IU/mL. In some embodiments, a trough level of VWF:GPIbM or VWF:GPIbR that is substantially equivalent to a trough level of VWF:RCo of greater than 0.3 IU/mL is maintained for about 1-2 days, about 1-3 days, about 2-5 days, about 1-5 days, about 1-7 days, about 2-7 days, about 3-7 days, about 4-7 days, about 5-7 days, about 6-7 days, or more. In some embodiments, a trough level of VWF:GPIbM or VWF:GPIbR that is substantially equivalent to a trough level of VWF:RCo of greater than 0.5 IU/mL is maintained for 1 week, 2 weeks, 3 weeks, or more until resolution of the bleeding episode.

In some embodiments, recombinant Factor VIII (rFVIII) is also administered to the subject with severe VWD to treat a menorrhagia bleeding episode. In some cases, the treatment administered comprises rVWF and rFVIII. In other cases, the treatment administered does not include rFVIII. In some embodiments, rFVIII is administered to the subject at a range of about 10-70 IU/kg, *e.g*., 10, 15, 20, 25, 30, 35, 40, 45, 50, 55, 60, 65, 70, 10-70, 10-60, 10-50, 10-40, 10-30, 10-20, 20-30, 30-40, 40-50, 50-60, or 60-70 IU/kg. In some instances, rFVIII is administered in the initial (first) dose or initial (first) infusion. In some cases, rFVIII is not administered as part of a second dose or second infusion. In some embodiments, a subject with VWD who is experiencing a menorrhagia bleeding episode is administered a single infusion of rVWF and rFVIII. In some embodiments, the second administration of rVWF is not administered with rFVIII.

In some embodiments, of the method, when rVWF and FVIII are administered together, the rVWF to FVIII ratio is about 1.5:0.8. In some embodiments, of the method, when rVWF and FVIII are administered together, the rVWF to FVIII ratio is about 1.3:1. In some embodiments, of the method, when rVWF and FVIII are administered together, the rVWF to FVIII ratio is about 1.1:0.8. In some embodiments, of the method, when rVWF and FVIII are administered together, the rVWF to FVIII ratio is about 1.5:1. In some embodiments, of the method, when rVWF and FVIII are administered together, the rVWF to FVIII ratio is about 1.1:1.2.

In some embodiments, 40-50 IU/kg rVWF is administered when the menorrhagia is minor or moderate menorrhagia. In some embodiments, about 40 IU/kg rVWF is administered when the menorrhagia is minor or moderate menorrhagia. In some embodiments, about 45 IU/kg rVWF is administered when the menorrhagia is minor or moderate menorrhagia. In some embodiments, about 50 IU/kg rVWF is administered when the menorrhagia is minor or moderate menorrhagia. In some embodiments, 1 or 2 doses of 40-50 IU/kg rVWF is administered, wherein the menorrhagia is minor or moderate menorrhagia. In some embodiments, 1 or 2 doses of about 40 IU/kg rVWF is administered, when the menorrhagia is minor or moderate menorrhagia. In some embodiments, 1 or 2 doses of about 45 IU/kg rVWF is administered, when the menorrhagia is minor or moderate menorrhagia. In some embodiments, 1 or 2 doses of about 50 IU/kg rVWF is administered, when the menorrhagia is minor or moderate menorrhagia. In some embodiments, 1 or 2 doses of 40-50 IU/kg rVWF is administered when the menorrhagia is minor or moderate menorrhagia, and wherein the subject severe Type 1 or Type 2 VWD. In some embodiments, 1 or 2 doses of about 40 IU/kg rVWF is administered, when the menorrhagia is minor or moderate menorrhagia and the severe VWD is Type 1 VWD. In some embodiments, 1 or 2 doses of about 45 IU/kg rVWF is administered, when the menorrhagia is minor or moderate menorrhagia and the severe VWD is Type 1 VWD. In some embodiments, 1 or 2 doses of about 50 IU/kg rVWF is administered, when the menorrhagia is minor or moderate menorrhagia and the severe VWD is Type 1 VWD. In some embodiments, 1 or 2 doses of about 40 IU/kg rVWF is administered, when the menorrhagia is minor or moderate menorrhagia and the severe VWD is Type 2 VWD. In some embodiments, 1 or 2 doses of about 45 IU/kg rVWF is administered, when the menorrhagia is minor or moderate menorrhagia and the severe VWD is Type 2 VWD. In some embodiments, 1 or 2 doses of about 50 IU/kg rVWF is administered, when the menorrhagia is minor or moderate menorrhagia and the severe VWD is Type 2 VWD.

In some embodiments, a first dose of about 40-90 IU/kg or about 50-80 IU/kg rVWF is administered when the menorrhagia is major or severe menorrhagia. In some embodiments, a first dose of about 40-90 IU/kg rVWF when the menorrhagia is major or severe menorrhagia and the severe VWD is Type 1 VWD. In some embodiments, a first dose of about 50-75 IU/kg rVWF when the menorrhagia is major or severe menorrhagia and the severe VWD is Type 1 VWD. In some further embodiments, a first dose of about 50 IU/kg rVWF and when the menorrhagia is major or severe menorrhagia and the severe VWD is Type 1 VWD. In some further embodiments, a first dose of about 55 IU/kg rVWF and when the menorrhagia is major or severe menorrhagia and the severe VWD is Type 1 VWD. In some further embodiments, a first dose of about 60 IU/kg rVWF and when the menorrhagia is major or severe menorrhagia and the severe VWD is Type 1 VWD. In some further embodiments, a first dose of about 65 IU/kg rVWF and when the menorrhagia is major or severe menorrhagia and the severe VWD is Type 1 VWD. In some further embodiments, a first dose of about 70 IU/kg rVWF and when the menorrhagia is major or severe menorrhagia and the severe VWD is Type 1 VWD. In further embodiments, a first dose of about 75 IU/kg rVWF and when the menorrhagia is major or severe menorrhagia and the severe VWD is Type 1 VWD. In some embodiments, a first dose of about 80 IU/kg rVWF and when the menorrhagia is major or severe menorrhagia and the severe VWD is Type 1 VWD. In further embodiments, a first dose of about 85 IU/kg rVWF and when the menorrhagia is major or severe menorrhagia and the severe VWD is Type 1 VWD. In some embodiments, a first dose of about 90 IU/kg rVWF and when the menorrhagia is major or severe menorrhagia and the severe VWD is Type 1 VWD. In some further embodiments, after administering a first dose of rVWF, 40-60 IU/kg rVWF is administered every 8 to 12 hours for about 3 days when the menorrhagia is major or severe menorrhagia. In some further embodiments, about 40 IU/kg rVWF is administered every 8 to 12 hours for about 3 days when the menorrhagia is major or severe menorrhagia. In some further embodiments, about 45 IU/kg rVWF is administered every 8 to 12 hours for about 3 days when the menorrhagia is major or severe menorrhagia. In some further embodiments, about 50 IU/kg rVWF is administered every 8 to 12 hours for about 3 days when the menorrhagia is major or severe menorrhagia. In some further embodiments, about 55 IU/kg rVWF is administered every 8 to 12 hours for about 3 days when the menorrhagia is major or severe menorrhagia. In some embodiments, about 60 IU/kg rVWF is administered every 8 to 12 hours for about 3 days when the menorrhagia is major or severe menorrhagia.

In some further embodiments, a first dose of about 50 IU/kg rVWF is administered when the menorrhagia is major or severe menorrhagia and the severe VWD is Type 1 VWD, then about 40 IU/kg rVWF is administered every 8 to 12 hours for about 3 days. In some further embodiments, a first dose of about 50 IU/kg rVWF is administered when the menorrhagia is major or severe menorrhagia and the severe VWD is Type 1 VWD, then about 45 IU/kg rVWF is administered every 8 to 12 hours for about 3 days. In some further embodiments, a first dose of about 50 IU/kg rVWF is administered when the menorrhagia is major or severe menorrhagia and the severe VWD is Type 1 VWD, then about 50 IU/kg rVWF is administered every 8 to 12 hours for about 3 days. In some further embodiments, a first dose of about 50 IU/kg rVWF is administered when the menorrhagia is major or severe menorrhagia and the severe VWD is Type 1 VWD, then about 55 IU/kg rVWF is administered every 8 to 12 hours for about 3 days. In some further embodiments, a first dose of about 50 IU/kg rVWF is administered when the menorrhagia is major or severe menorrhagia and the severe VWD is Type 1 VWD, then about 60 IU/kg rVWF is administered every 8 to 12 hours for about 3 days. In some further embodiments, a first dose of about 55 IU/kg rVWF is administered when the menorrhagia is major or severe menorrhagia and the severe VWD is Type 1 VWD, then about 40 IU/kg rVWF is administered every 8 to 12 hours for about 3 days. In some further embodiments, a first dose of about 55 IU/kg rVWF is administered when the menorrhagia is major or severe menorrhagia and the severe VWD is Type 1 VWD, then about 45 IU/kg rVWF is administered every 8 to 12 hours for about 3 days. In some further embodiments, a first dose of about 55 IU/kg rVWF is administered when the menorrhagia is major or severe menorrhagia and the severe VWD is Type 1 VWD, then about 50 IU/kg rVWF is administered every 8 to 12 hours for about 3 days. In some further embodiments, a first dose of about 55 IU/kg rVWF is administered when the menorrhagia is major or severe menorrhagia and the severe VWD is Type 1 VWD, then about 55 IU/kg rVWF is administered every 8 to 12 hours for about 3 days. In some further embodiments, a first dose of about 55 IU/kg rVWF is administered when the menorrhagia is major or severe menorrhagia and the severe VWD is Type 1 VWD, then about 60 IU/kg rVWF is administered every 8 to 12 hours for about 3 days. In some further embodiments, a first dose of about 60 IU/kg rVWF is administered when the menorrhagia is major or severe menorrhagia and the severe VWD is Type 1 VWD, and then about 40 IU/kg rVWF is administered every 8 to 12 hours for about 3 days. In some further embodiments, a first dose of about 60 IU/kg rVWF is administered when the menorrhagia is major or severe menorrhagia and the severe VWD is Type 1 VWD, and then about 45 IU/kg rVWF is administered every 8 to 12 hours for about 3 days. In some further embodiments, a first dose of about 60 IU/kg rVWF is administered when the menorrhagia is major or severe menorrhagia and the severe VWD is Type 1 VWD, and then about 50 IU/kg rVWF is administered every 8 to 12 hours for about 3 days. In some further embodiments, a first dose of about 60 IU/kg rVWF is administered when the menorrhagia is major or severe menorrhagia and the severe VWD is Type 1 VWD, and then about 55 IU/kg rVWF is administered every 8 to 12 hours for about 3 days. In some further embodiments, a first dose of about 60 IU/kg rVWF is administered when the menorrhagia is major or severe menorrhagia and the severe VWD is Type 1 VWD, and then about 60 IU/kg rVWF is administered every 8 to 12 hours for about 3 days. In some further embodiments, a first dose of about 65 IU/kg rVWF is administered when the menorrhagia is major or severe menorrhagia and the severe VWD is Type 1 VWD, then about 40 IU/kg rVWF is administered every 8 to 12 hours for about 3 days. In some further embodiments, a first dose of about 65 IU/kg rVWF is administered when the menorrhagia is major or severe menorrhagia and the severe VWD is Type 1 VWD, and then about 45 IU/kg rVWF is administered every 8 to 12 hours for about 3 days. In some further embodiments, a first dose of about 65 IU/kg rVWF is administered when the menorrhagia is major or severe menorrhagia and the severe VWD is Type 1 VWD, then about 50 IU/kg rVWF is administered every 8 to 12 hours for about 3 days. In some further embodiments, a first dose of about 65 IU/kg rVWF is administered when the menorrhagia is major or severe menorrhagia and the severe VWD is Type 1 VWD, and then about 55 IU/kg rVWF is administered every 8 to 12 hours for about 3 days. In some further embodiments, a first dose of about 65 IU/kg rVWF is administered when the menorrhagia is major or severe menorrhagia and the severe VWD is Type 1 VWD, and then about 60 IU/kg rVWF is administered every 8 to 12 hours for about 3 days. In some further embodiments, a first dose of about 70 IU/kg rVWF is administered when the menorrhagia is major or severe menorrhagia and the severe VWD is Type 1 VWD, and then about 40 IU/kg rVWF is administered every 8 to 12 hours for about 3 days. In some further embodiments, a first dose of about 70 IU/kg rVWF is administered when the menorrhagia is major or severe menorrhagia and the severe VWD is Type 1 VWD, and then about 45 IU/kg rVWF is administered every 8 to 12 hours for about 3 days. In some further embodiments, a first dose of about 70 IU/kg rVWF is administered when the menorrhagia is major or severe menorrhagia and the severe VWD is Type 1 VWD, and then about 50 IU/kg rVWF is administered every 8 to 12 hours for about 3 days. In some further embodiments, a first dose of about 70 IU/kg rVWF is administered when the menorrhagia is major or severe menorrhagia and the severe VWD is Type 1 VWD, then about 55 IU/kg rVWF is administered every 8 to 12 hours for about 3 days. In some further embodiments, a first dose of about 70 IU/kg rVWF is administered when the menorrhagia is major or severe menorrhagia and the severe VWD is Type 1 VWD, and then about 60 IU/kg rVWF is administered every 8 to 12 hours for about 3 days. In some further embodiments, a first dose of about 75 IU/kg rVWF is administered when the menorrhagia is major or severe menorrhagia and the severe VWD is Type 1 VWD, then about 40 IU/kg rVWF is administered every 8 to 12 hours for about 3 days. In some further embodiments, a first dose of about 75 IU/kg rVWF is administered when the menorrhagia is major or severe menorrhagia and the severe VWD is Type 1 VWD, and then about 45 IU/kg rVWF is administered every 8 to 12 hours for about 3 days. In some further embodiments, a first dose of about 75 IU/kg rVWF is administered when the menorrhagia is major or severe menorrhagia and the severe VWD is Type 1 VWD, then about 50 IU/kg rVWF is administered every 8 to 12 hours for about 3 days. In some further embodiments, a first dose of about 75 IU/kg rVWF is administered when the menorrhagia is major or severe menorrhagia and the severe VWD is Type 1 VWD, and then about 55 IU/kg rVWF is administered every 8 to 12 hours for about 3 days. In some further embodiments, a first dose of about 75 IU/kg rVWF is administered when the menorrhagia is major or severe menorrhagia and the severe VWD is Type 1 VWD, and then about 60 IU/kg rVWF is administered every 8 to 12 hours for about 3 days.

In some embodiments, a first dose of about 50-80 IU/kg rVWF or about 60-80 IU/kg rVWF is administered when the menorrhagia is major or severe menorrhagia. In some further embodiments, a first dose of about 60-80 IU/kg rVWF when the menorrhagia is major or severe menorrhagia and the severe VWD is Type 2 or Type 3 VWD In some further embodiments, a first dose of about 60 IU/kg rVWF and when the menorrhagia is major or severe menorrhagia and the severe VWD is Type 2 or Type 3 VWD. In some further embodiments, a first dose of about 65 IU/kg rVWF and when the menorrhagia is major or severe menorrhagia and the severe VWD is Type 2 or Type 3 VWD. In some further embodiments, a first dose of about 70 IU/kg rVWF and when the menorrhagia is major or severe menorrhagia and the severe VWD is Type 2 or Type 3 VWD. In some further embodiments, a first dose of about 75 IU/kg rVWF and when the menorrhagia is major or severe menorrhagia and the severe VWD is Type 2 or Type 3 VWD. In some further embodiments, a first dose of about 80 IU/kg rVWF and when the menorrhagia is major or severe menorrhagia and the severe VWD is Type 2 or Type 3 VWD. In some further embodiments, after administering a first dose of rVWF, 40-60 IU/kg rVWF is administered every 8 to 12 hours for about 3 days when the menorrhagia is major or severe menorrhagia. In some further embodiments, about 40 IU/kg rVWF is administered every 8 to 12 hours for about 3 days when the menorrhagia is major or severe menorrhagia. In some further embodiments, about 45 IU/kg rVWF is administered every 8 to 12 hours for about 3 days when the menorrhagia is major or severe menorrhagia. In some further embodiments, about 50 IU/kg rVWF is administered every 8 to 12 hours for about 3 days when the menorrhagia is major or severe menorrhagia. In some further embodiments, about 55 IU/kg rVWF is administered every 8 to 12 hours for about 3 days when the menorrhagia is major or severe menorrhagia. In some embodiments, about 60 IU/kg rVWF is administered every 8 to 12 hours for about 3 days when the menorrhagia is major or severe menorrhagia.

In some further embodiments, an initial dose (also referred to as a first dose) of about 60 IU/kg rVWF is administered when the menorrhagia is major or severe menorrhagia and the severe VWD is Type 2 or Type 3 VWD, and then about 40 IU/kg rVWF is administered every 8 to 12 hours for about 3 days. In some further embodiments, a first dose of about 60 IU/kg rVWF is administered when the menorrhagia is major or severe menorrhagia and the severe VWD is Type 2 or Type 3 VWD, and then about 45 IU/kg rVWF is administered every 8 to 12 hours for about 3 days. In some further embodiments, a first dose of about 60 IU/kg rVWF is administered when the menorrhagia is major or severe menorrhagia and the severe VWD is Type 2 or Type 3 VWD, then about 50 IU/kg rVWF is administered every 8 to 12 hours for about 3 days. In some further embodiments, a first dose of about 60 IU/kg rVWF is administered when the menorrhagia is major or severe menorrhagia and the severe VWD is Type 2 or Type 3 VWD, and then about 55 IU/kg rVWF is administered every 8 to 12 hours for about 3 days. In some further embodiments, a first dose of about 60 IU/kg rVWF is administered when the menorrhagia is major or severe menorrhagia and the severe VWD is Type 2 or Type 3 VWD, and then about 60 IU/kg rVWF is administered every 8 to 12 hours for about 3 days. In some further embodiments, a first dose of about 65 IU/kg rVWF is administered when the menorrhagia is major or severe menorrhagia and the severe VWD is Type 2 or Type 3 VWD, and then about 40 IU/kg rVWF is administered every 8 to 12 hours for about 3 days. In some further embodiments, a first dose of about 65 IU/kg rVWF is administered when the menorrhagia is major or severe menorrhagia and the severe VWD is Type 2 or Type 3 VWD, and then about 45 IU/kg rVWF is administered every 8 to 12 hours for about 3 days. In some further embodiments, a first dose of about 65 IU/kg rVWF is administered when the menorrhagia is major or severe menorrhagia and the severe VWD is Type 2 or Type 3 VWD, and then about 50 IU/kg rVWF is administered every 8 to 12 hours for about 3 days. In some further embodiments, a first dose of about 65 IU/kg rVWF is administered when the menorrhagia is major or severe menorrhagia and the severe VWD is Type 2 or Type 3 VWD, and then about 55 IU/kg rVWF is administered every 8 to 12 hours for about 3 days. In some further embodiments, a first dose of about 65 IU/kg rVWF is administered when the menorrhagia is major or severe menorrhagia and the severe VWD is Type 2 or Type 3 VWD, and then about 60 IU/kg rVWF is administered every 8 to 12 hours for about 3 days. In some further embodiments, a first dose of about 70 IU/kg rVWF is administered when the menorrhagia is major or severe menorrhagia and the severe VWD is Type 2 or Type 3 VWD, and then about 40 IU/kg rVWF is administered every 8 to 12 hours for about 3 days. In some further embodiments, a first dose of about 70 IU/kg rVWF is administered when the menorrhagia is major or severe menorrhagia and the severe VWD is Type 2 or Type 3 VWD, and then about 45 IU/kg rVWF is administered every 8 to 12 hours for about 3 days. In some further embodiments, a first dose of about 70 IU/kg rVWF is administered when the menorrhagia is major or severe menorrhagia and the severe VWD is Type 2 or Type 3 VWD, and then about 50 IU/kg rVWF is administered every 8 to 12 hours for about 3 days. In some further embodiments, a first dose of about 70 IU/kg rVWF is administered when the menorrhagia is major or severe menorrhagia and the severe VWD is Type 2 or Type 3 VWD, and then about 55 IU/kg rVWF is administered every 8 to 12 hours for about 3 days. In some further embodiments, a first dose of about 70 IU/kg rVWF is administered when the menorrhagia is major or severe menorrhagia and the severe VWD is Type 2 or Type 3 VWD, and then about 60 IU/kg rVWF is administered every 8 to 12 hours for about 3 days. In some further embodiments, a first dose of about 75 IU/kg rVWF is administered when the menorrhagia is major or severe menorrhagia and the severe VWD is Type 2 or Type 3 VWD, and then about 40 IU/kg rVWF is administered every 8 to 12 hours for about 3 days. In some further embodiments, a first dose of about 75 IU/kg rVWF is administered when the menorrhagia is major or severe menorrhagia and the severe VWD is Type 2 or Type 3 VWD, and then about 45 IU/kg rVWF is administered every 8 to 12 hours for about 3 days. In some further embodiments, a first dose of about 75 IU/kg rVWF is administered when the menorrhagia is major or severe menorrhagia and the severe VWD is Type 2 or Type 3 VWD, and then about 50 IU/kg rVWF is administered every 8 to 12 hours for about 3 days. In some further embodiments, a first dose of about 75 IU/kg rVWF is administered when the menorrhagia is major or severe menorrhagia and the severe VWD is Type 2 or Type 3 VWD, and then about 55 IU/kg rVWF is administered every 8 to 12 hours for about 3 days. In some further embodiments, a first dose of about 75 IU/kg rVWF is administered when the menorrhagia is major or severe menorrhagia and the severe VWD is Type 2 or Type 3 VWD, and then about 60 IU/kg rVWF is administered every 8 to 12 hours for about 3 days. In some further embodiments, a first dose of about 80 IU/kg rVWF is administered when the menorrhagia is major or severe menorrhagia and the severe VWD is Type 2 or Type 3 VWD, and then about 40 IU/kg rVWF is administered every 8 to 12 hours for about 3 days. In some further embodiments, a first dose of about 80 IU/kg rVWF is administered when the menorrhagia is major or severe menorrhagia and the severe VWD is Type 2 or Type 3 VWD, and then about 45 IU/kg rVWF is administered every 8 to 12 hours for about 3 days. In some further embodiments, a first dose of about 80 IU/kg rVWF is administered when the menorrhagia is major or severe menorrhagia and the severe VWD is Type 2 or Type 3 VWD, and then about 50 IU/kg rVWF is administered every 8 to 12 hours for about 3 days. In some further embodiments, a first dose of about 80 IU/kg rVWF is administered when the menorrhagia is major or severe menorrhagia and the severe VWD is Type 2 or Type 3 VWD, and then about 55 IU/kg rVWF is administered every 8 to 12 hours for about 3 days. In some further embodiments, a first dose of about 80 IU/kg rVWF is administered when the menorrhagia is major or severe menorrhagia and the severe VWD is Type 2 or Type 3 VWD, and then about 60 IU/kg rVWF is administered every 8 to 12 hours for about 3 days.

In some further embodiments, a first dose of about 50-80 IU/kg rVWF or about 60-80 IU/kg rVWF is administered when the menorrhagia is major or severe menorrhagia, then 40-60 IU/kg rVWF is administered every 8-12 hours for about 3 days, and then 40-60 IU/kg rVWF is further administered until the total duration of the treatment is 7 days. In some further embodiments, a first dose of 50-80 IU/kg rVWF is administered when the menorrhagia is major or severe menorrhagia, then about 40-60 IU/kg rVWF is administered every 8-12 hours for about 3 days, and then about 40 IU/kg rVWF is further administered until the total duration of the treatment is 7 days. In some further embodiments, a first dose of 50-80 IU/kg rVWF is administered when the menorrhagia is major or severe menorrhagia, then 40-60 IU/kg rVWF is administered every 8-12 hours for about 3 days, and then about 45 IU/kg rVWF is further administered until the total duration of the treatment is 7 days. In some further embodiments, a first dose of 50-80 IU/kg rVWF is administered when the menorrhagia is major or severe menorrhagia, then 40-60 IU/kg rVWF is administered every 8-12 hours for about 3 days, and then about 50 IU/kg rVWF is further administered until the total duration of the treatment is 7 days. In some further embodiments, a first dose of 50-80 IU/kg rVWF is administered when the menorrhagia is major or severe menorrhagia, then 40-60 IU/kg rVWF is administered every 8-12 hours for about 3 days, and then about 55 IU/kg rVWF is further administered until the total duration of the treatment is 7 days. In some further embodiments, a first dose of 50-80 IU/kg rVWF is administered when the menorrhagia is major or severe menorrhagia, then 40-60 IU/kg rVWF is administered every 8-12 hours for about 3 days, and then about 60 IU/kg rVWF is further administered until the total duration of the treatment is 7 days.

Generally, Type 1 VWD is indicated by <30 IU/dL VWF:RCo or an equivalent thereof as determined by platelet binding activity of VWF (*i.e*., VWF:GPIbM and VWF:GPIbR), <30 IU/dL VWF:Ag, low or normal FVIII, and >0.5-0.7 IU/dL VWF:RCo/VWF:Ag ratio or an equivalent thereof based on platelet binding activity of VWF/VWF:Ag ratio. Type 2A VWD is indicated by <30 IU/dL VWF:RCo or an equivalent thereof as determined by platelet binding activity of VWF (*i.e*., VWF:GPIbM and VWF:GPIbR), <30-200 IU/dL VWF:Ag, low or normal FVIII, and <0.5-0.7 IU/dL VWF:RCo/VWF:Ag ratio or an equivalent thereof based on platelet binding activity of VWF/VWF:Ag ratio. Type 2B VWD is indicated by <30-200 IU/dL VWF:RCo or an equivalent thereof as determined by platelet binding activity of VWF (*i.e*., VWF:GPIbM and VWF:GPIbR), <30 IU/dL VWF:Ag, low or normal FVIII, and usually <0.5-0.7 IU/dL VWF:RCo/VWF: Ag ratio or an equivalent thereof based on platelet binding activity of VWF/VWF:Ag ratio. Type 2M VWD is indicated by <30 IU/dL VWF:RCo or an equivalent thereof as determined by platelet binding activity of VWF (*i.e*., VWF:GPIbM and VWF:GPIbR), <30-200 IU/dL VWF:Ag, low or normal FVIII, and <0.5-0.7 IU/dL VWF:RCo/VWF:Ag ratio or an equivalent thereof based on platelet binding activity of VWF/VWF:Ag ratio. Type 2N VWD is indicated by 30-2000 IU/dL VWF:RCo or an equivalent thereof as determined by platelet binding activity of VWF (*i.e*., VWF:GPIbM and VWF:GPIbR), 30-200 IU/dL VWF:Ag, very low FVIII, and >0.5-0.7 IU/dL VWF:RCo/VWF:Ag ratio or an equivalent thereof based on platelet binding activity of VWF/VWF:Ag ratio. Type 3 VWD is indicated by <3 IU/dL VWF:RCo or an equivalent thereof as determined by platelet binding activity of VWF (*i.e*., VWF:GPIbM and VWF:GPIbR), <3 IU/dL VWF:Ag, extremely low (<10 IU/dL) FVIII, and the VWF: RCo/VWF:Ag ratio is not applicable. Normal is indicated by 50-200 IU/dL VWF:RCo or an equivalent thereof as determined by platelet binding activity of VWF (*i.e*., VWF:GPIbM and VWF:GPIbR), 50-200 IU/dL VWF:Ag, normal FVIII, and >0.5-0.7 IU/dL VWF:RCo/VWF:Ag ratio or an equivalent thereof based on platelet binding activity of VWF/VWF:Ag ratio. In some embodiments, the subject has Type 3 VWD. In some embodiments, the subject has severe type 1 VWD. In some embodiments, the subject has severe type 2 VWD.

In some embodiments, the subject had been treated for at least 1 bleeding event within the previous 12 months. In some embodiments, the subject had been treated for more than 1 bleeding event within the previous 12 months.

In one aspect, a subject with VWD is administered at least one dose rVWF ranging from about 30 IU/kg to about 90 IU/kg for the treatment of menorrhagia. In some embodiments, a subject with VWD is administered at least one dose rVWF ranging from about 30 IU/kg to about 90 IU/kg for the treatment of minor menorrhagia. In some embodiments, a subject with VWD is administered at least one dose rVWF ranging from about 30 IU/kg to about 90 IU/kg for the treatment of moderate menorrhagia. In some embodiments, a subject with VWD is administered at least one dose rVWF ranging from about 30 IU/kg to about 90 IU/kg for the treatment of major menorrhagia. In some embodiments, a subject with Type 1 VWD is administered at least one dose rVWF ranging from about 30 IU/kg to about 90 IU/kg for the treatment of menorrhagia including a minor, moderate, or major menorrhagia episode. In some embodiments, a subject with Type 2 VWD is administered at least one dose rVWF ranging from about 30 IU/kg to about 90 IU/kg for the treatment of menorrhagia including a minor, moderate, or major menorrhagia episode. In some embodiments, a subject with Type 2A VWD is administered at least one dose rVWF ranging from about 30 IU/kg to about 90 IU/kg for the treatment of menorrhagia including a minor, moderate, or major menorrhagia episode. In some embodiments, a subject with Type 2B VWD is administered at least one dose rVWF ranging from about 30 IU/kg to about 90 IU/kg for the treatment of menorrhagia including a minor, moderate, or major menorrhagia episode. In some embodiments, a subject with Type 2M VWD is administered at least one dose rVWF ranging from about 30 IU/kg to about 90 IU/kg for the treatment of menorrhagia including a minor, moderate, or major menorrhagia episode. In some embodiments, a subject with Type 2N VWD is administered at least one dose rVWF ranging from about 30 IU/kg to about 90 IU/kg for the treatment of menorrhagia including a minor, moderate, or major menorrhagia episode. In some embodiments, a subject with Type 3 VWD is administered at least one dose rVWF ranging from about 30 IU/kg to about 90 IU/kg for the treatment of menorrhagia including a minor, moderate, or major menorrhagia episode. In some embodiments, a subject with severe Type 3 VWD is administered at least one dose rVWF ranging from about 30 IU/kg to about 90 IU/kg for the treatment of menorrhagia including a minor, moderate, or major menorrhagia episode. In some embodiments, a subject with non-severe Type 3 VWD is administered at least one dose rVWF ranging from about 30 IU/kg to about 90 IU/kg for the treatment of menorrhagia including a minor, moderate, or major menorrhagia episode.

In some embodiments, a subject with VWD with minor menorrhagia is administered a first dose of 30-60 IU/kg rVWF. In some embodiments, a subject with VWD with minor menorrhagia is administered a first dose of 30-60 IU/kg rVWF, and a second dose of 30-60 IU/kg rVWF. In some embodiments, a subject with Type 1 VWD with minor menorrhagia is administered a first dose of 30-60 IU/kg rVWF. In some embodiments, a subject with VWD with minor menorrhagia is administered a first dose of 30-60 IU/kg rVWF, and a second dose of 30-60 IU/kg rVWF. In some embodiments, a subject with Type 2 VWD with minor menorrhagia is administered a first dose of 30-60 IU/kg rVWF. In some embodiments, a subject with VWD with minor menorrhagia is administered a first dose of 30-60 IU/kg rVWF, and a second dose of 30-60 IU/kg rVWF. In some embodiments, a subject with Type 3 VWD with minor menorrhagia is administered a first dose of 30-60 IU/kg rVWF. In some embodiments, a subject with VWD with minor menorrhagia is administered a first dose of 30-60 IU/kg rVWF, and a second dose of 30-60 IU/kg rVWF.

In some embodiments, a subject with VWD with major menorrhagia is administered a first dose of 40-90 IU/kg rVWF. In some embodiments, a subject with VWD with major menorrhagia is administered a first dose of 40-90 IU/kg rVWF and subsequent doses of 30-70 IU/kg rVWF every 8 to 12 hours for about 3 days. In some embodiments, a subject with VWD with major menorrhagia is administered a first dose of 40-90 IU/kg rVWF, additional doses of 30-70 IU/kg rVWF every 8 to 12 hours for about 3 days, and subsequent doses of 30-70 IU/kg rVWF once a day, wherein the total duration of the rVWF treatment is no more than 7 days. In particular embodiments, at least one of the doses of rVWF is administered with recombinant Factor VIII.

In particular embodiments, each dose of rVWF is administered with recombinant Factor VIII. In some embodiments, the first dose of rVWF is administered with recombinant Factor VIII. In some embodiments, at least one of the doses of rVWF administered every 8-12 hours is administered with recombinant Factor VIII. In some embodiments, at least one of the doses of rVWF administered every day is administered with recombinant Factor VIII. In some instances, rVWF and rFVIII are administered concomitantly or sequentially to the subject. In some embodiments, rFVIII is administered to the subject at a dose of about 20 IU/kg to about 50 IU/kg.

In some embodiments, a subject with Type 1 VWD with major menorrhagia is administered a first dose of 40-90 IU/kg rVWF. In some embodiments, a subject with Type 1 VWD with major menorrhagia is administered a first dose of 40-90 IU/kg rVWF and subsequent doses of 30-70 IU/kg rVWF every 8 to 12 hours for about 3 days. In some embodiments, a subject with Type 1 VWD with major menorrhagia is administered a first dose of 40-90 IU/kg rVWF, additional doses of 30-70 IU/kg rVWF every 8 to 12 hours for about 3 days, and subsequent doses of 30-70 IU/kg rVWF once a day, wherein the total duration of the rVWF treatment is no more than 7 days. In particular embodiments, at least one of the doses of rVWF is administered with recombinant Factor VIII. In particular embodiments, each dose of rVWF is administered with recombinant Factor VIII. In some embodiments, the first dose of rVWF is administered with recombinant Factor VIII. In some embodiments, at least one of the doses of rVWF administered every 8-12 hours is administered with recombinant Factor VIII. In some embodiments, at least one of the doses of rVWF administered every day is administered with recombinant Factor VIII. In some instances, rVWF and rFVIII are administered concomitantly or sequentially to the subject. In some embodiments, rFVIII is administered to the subject at a dose of about 20 IU/kg to about 50 IU/kg.

In some embodiments, a subject with Type 2 VWD with major menorrhagia is administered a first dose of 50-90 IU/kg rVWF. In some embodiments, a subject with Type 2 VWD with major menorrhagia is administered a first dose of 50-90 IU/kg rVWF and subsequent doses of 30-70 IU/kg rVWF every 8 to 12 hours for about 3 days. In some embodiments, a subject with Type 2 VWD with major menorrhagia is administered a first dose of 50-90 IU/kg rVWF, additional doses of 30-70 IU/kg rVWF every 8 to 12 hours for about 3 days, and subsequent doses of 30-70 IU/kg rVWF once a day, wherein the total duration of the rVWF treatment is no more than 7 days. In particular embodiments, at least one of the doses of rVWF is administered with recombinant Factor VIII. In particular embodiments, each dose of rVWF is administered with recombinant Factor VIII. In some embodiments, the first dose of rVWF is administered with recombinant Factor VIII. In some embodiments, at least one of the doses of rVWF administered every 8-12 hours is administered with recombinant Factor VIII. In some embodiments, at least one of the doses of rVWF administered every day is administered with recombinant Factor VIII. In some instances, rVWF and rFVIII are administered concomitantly or sequentially to the subject. In some embodiments, rFVIII is administered to the subject at a dose of about 20 IU/kg to about 50 IU/kg.

In some embodiments, a subject with Type 3 VWD with major menorrhagia is administered a first dose of 50-90 IU/kg rVWF. In some embodiments, a subject with Type 3 VWD with major menorrhagia is administered a first dose of 50-90 IU/kg rVWF and subsequent doses of 30-70 IU/kg rVWF every 8 to 12 hours for about 3 days. In some embodiments, a subject with Type 3 VWD with major menorrhagia is administered a first dose of 50-90 IU/kg rVWF, additional doses of 30-70 IU/kg rVWF every 8 to 12 hours for about 3 days, and subsequent doses of 30-70 IU/kg rVWF once a day, wherein the total duration of the rVWF treatment is no more than 7 days. In particular embodiments, at least one of the doses of rVWF is administered with recombinant Factor VIII. In particular embodiments, each dose of rVWF is administered with recombinant Factor VIII. In some embodiments, the first dose of rVWF is administered with recombinant Factor VIII. In some embodiments, at least one of the doses of rVWF administered every 8-12 hours is administered with recombinant Factor VIII. In some embodiments, at least one of the doses of rVWF administered every day is administered with recombinant Factor VIII. In some instances, rVWF and rFVIII are administered concomitantly or sequentially to the subject. In some embodiments, rFVIII is administered to the subject at a dose of about 20 IU/kg to about 50 IU/kg.

In some embodiments, a subject with severe Type 1 VWD and undergoing a major menorrhagia episode is administered for a total duration of no more than 7 days: (a) a first dose of 50-75 IU/kg rVWF; (b) a second set of doses of 40-60 IU/kg rVWF every 8-12 hours for 3 days such that the subject's the trough level of VWF:RCo is at least 50%; and (c) a third set of doses of 40-60 IU/kg rVWF to the subject once a day.

In some embodiments, a subject with severe Type 2 VWD and undergoing a major menorrhagia episode is administered for a total duration of no more than 7 days: (a) a first dose of 60-80 IU/kg rVWF; (b) a second set of doses of 40-60 IU/kg rVWF every 8-12 hours for 3 days such that the subject's the trough level of VWF:RCo is at least 50%; and (c) a third set of doses of 40-60 IU/kg rVWF to the subject once a day.

In some embodiments, if the subject is experiencing a minor to moderate menorrhagia episode, one or more doses of rVWF are administered. In some embodiments, for a minor to moderate bleeding episode the subject is administered one dose of rVWF. In some embodiments, for a minor to moderate bleeding episode the subject is administered two doses of rVWF. In some embodiments, for a major menorrhagia bleeding episode the subject is administered one dose of rVWF. In some embodiments, for a major bleeding episode the subject is administered two doses of rVWF.

In some embodiments, a subject with a menorrhagia episode is administered a dose of rVWF ranging from about 30-90 IU/kg, about 40-80 IU/kg, about 50-75 IU/kg, about 50-90 IU/kg, about 50-80 IU/kg, about 70-90 IU/kg, or about 40-60 IU/kg. In some embodiments, the subject with a major menorrhagia episode is administered a dose of rVWF ranging from about 30-90 IU/kg, about 40-80 IU/kg, about 50-75 IU/kg, about 50-90 IU/kg, about 50-80 IU/kg, about 70-90 IU/kg, or about 40-60 IU/kg. In some embodiments, the subject with a moderate menorrhagia episode is administered a dose of rVWF ranging from about 30-90 IU/kg, about 40-80 IU/kg, about 50-75 IU/kg, about 50-90 IU/kg, about 50-80 IU/kg, about 70-90 IU/kg, or 40-60 IU/kg. In some embodiments, the subject with a minor menorrhagia episode is administered a dose of rVWF ranging from about 30-90 IU/kg, about 40-80 IU/kg, about 50-75 IU/kg, about 50-90 IU/kg, about 50-80 IU/kg, about 70-90 IU/kg, or about 40-60 IU/kg. In some embodiments, the subject is administered 1, 2, or more doses of rVWF.

In some embodiments, a subject with a menorrhagia episode is administered a first dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg. In certain embodiments, the subject is administered a second dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg.

In some embodiments, a subject with a major menorrhagia episode is administered a first dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg. In certain embodiments, the subject is administered a second dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg.

In some embodiments, the subject with a moderate menorrhagia episode is administered a first dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg. In certain embodiments, the subject is administered a second dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg.

In some embodiments, the subject with a mild menorrhagia episode is administered a first dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg. In certain embodiments, the subject is administered a second dose of rVWF ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg.

In some embodiments, the subject with a menorrhagia episode is administered a first dose ranging from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg. In some embodiments, subsequent doses of rVWF range from about 30-90 IU/kg, about 30-60 IU/kg, about 40-80 IU/kg, about 50-75 IU/kg, about 50-90 IU/kg, about 50-80 IU/kg, about 70-90 IU/kg, or about 40-60 IU/kg every 4-12 hours.

In some embodiments, subsequent doses of rVWF range from about 30-70 IU/kg or about 40-60 IU/kg every 4-12 hours, 6-12 hours, 8-12 hours, 10-12 hours, 1 hour, 2 hours, 3 hours, 4 hours, 5 hours, 6 hours, 7 hours, 8 hours, 9 hours, 10 hours, 11 hours, and 12 hours. In some embodiments, subsequent doses of rVWF are administered over the duration of the bleeding episode. In some embodiments, subsequent doses of rVWF are administered until the resolution of the bleeding episode. In some embodiments, subsequent doses of rVWF are administered for about 1-2 days, about 1-3 days, about 2-5 days, about 1-5 days, about 1-7 days, about 2-7 days, about 3-7 days, about 4-7 days, about 5-7 days, about 6-7 days, or more. In some embodiments after the first dose, rVWF is administered at a dose ranging from about 30-70 IU/kg or about 40-60 IU/kg every 8-12 hours for about 3 days.

In some embodiments, the first dose of rVWF is administered on the first day of treatment and the subsequent doses are administered for a total of up to 7 days of treatment. In some embodiments, a first dose of rVWF is administered on day 1 and the following doses are administered for 2 days, 3 days, 4 days, 5 days, or more. In certain embodiments, administration of rVWF maintains a trough level of VWF:RCo of 0.5 IU/mL or greater or an equivalent thereof as determined by a comparable VWF activity assay such as, but not limited to, a VWF:GPIbM assay and a VWF:GPIbR assay.

In some embodiments, a subject having a minor or moderate menorrhagia episode is administered 40-50 IU/kg in one or two doses (*i.e*., one or two infusions). In some embodiments, the subject is diagnosed with severe Type 1 VWD. In some embodiments, the subject is diagnosed with severe Type 1 VWD and a baseline VWF:RCo activity of 20% or an equivalent thereof as determined by a comparable VWF activity assay such as, but not limited to, a VWF:GPIbM assay and a VWF:GPIbR assay.

In some embodiments, a subject having a major or severe menorrhagia episode is administered an initial dose (*i.e*., a first dose or a first set of dose) of about 50-75 IU/kg, a second dose or a second set of doses of about 40-60 IU/kg every 8-12 hours for 3 days, and a third dose or a third set of doses of about 40-60 IU/kg daily for a total of up to 7 days of treatment. In some embodiments, a subject having a major or severe menorrhagia episode is administered a first dose of about 60-80 IU/kg and additional, subsequent doses of about 40-60 IU/kg every 8-12 hours for 3 days. In some embodiments, a subject having a major or severe menorrhagia episode is administered a first dose of about 60-80 IU/kg and additional, subsequent dose of about 40-60 IU/kg daily for a total of up to 7 days of treatment. In some instances, the additional doses after the first dose keeps the trough level of VWF:RCo of 0.5 IU/mL or greater or an equivalent thereof as determined by a comparable VWF activity assay. In some embodiments, the subject is diagnosed with severe Type 1 VWD. In some embodiments, the subject is diagnosed with severe Type 1 VWD and a baseline VWF:RCo activity of 20% or an equivalent thereof as determined by a comparable VWF assay such as, but not limited to, a VWF:GPIbM assay and a VWF:GPIbR assay.

In some embodiments, a subject having a minor or moderate menorrhagia episode is administered 40-50 IU/kg in one or two doses (*i.e*., one or two infusions). In some embodiments, the subject is diagnosed with Type 2 VWD, including but not limited to Type 2A, Type 2B, Type 2N or Type 2M. In some embodiments, the subject is diagnosed with Type 3 VWD.

In some embodiments, a subject having a major or severe menorrhagia episode is administered a first dose (*i.e*., a first dose or a first set of doses) of about 60-80 IU/kg, a second dose or a second set of doses of about 40-60 IU/kg every 8-12 hours for 3 days, and a third dose or a third set of doses of about 40-60 IU/kg daily for a total of up to 7 days of treatment. In some embodiments, a subject having a major or severe menorrhagia episode is administered a first dose of about 60-80 IU/kg and additional, subsequent doses of about 40-60 IU/kg every 8-12 hours for 3 days. In some embodiments, a subject having a major or severe menorrhagia episode is administered a first dose of about 60-80 IU/kg and additional, subsequent dose of about 40-60 IU/kg daily for a total of up to 7 days of treatment. In some instances, the doses after the first dose keep the trough level of VWF:RCo of 0.5 IU/mL or greater or an equivalent thereof as determined by a comparable VWF activity assay. In some embodiments, the subject is diagnosed with Type 2 VWD, including but not limited to Type 2A, Type 2B, Type 2N or Type 2M VWD. In some embodiments, the subject is diagnosed with Type 3 VWD, *i.e*., severe or non-severe Type 3 VWD.

In some embodiments, a subject having a menorrhagia episode is administered rVWF ranging from about 40 IU/kg to about 200 IU/kg (*e.g*., about 40 IU/kg to about 200 IU/kg, about 40 IU/kg to about 195 IU/kg, about 40 IU/kg to about 190 IU/kg, about 40 IU/kg to about 185 IU/kg, about 40 IU/kg to about 180 IU/kg, about 40 IU/kg to about 175 IU/kg, about 40 IU/kg to about 170 IU/kg, about 40 IU/kg to about 165 IU/kg, about 40 IU/kg to about 160 IU/kg, about 40 IU/kg to about 155 IU/kg, about 40 IU/kg to about 150 IU/kg, about 41 IU/kg to about 200 IU/kg, about 42 IU/kg to about 200 IU/kg, about 43 IU/kg to about 200 IU/kg, about 44 IU/kg to about 200 IU/kg, about 45 IU/kg to about 200 IU/kg, about 46 IU/kg to about 200 IU/kg, about 47 IU/kg to about 200 IU/kg, about 48 IU/kg to about 200 IU/kg, about 49 IU/kg to about 200 IU/kg, about 50 IU/kg to about 200 IU/kg, about 52 IU/kg to about 200 IU/kg, about 55 IU/kg to about 200 IU/kg, about 58 IU/kg to about 200 IU/kg, about 60 IU/kg to about 200 IU/kg, about 62 IU/kg to about 200 IU/kg, about 64 IU/kg to about 200 IU/kg, about 66 IU/kg to about 200 IU/kg, about 68 IU/kg to about 200 IU/kg, about 69 IU/kg to about 200 IU/kg, about 70 IU/kg to about 200 IU/kg, about 72 IU/kg to about 200 IU/kg, about 74 IU/kg to about 200 IU/kg, about 75 IU/kg to about 200 IU/kg, about 77 IU/kg to about 200 IU/kg, about 79 IU/kg to about 200 IU/kg, about 80 IU/kg to about 200 IU/kg, about 40 IU/kg to about 160 IU/kg, about 40 IU/kg to about 159 IU/kg, about 44 IU/kg to about 185 IU/kg, and the like) over the course (*i.e*., the duration) of the bleeding episode.

In some embodiments, a subject having a menorrhagia episode is administered rFVIII ranging from about 15 IU/kg to about 100 IU/kg (*e.g*., about 15 IU/kg to about 100 IU/kg, about 17 IU/kg to about 100 IU/kg, about 18 IU/kg to about 100 IU/kg, about 19 IU/kg to about 100 IU/kg, about 20 IU/kg to about 100 IU/kg, about 21 IU/kg to about 100 IU/kg, about 22 IU/kg to about 100 IU/kg, about 23 IU/kg to about 100 IU/kg, about 24 IU/kg to about 100 IU/kg, about 25 IU/kg to about 100 IU/kg, about 26 IU/kg to about 100 IU/kg, about 27 IU/kg to about 100 IU/kg, about 28 IU/kg to about 100 IU/kg, about 29 IU/kg to about 100 IU/kg, about 30 IU/kg to about 100 IU/kg, about 31 IU/kg to about 100 IU/kg, about 32 IU/kg to about 100 IU/kg, about 33 IU/kg to about 100 IU/kg, about 34 IU/kg to about 100 IU/kg, about 35 IU/kg to about 100 IU/kg ,about 36 IU/kg to about 100 IU/kg, about 37 IU/kg to about 100 IU/kg, about 38 IU/kg to about 100 IU/kg, about 39 IU/kg to about 100 IU/kg, about 40 IU/kg to about 100 IU/kg, about 41 IU/kg to about 100 IU/kg, about 42 IU/kg to about 100 IU/kg, about 43 IU/kg to about 100 IU/kg, about 44 IU/kg to about 100 IU/kg, about 45 IU/kg to about 100 IU/kg, about 46 IU/kg to about 100 IU/kg, about 47 IU/kg to about 100 IU/kg, about 48 IU/kg to about 100 IU/kg, about 49 IU/kg to about 100 IU/kg, about 50 IU/kg to about 100 IU/kg, about 15 IU/kg to about 75 IU/kg, about 20 IU/kg to about 75 IU/kg, about 25 IU/kg to about 75 IU/kg, about 30 IU/kg to about 75 IU/kg, about 25 IU/kg to about 45 IU/kg, about 28 IU/kg to about 40 IU/kg, about 25 IU/kg to about 74 IU/kg, about 30 IU/kg to about 74 IU/kg, about 31 IU/kg to about 74 IU/kg, and the like) over the course of the bleeding episode.

In some embodiments, the subject who is administered rVWF to treat a menorrhagia episode has previously been administered hormonal therapy to treat a previous menorrhagia episode. In many embodiments, a previous menorrhagia episode was treated using a combined contraception or a levonorgestrel-releasing intrauterine system. In some embodiments, a previous menorrhagia episode was treated using transexamic acid. In other embodiments, a previous menorrhagia episode was treated using desmopressin. Descriptions of other methods for treating a menorrhagia episode in a subject with VWD can be found, for example, in Connell et al., Blood, 2021, 5(1):301-325; Rimmer et al., Haemophilia, 2013, 19(6):933-938; Adeyemi-Fowode et al.,; J Pediatr Adolesc Gynecol, 2017, 30(4):479-483; Chi et al., Contraception, 2011, 83(3): 242-247; Kouides et al., Br J Haematol, 2009, 145(2):212-220; Kingman et al., BJOG, 2004, 111(12):1425-1428; Amesse et al., J Pediatr Hemaltol Oncol, 2005, 27(7):357-363; and Lukes et al., Fertil Steril, 2008, 90(3):673-677; the contents of which are thereby incorporated by reference in their entirety.

In some embodiments, the subject is treated with rVWF alone. In some embodiments, the subject is not treated with plasma-derived VWF. In some embodiments, the subject is administered recombinant FVIII. In some embodiments, the subject is not administered recombinant FVIII to treat a menorrhagia episode.

In some embodiments, the subject is administered one dose of rVWF for a menorrhagia episode. In some embodiments, the subject is administered at least one dose of rVWF for a menorrhagia episode. In some embodiments, the subject is administered 2-6 doses, *e.g.,* 2 doses, 3 doses, 4 doses, 5 doses or 6 doses of rVWF for a menorrhagia episode. In some embodiments, the subject is administered 2-6 infusions, *e.g*., 2 infusions, 3 infusions, 4 infusions, 5 infusions or 6 infusions of rVWF for a menorrhagia episode.

Menorrhagia, or heavy menstrual bleeding (HMB), is a common symptom that women with bleeding disorders such as VWD experience. The average menstrual blood loss is 25-80 ml without significant clots for women who do not have a bleeding disorder. In some cases, a subject is considered to experience menorrhagia based on the subject's self-reported symptoms and the effect of such symptoms on quality of life rather than the subject's average menstrual blood loss. As such, a subject with menorrhagia can have an average blood less of less than 80 ml per menorrhagia bleeding episode. Guidelines for classification and management of menorrhagia can be found, *e.g*., in National Institute for Health and Care Excellence (NICE) Heavy menstrual bleeding: assessment and management. NICE guideline, 2018; Quinn and Higham, Women's Health (Lond.), 2016, 122(1):21-26; and Magnay et al., BMC Women's Health, 2020, 20:24. In some instances, menorrhagia is be classified according to the PALM-COEIN system (Management of acute abnormal uterine bleeding in nonpregnant reproductive-aged women. Committee Opinion No. 557. American College of Obstetricians and Gynecologists. Obstet Gynecol 2013;121:891-6.).

A menorrhagia bleeding episode can be evaluated by measurements and assessments recognized by those skilled in the art. Non-limiting examples of an assessment include the Ruta Menorrhagia Score, the FIGO AUB System 1, the FIGO AUB System 2, the pictorial blood assessment chart (PBAC), cycle severity score (CSR), cycle length (CL), a questionnaire, a health survey, and any combination thereof.

In some embodiments, minor menorrhagia refers to a menstrual bleeding episode such that the subject's hemoglobin level is greater than 12 g/dL. In some embodiments, moderate menorrhagia refers to a menstrual bleeding episode such that the subject's hemoglobin level is about 9-12 g/dL. In some embodiments, major or severe menorrhagia refers to a menstrual bleeding episode such that the subject's hemoglobin level is about less than 9 g/dL. In some instances, menorrhagia is based on one or more of the following factors: (a) amount of menstrual bleeding based on the subject's own experience, (b) the incidence of menorrhagia, (c) frequency of menses, (d) duration of flow, and (e) the volume of monthly blood loss.

In some embodiments, minor menorrhagia includes a menstrual bleeding episode characterized as having a lower bleeding severity compared to major menorrhagia. In some embodiments, major or severe menorrhagia includes o a menstrual bleeding episode characterized as having a greater bleeding severity compared to minor menorrhagia. In some instances, a minor, moderate, or major menorrhagia bleeding episode is determined by the subject upon evaluating/assessing the effect of the bleeding episode on the subject's physical, social, emotional and/or material quality of life.

Generally, minor bleeding is characterized by acute or subacute clinically overt bleeding that did not satisfy the criteria for major hemorrhage, major bleeding and/or clinically relevant non-major bleeding (CRNM bleeding) (Aristotle clinical definition). Generally major bleeding is characterized by International Society on Thrombosis and Haemostasis (ISTH) standards, and includes, any life threatening and/or fatal bleeding and symptomatic bleeding into a critical area or organ. Major bleeding events can include those where there is fall in hemoglobin at least 20g/L or transfusion of > 2 units of whole blood (packed cells mentioned in life-threatening bleed definition; RE-LY definition of life-threatening bleeding: ≥ 1 of the following criteria: reduction in hemoglobin level of at least 5.0 g/L; transfusion of at least 4 U of blood or packed cells; associated with hypotension requiring the use of intravenous inotropic agents; fall in hemoglobin > 2g/dL or transfusion of > 2 units of whole blood/red cells (ISTH or Rocket-AF clinical definition); and/or bleeding requiring surgery or transfusion of ≥2 U or associated with a decrease in hemoglobin of ≥ 2.0 g/L episodes. *See*, for example, Rodeghiero et al., Journal of Thrombosis and Haemostasis, 2010, 8:2063-2065 and supplementary materials for the ISTH Blood Assessment Tool; "The Diagnosis, Evaluation, and Management of von Willebrand Disease," NIH Publication No. 08-5832, December 2007; and Connell et al., Blood Advances, 2021, 5(1):301-325.

### II. Treatment Efficacy of Administration of rVWF for Treating Menorrhagia in Patient with Severe VWD

In some embodiments, the present invention provides for treating menorrhagia episodes in a subject with severe von Willebrand Disease (VWD). In some embodiments, the treatment comprises administering to the subject recombinant von Willebrand Factor (rVWF) in order to control a menorrhagia episode. In some embodiments, controlling a menorrhagia episode includes but is not limited to reducing bleed severity, bleed duration, bleed frequency, and time to resolution of the menorrhagia episode. In some embodiments, controlling a menorrhagia episode is indicative of treatment efficacy.

In some embodiments, treatment efficacy of rVWF administration is indicated by an improvement in FVIII, FVIII:C, VWF:RCo, VWF:GPIbM, VWF:GPIbR, platelet-dependent VWF activity/VWF:Ag ratio, ratio of VWF collagen binding to antigen (VWF:CB/VWF:Ag ratio) and/or VWF:Ag activity levels after the treatment with rVWF as compared to the levels prior to the treatment with rVWF. In some embodiments, an improvement in FVIII, FVIII:C, VWF:RCo, VWF:GPIbM, VWF:GPIbR, platelet-dependent VWF activity/VWF:Ag ratio, ratio of VWF collagen binding to antigen (VWF:CB/VWF:Ag ratio) and/or VWF:Ag activity levels includes a change in the activity levels such that the activity levels are closer to normal levels, *i.e*., levels in a subject that does not have VWD.

In some embodiments, treatment efficacy is determined according to a clinical rating scale. In some embodiments, the clinical rating scale includes 3, 4, or 5 categories or criteria. In some embodiments, the clinical rating scale includes a rating of "excellent", "good", "moderate" and "none".

In some embodiments, administration of rVWF according to the methods provided herein to a patient experiencing a major or severe menorrhagia episode provides a therapeutic efficacy categorized as "excellent" or "good". In some instances, the therapeutic efficacy of rVWF administration is defined as "moderate". In some embodiments, administration of rVWF according to the methods provided herein to a patient experiencing a minor and/or moderate menorrhagia episode provides a therapeutic efficacy categorized as "excellent" or "good". In some instances, the therapeutic efficacy of rVWF administration is defined as "moderate".

In some embodiments, a therapeutic efficacy rating of "excellent" for treatment of minor and/or moderate menorrhagia episodes is indicated when the number of infusions is equal to or less than the estimated number of infusions required to treat a comparable episode in a subject who does not have Type 1, Type 2A, Type 2B, Type 2M, Type 2N, or Type 3 VWD and when an additional VWF-containing coagulation factor therapy is not needed to resolve the menorrhagia episode. In some embodiments, a treatment efficacy rating of "good" for treatment of minor and moderate menorrhagia episodes is indicated when 1-2 more additional infusions are needed to resolve an episode compared to the number required to treat a comparable episode in a subject who does not have Type 1, Type 2A, Type 2B, Type 2M, Type 2N, or Type 3 VWD and when an additional VWF-containing coagulation factor therapy is not needed to resolve the menorrhagia episode. In some embodiments, a treatment efficacy rating of "moderate" for treatment of minor and moderate menorrhagia episodes is indicated when 3 or more additional infusions are needed to resolve an episode compared to the number required to treat a comparable episode in a subject who does not have Type 1, Type 2A, Type 2B, Type 2M, Type 2N, or Type 3 VWD and when an additional VWF-containing coagulation factor therapy is not needed to resolve the menorrhagia episode. In some embodiments, a treatment efficacy rating of "none" for treatment of minor and moderate menorrhagia episodes is indicated when severe and uncontrolled bleeding is unchanged by rVWF treatment and an additional VWF-containing coagulation factor therapy is required.

In some embodiments, a treatment efficacy rating of "excellent" for treatment of major or severe menorrhagia episodes is indicated when the number of infusions is equal to or less than the estimated number of infusions required to treat a comparable episode in a subject who does not have Type 1, Type 2A, Type 2B, Type 2M, Type 2N, or Type 3 VWD and when an additional VWF-containing coagulation factor therapy is not needed to resolve the menorrhagia episode. In some embodiments, a treatment efficacy rating of "good" for treatment of major or severe menorrhagia episodes is indicated when less than 1.5-fold more infusions are needed to resolve an episode compared to the number required to treat a comparable episode in a subject who does not have Type 1, Type 2A, Type 2B, Type 2M, Type 2N, or Type 3 VWD and when an additional VWF-containing coagulation factor therapy is not needed to resolve the menorrhagia episode. In some embodiments, a treatment efficacy rating of "moderate" for treatment of major or severe menorrhagia episodes is indicated when at least 1.5-fold (*e.g*., ≥ 1.5-fold) more infusions are needed to resolve an episode compared to the number required to treat a comparable episode in a subject who does not have Type 1, Type 2A, Type 2B, Type 2M, Type 2N, or Type 3 VWD and when an additional VWF-containing coagulation factor therapy is not needed to resolve the menorrhagia episode.

In some embodiments, a treatment efficacy rating of "none" for treatment of major or severe menorrhagia episodes is indicated when severe and uncontrolled bleeding or the intensity of the bleeding is unchanged by rVWF treatment and an additional VWF-containing coagulation factor therapy is required.

Treatment efficacy can also be assessed using one or more of the following measures that are useful for evaluating menorrhagia: menstrual bleeding by PBAC, cycle severity, cycle duration, and health-related quality-of-life (HRQoL) questionnaires, including Rand Short Form 36-Question Health Survey (SF-36), Ruta Menorrhagia Severity Scale, Center for Disease Control Health-Related Quality of Life-14 Question Form (CDC-HRQoL14), and Center for Epidemiologic Studies Depression Scale (CES-D).

### III. Recombinant von Willebrand Factor (rVWF)

The present invention utilizes compositions comprising rVWF for treating a subject with severe VWD who has experienced or is experiencing a menorrhagia bleeding episode. In some embodiments, the composition comprising rVWF is for preventing the onset of a menorrhagia bleeding episode in a subject with severe VWD.

In certain embodiments, VWF proteins of the invention may comprise a construct, such as those described in WO 1986/06096 and US 8,597,910 for producing recombinant VWF. The useful VWF provided includes all potential forms, including the monomeric and multimeric forms. One particularly useful form of VWF is a homo-multimer of at least two VWFs. The VWF proteins may be either a biologically active derivative, or when to be used solely as a stabilizer for FVIII the VWF, may be of a form not biologically active. It should also be understood that the present invention encompasses different forms of VWF to be used in combination. For example, a composition useful for the present invention may include different multimers, different derivatives and both biologically active derivatives and derivatives not biologically active.

An advantage of the rVWF compositions provided over pdVWF is that rVWF exhibits a higher specific activity than pdVWF. The specific activity of pdVWF can range from about 40 mU/µg to about 70 mU/µg (*i.e.*, about 40 IU VWF:RCo/mg protein to about 70 IU VWF:RCo/mg protein). In some embodiments, the rVWF of the invention has a specific activity of at least about 20, 22.5, 25, 27.5, 30, 32.5, 35, 37.5, 40, 42.5, 45, 47.5, 50, 52.5, 55, 57.5, 60, 62.5, 65, 67.5, 70, 72.5, 75, 77.5, 80, 82.5, 85, 87.5, 90, 92.5, 95, 97.5, 100, 105, 110, 115, 120, 125, 130, 135, 140, 145, 150 or more mU/µg. In some embodiments, the rVWF of the invention has a specific activity of between about 20 to about 30 mU/µg. In some embodiments, the rVWF of the invention has a specific activity of between about 30 to about 40 mU/µg. In some embodiments, the rVWF of the invention has a specific activity of between about 40 to about 50 mU/µg. In some embodiments, the rVWF of the invention has a specific activity of between about 50 to about 60 mU/µg. In some embodiments, the rVWF of the invention has a specific activity of between about 60 to about 70 mU/µg. In some embodiments, the rVWF of the invention has a specific activity of between about 70 to about 80 mU/µg. In some embodiments, the rVWF of the invention has a specific activity of between about 80 to about 90 mU/µg. In some embodiments, the rVWF of the invention has a specific activity of between about 90 to about 100 mU/µg. In some embodiments, the rVWF of the invention has a specific activity of between about 100 to about 110 mU/µg. In some embodiments, the rVWF of the invention has a specific activity of between about 110 to about 120 mU/µg. In some embodiments, the rVWF of the invention has a specific activity of between about 120 to about 130 mU/µg. In some embodiments, the rVWF of the invention has a specific activity of between about 130 to about 140 mU/µg. In some embodiments, the rVWF of the invention has a specific activity of between about 140 to about 150 mU/µg. In some embodiments, the rVWF of the invention has a specific activity of more than about 150 mU/µg.

In some embodiments, the rVWF purified according to the methods outlined herein has a specific activity of at least about 20, 22.5, 25, 27,5, 30, 32.5, 35, 37.5, 40, 42.5, 45, 47.5, 50, 52.5, 55, 57.5, 60, 62.5, 65, 67.5, 70, 72.5, 75, 77.5, 80, 82.5, 85, 87.5, 90, 92.5, 95, 97.5, 100, 105, 110, 1 15, 120, 125, 130, 135, 140, 145, 150 or more mU/µg. In some embodiments, rVWF used in the methods described herein has a specific activity of from 20 mU/µg to 150 mU /µg. In some embodiments, the rVWF has a specific activity of from 30 mU/µg to 120 mU/µg. In some embodiments, the rVWF has a specific activity from 40 mU/µg to 90 mU/µg. In some embodiments, the rVWF has a specific activity selected from variations 1 to 133 found in Table 1 below which is a reproduction of Table 1 of WO 2012/171031.

**Table 1. Exemplary embodiments for the specific activity of rVWF found in the compositions and used in the methods provided herein.**

| (mU/µg) | | (mU/µg) | | (mU/µg) | | (mU/µg) | |
|---|---|---|---|---|---|---|---|
| 20 | Var. 1 | 110 | Var. 35 | 40-150 | Var. 68 | 70-120 | Var. 101 |
| 22.5 | Var. 2 | 115 | Var. 36 | 40-140 | Var. 69 | 70-110 | Var. 102 |
| 25 | Var. 3 | 120 | Var. 37 | 40-130 | Var. 70 | 70-100 | Var. 103 |
| 27.5 | Var. 4 | 125 | Var. 38 | 40-120 | Var. 71 | 70-90 | Var. 104 |
| 30 | Var. 5 | 130 | Var. 39 | 40-110 | Var. 72 | 70-80 | Var. 105 |
| 32.5 | Var. 6 | 135 | Var. 40 | 40-100 | Var. 73 | 80-150 | Var. 106 |
| 35 | Var. 7 | 140 | Var. 41 | 40-90 | Var. 74 | 80-140 | Var. 107 |
| 37.5 | Var. 8 | 145 | Var. 42 | 40-80 | Var. 75 | 80-130 | Var. 108 |
| 40 | Var. 9 | 150 | Var. 43 | 40-70 | Var. 76 | 80-120 | Var. 109 |
| 42.5 | Var. 10 | 20-150 | Var. 44 | 40-60 | Var. 77 | 80-110 | Var. 110 |
| 45 | Var. 11 | 20-140 | Var. 45 | 40-50 | Var. 78 | 80-100 | Var. 111 |
| 47.5 | Var. 12 | 20-130 | Var. 46 | 50-150 | Var. 79 | 80-90 | Var. 112 |
| 50 | Var. 13 | 20-120 | Var. 47 | 50-140 | Var. 80 | 90-150 | Var. 113 |
| 52.5 | Var. 14 | 20-110 | Var. 48 | 50-130 | Var. 81 | 90-140 | Var. 114 |
| 55 | Var. 15 | 20-100 | Var. 49 | 50-120 | Var. 82 | 90-130 | Var. 115 |
| 57.5 | Var. 16 | 20-90 | Var. 50 | 50-110 | Var. 83 | 90-120 | Var. 116 |
| 60 | Var. 17 | 20-80 | Var. 51 | 50-100 | Var. 84 | 90-110 | Var. 117 |
| 62.5 | Var. 18 | 20-70 | Var. 52 | 50-90 | Var. 85 | 90-100 | Var. 118 |
| 65 | Var. 19 | 20-60 | Var. 53 | 50-80 | Var. 86 | 100-150 | Var. 119 |
| 67.5 | Var. 20 | 20-50 | Var. 54 | 50-70 | Var. 87 | 100-140 | Var. 120 |
| 70 | Var. 21 | 20-40 | Var. 55 | 50-60 | Var. 88 | 100-130 | Var. 121 |
| 72.5 | Var. 22 | 30-150 | Var. 56 | 60-150 | Var. 89 | 100-120 | Var. 122 |
| 75 | Var. 23 | 30-140 | Var. 57 | 60-140 | Var. 90 | 100-110 | Var. 123 |
| 77.5 | Var. 24 | 30-130 | Var. 58 | 60-130 | Var. 91 | 110-150 | Var. 124 |
| 80 | Var. 25 | 30-120 | Var. 59 | 60-120 | Var. 92 | 110-140 | Var. 125 |
| 82.5 | Var. 26 | 30-110 | Var. 60 | 60-110 | Var. 93 | 110-130 | Var. 126 |
| 85 | Var. 27 | 30-100 | Var. 61 | 60-100 | Var. 94 | 110-120 | Var. 127 |
| 87.5 | Var. 28 | 30-90 | Var. 62 | 60-90 | Var. 95 | 120-150 | Var. 128 |
| 90 | Var. 29 | 30-80 | Var. 63 | 60-80 | Var. 96 | 120-140 | Var. 129 |
| 92.5 | Var. 30 | 30-70 | Var. 64 | 60-70 | Var. 97 | 120-130 | Var. 130 |
| 95 | Var. 31 | 30-60 | Var. 65 | 70-150 | Var. 98 | 130-150 | Var. 131 |
| 97.5 | Var. 32 | 30-50 | Var. 66 | 70-140 | Var. 99 | 130-140 | Var. 132 |
| 100 | Var. 33 | 30-40 | Var. 67 | 70-130 | Var. 100 | 140-150 | Var. 133 |
| 105 | Var. 34 | | | | | | |

| | | | | | | | |
|---|---|---|---|---|---|---|---|
| Var. = Variation | | | | | | | |

The rVWF composition outlined herein is highly multimeric comprising about 10 to about 40 subunits. In some embodiments, the multimeric rVWF produced using methods outlined below comprise about 10-30, 12-28, 14-26, 16-24, 18-22, 20-21 subunits. In further embodiments, the rVWF is present in multimers varying in size from dimers to multimers of over 40 subunits (>10 million Daltons). The largest multimers provide multiple binding sites that can interact with both platelet receptors and subendothelial matrix sites of injury and are the most hemostatically active form of VWF. Application of ADAMTS13 will cleave the ultra-large rVWF multimers over time, but during production (generally through expression in cell culture), rVWF compositions of the present invention are generally not exposed to ADAMTS13 and retain their highly multimeric structure. Additional details relating to VWF multimers are found in the sections below.

In specific aspects, the rVWF is not modified with any conjugation, post-translation or covalent modifications. In particular embodiments, the rVWF of the present invention is not modified with a water soluble polymer, including without limitation, a polyethylene glycol (PEG), a polypropylene glycol, a polyoxyalkylene, a polysialic acid, hydroxyl ethyl starch, a poly-carbohydrate moiety, and the like.

In other aspects, the rVWF is modified through conjugation, post-translation modification, or covalent modification, including modifications of the N- or C-terminal residues as well as modifications of selected side chains, for example, at free sulfhydryl-groups, primary amines, and hydroxyl-groups. In one embodiment, a water-soluble polymer is linked to the protein (directly or via a linker) by a lysine group or other primary amine. In one embodiment, the rVWF proteins of the present invention may be modified by conjugation of a water soluble polymer, including without limitation, a polyethylene glycol (PEG), a polypropylene glycol, a polyoxyalkylene, a polysialic acid, hydroxyl ethyl starch, a poly-carbohydrate moiety, and the like.

Water-soluble polymers that may be used to modify the rVWF and/or FVIII include linear and branched structures. The conjugated polymers may be attached directly to the coagulation proteins of the invention, or alternatively may be attached through a linking moiety. Non-limiting examples of protein conjugation with water-soluble polymers can be found in U.S. Pat. Nos. 4,640,835; 4,496,689; 4,301,144; 4,670,417; 4,791,192, and 4,179,337, as well as in Abuchowski and Davis "Enzymes as Drugs," Holcenberg and Roberts, Eds., pp. 367 383, John Wiley and Sons, New York (1981), and Hermanson G., Bioconjugate Techniques 2nd Ed., Academic Press, Inc. 2008.

### IV. Production of Recombinant VWF

The free mature recombinant von Willebrand Factor (rVWF) of the present invention can be produced recombinantly. One skilled in the art recognizes useful methods for expressing a recombinant protein in a host cell. In some instances, the method includes expressing a nucleic acid sequence encoding rVWF in a host cell such as a CHO cell and culturing the resulting host cell under certain conditions to produce rVWF, prepro-VWF, pro-VWF, and the like.

In certain embodiments, the nucleic acid sequence comprising a sequence coding for VWF can be an expression vector. The vector can be delivered by a virus or can be a plasmid. The nucleic acid sequence coding for the protein can be a specific gene or a biologically functional part thereof. In one embodiment, the protein is at least a biologically active part of VWF. The nucleic acid sequence can further comprise other sequences suitable for a controlled expression of a protein such as promoter sequences, enhancers, TATA boxes, transcription initiation sites, polylinkers, restriction sites, poly-A-sequences, protein processing sequences, selection markers, and the like which are generally known to a person of ordinary skill in the art.

A wide variety of vectors can be used for the expression of the VWF and can be selected from eukaryotic expression vectors. Examples of vectors for eukaryotic expression include: (i) for expression in yeast, vectors such as pAO, pPIC, pYES, pMET, using promoters such as AOX1, GAP, GAL1, AUG1, etc; (ii) for expression in insect cells, vectors such as pMT, pAc5, pIB, pMIB, pBAC, etc., using promoters such as PH, p10, MT, Ac5, OpIE2, gp64, polh, etc., and (iii) for expression in mammalian cells, vectors such as pSVL, pCMV, pRc/RSV, pcDNA3, pBPV, etc., and vectors derived from viral systems such as vaccinia virus, adeno-associated viruses, herpes viruses, retroviruses, etc., using promoters such as CMV, SV40, EF-1, UbC, RSV, ADV, BPV, and β -actin.

In some embodiments, the rVWF used in the methods of the present invention is produced by expression in a mammalian cell culture using methods known in the art. In particular embodiments, the mammalian culture comprises CHO cells. In further embodiments, the rVWF is co-expressed with recombinant Factor VIII (rFVIII) in the same culture. In such embodiments, the rVWF and the rFVIII are purified together (co-purified) or separately using methods known in the art. In other embodiments, the rVWF is expressed in a culture that does not contain rFVIII.

In some embodiments, rVWF is expressed and isolated from a suitable eukaryotic host system. Examples of eukaryotic cells include, without limitation, mammalian cells, such as CHO, COS, HEK 293, BHK, SK-Hep, and HepG2; insect cells, *e.g.,* SF9 cells, SF21 cells, S2 cells, and High Five cells; and yeast cells, e.g., Saccharomyces or Schizosaccharomyces cells. In one embodiment, the VWF can be expressed in yeast cells, insect cells, avian cells, mammalian cells, and the like. For example, in a human cell line, a hamster cell line, or a murine cell line. In one particular embodiment, the cell line is a CHO, BHK, or HEK cell line. Typically, mammalian cells, *e.g.,* CHO cell from a continuous cell line, can be used to express the VWF of the present invention. In certain instances, VWF protein is expressed and isolated from a CHO cell expression system.

VWF can be produced in a cell culture system or according to any cell culture method recognized by those in the art. In some embodiments, the cell cultures can be performed in large bioreactors under conditions suitable for providing high volume-specific culture surface areas to achieve high cell densities and protein expression. One means for providing such growth conditions is to use microcarriers for cell-culture in stirred tank bioreactors. The concept of cell-growth on microcarriers was first described by van Wezel (van Wezel, A. L., Nature, 1967, 216:64-5) and allows for cell attachment on the surface of small solid particles suspended in the growth medium. These methods provide for high surface-to-volume ratios and thus allow for efficient nutrient utilization. Furthermore, for expression of secreted proteins in eukaryotic cell lines, the increased surface-to-volume ratio allows for higher levels of secretion and thus higher protein yields in the supernatant of the culture. Finally, these methods allow for the easy scale-up of eukaryotic expression cultures.

The cells expressing VWF can be bound to a spherical or a porous microcarrier during cell culture growth. The microcarrier can be a microcarrier selected from the group of microcarriers based on dextran, collagen, plastic, gelatin and cellulose and others as described in Butler (1988. In: Spier & Griffiths, Animal Cell Biotechnology 3:283-303). I t is also possible to grow the cells to a biomass on spherical microcarriers and subculture the cells when they have reached final fermenter biomass and prior to production of the expressed protein on a porous microcarrier or vice versa. Suitable spherical microcarriers can include smooth surface microcarriers, such as Cytodex^{™} 1, Cytodex^{™} 2, and Cytodex^{™} 3 (GE Healthcare) and macroporous microcarriers such as Cytopore^{™} 1, Cytopore^{™} 2, Cytoline^{™} 1, and Cytoline^{™} 2 (GE Healthcare).

In a further embodiment, the VWF propeptide is cleaved from the non-mature VWF in vitro through exposure of the pro-VWF to furin. In some embodiments, the furin used for propeptide cleavage is recombinant furin.

In certain embodiments, rVWF is expressed in cells cultured in cell culture media that produces high molecular weight rVWF. The terms "cell culture solution, " "cell culture medium or media," and "cell culture supernatant" refer to aspects of cell culture processes generally well known in the art. In the context of the present invention, a cell culture solution can include cell culture media and cell culture supernatant. The cell culture media are externally added to the cell culture solution, optionally together with supplements, to provide nutrients and other components for culturing the cells expressing VWF. The cell culture supernatant refers to a cell culture solution comprising the nutrients and other components from the cell culture medium as well as products released, metabolized, and/or excreted from the cells during culture. In further embodiments, the media can be animal protein-free and chemically defined. Methods of preparing animal protein-free and chemically defined culture media are known in the art, for example in US 2006/0094104, US 2007/0212770, and US 2008/0009040, which are both incorporated herein for all purposes and in particular for all teachings related to cell culture media. "Protein free" and related terms refers to protein that is from a source exogenous to or other than the cells in the culture, which naturally shed proteins during growth. In another embodiment, the culture medium is polypeptide free. In another embodiment, the culture medium is serum free. In another embodiment the culture medium is animal protein free. In another embodiment, the culture medium is animal component free. In another embodiment, the culture medium contains protein, *e.g*., animal protein from serum such as fetal calf serum. In another embodiment, the culture has recombinant proteins exogenously added. In another embodiment, the proteins are from a certified pathogen free animal. The term "chemically defined" as used herein shall mean, that the medium does not comprise any undefined supplements, such as, for example, extracts of animal components, organs, glands, plants, or yeast. Accordingly, each component of a chemically defined medium is accurately defined. In a preferred embodiment, the media are animal-component free and protein free.

In certain embodiments, the culture of cells expressing VWF can be maintained for at least about 7 days, or at least about 14 days, 21 days, 28 days, or at least about 5 weeks, 6 weeks, 7 weeks, or at least about 2 months, or 3, 4, 5, 6, 7, 8, 9, 10, 11, 12, 13, 14, 15, 16, 17, 18 months or longer. The cell density at which a cell-culture is maintained at for production of a recombinant VWF protein will depend upon the culture-conditions and medium used for protein expression. One of skill in the art will readily be able to determine the optimal cell density for a cell-culture producing a VWF. In one embodiment, the culture is maintained at a cell density of between about 0.5x10⁶ and 4x10⁷ cells/ml for an extended period of time. In other embodiments, the cell density is maintained at a concentration of between about 1.0x10⁶ and about 1.0x10⁷ cells/ml for an extended period of time. In other embodiments, the cell density is maintained at a concentration of between about 1.0x10⁶ and about 4.0x10⁶ cells/ml for an extended period of time. In other embodiments, the cell density is maintained at a concentration of between about 1.0x10⁶ and about 4.0x10⁶ cells/ml for an extended period of time. In yet other embodiments, the cell density may be maintained at a concentration between about 2.0x10⁶ and about 4.0x10⁶, or between about 1.0x10⁶ and about 2.5x10⁶, or between about 1.5x10⁶ and about 3.5x10⁶, or any other similar range, for an extended period of time. After an appropriate time in cell culture, the rVWF can be isolated from the expression system using methods known in the art.

In a specific embodiment, the cell density of the continuous cell culture for production of rVWF is maintained at a concentration of no more than 2.5x10⁶ cells/mL for an extended period. In other specific embodiments, the cell density is maintained at no more than 2.0x10⁶ cells/mL, 1.5x10⁶ cells/mL, 1.0x10⁶ cells/mL, 0.5x10⁶ cells/mL, or less. In one embodiment, the cell density is maintained at between 1.5x10⁶ cells/mL and 2.5x10⁶ cells/mL.

In one embodiment of the cell cultures described above, the cell culture solution comprises a medium supplement comprising copper. Such cell culture solutions are described, for example, in U.S. Patent No. 8,852,888 and U.S. Patent No. 9,409,971, which is hereby incorporated by reference in its entirety for all purposes and in particular for all teachings related to cell culture methods and compositions for producing recombinant VWF.

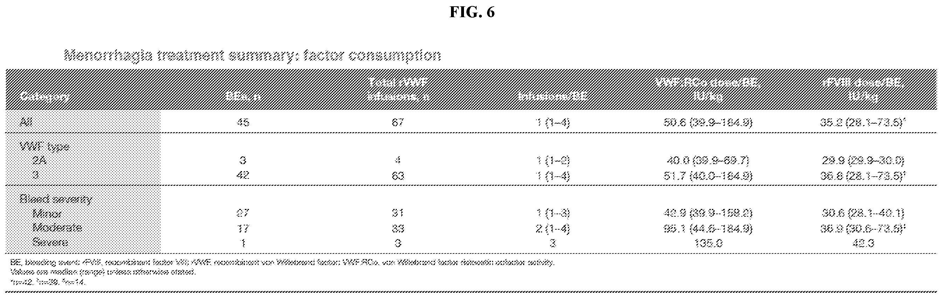

The polynucleotide and amino acid sequences of prepro-VWF are set out in SEQ ID NO:1 and SEQ ID NO:2, respectively, and are available at GenBank Accession Nos. NM_000552 (Homo sapiens von Willebrand factor (VWF) mRNA) and NP_000543, respectively. The amino acid sequence corresponding to the mature VWF protein is set out in SEQ ID NO:3 (corresponding to amino acids 764-2813 of the full length prepro-VWF amino acid sequence). In some embodiments, the VWF exhibits at least 80%, at least 85%, at least 90%, at least 95%, at least 96%, at least 97%, at least 98%, at least 99%, or at least 100% identity to the sequence of SEQ ID NO:3. In some embodiments, the rVWF of the invention exhibits at least 80%, at least 85%, at least 90%, at least 95%, at least 96%, at least 97%, at least 98%, at least 99%, or at least 100% identity to the sequence of SEQ ID NO:3. *See*, for example, U.S. Patent No. 8,597,910, U.S. Patent Publication No. 2016/0129090, as well as FIG. 6.

One form of useful rVWF has at least the property of in vivo-stabilizing, *i.e*., binding, of at least one Factor VIII (FVIII) molecule and having optionally a glycosylation pattern which is pharmacologically acceptable. Specific examples thereof include VWF without the A2 domain thus resistant to proteolysis (Lankhof et al., Thromb. Haemost. 77: 1008-1013, 1997), and a VWF fragment from Val 449 to Asn 730 including the glycoprotein lb-binding domain and binding sites for collagen and heparin (Pietu et al., Biochem. Biophys. Res. Commun. 164: 1339-1347, 1989). The determination of the ability of a VWF to stabilize at least one FVIII molecule is, in one aspect, carried out in VWF-deficient mammals according to methods known in the state in the art.

The rVWF of the present invention can be produced by any method known in the art. Thus, methods are known in the art for (i) the production of recombinant DNA by genetic engineering, *e.g*., via reverse transcription of RNA and/or amplification of DNA, (ii) introducing recombinant DNA into prokaryotic or eukaryotic cells by transfection, *e.g*., via electroporation or microinjection, (iii) cultivating the transformed cells, *e.g.* in a continuous or batchwise manner, (iv) expressing VWF, *e.g*., constitutively or upon induction, and (v) isolating the VWF, *e.g*., from the culture medium or by harvesting the transformed cells, in order to (vi) obtain purified rVWF, *e.g*., via anion exchange chromatography or affinity chromatography. A recombinant VWF is, in one aspect, made in transformed host cells using recombinant DNA techniques well known in the art. For instance, sequences coding for the polypeptide could be excised from DNA using suitable restriction enzymes. Alternatively, the DNA molecule is, in another aspect, synthesized using chemical synthesis techniques, such as the phosphoramidate method. Also, in still another aspect, a combination of these techniques is used.

The invention also provides vectors encoding polypeptides of the invention in an appropriate host. The vector comprises the polynucleotide that encodes the polypeptide operatively linked to appropriate expression control sequences. Methods of effecting this operative linking, either before or after the polynucleotide is inserted into the vector, are well known. Expression control sequences include promoters, activators, enhancers, operators, ribosomal binding sites, start signals, stop signals, cap signals, polyadenylation signals, and other signals involved with the control of transcription or translation. The resulting vector having the polynucleotide therein is used to transform an appropriate host. This transformation may be performed using methods well known in the art.

Any of a large number of available and well-known host cells are used in the practice of this invention. The selection of a particular host is dependent upon a number of factors recognized by the art, including, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all host cells are equally effective for the expression of a particular DNA sequence. Within these general guidelines, useful microbial host cells include, without limitation, bacteria, yeast and other fungi, insects, plants, mammalian (including human) cells in culture, or other hosts known in the art.

Transformed host cells are cultured under conventional fermentation conditions so that the desired compounds are expressed. Such fermentation conditions are well known in the art. Finally, the polypeptides are purified from culture media or the host cells themselves by methods well known in the art.

Depending on the host cell utilized to express a compound of the invention, carbohydrate (oligosaccharide) groups are optionally attached to sites that are known to be glycosylation sites in proteins. Generally, O-linked oligosaccharides are attached to serine (Ser) or threonine (Thr) residues while N-linked oligosaccharides are attached to asparagine (Asn) residues when they are part of the sequence Asn-X-Ser/Thr, where X can be any amino acid except proline. X is preferably one of the 19 naturally occurring amino acids not counting proline. The structures of N-linked and O-linked oligosaccharides and the sugar residues found in each type are different. One type of sugar that is commonly found on both N-linked and O-linked oligosaccharides is N-acetylneuraminic acid (referred to as sialic acid). Sialic acid is usually the terminal residue of both N-linked and O-linked oligosaccharides and, by virtue of its negative charge, in one aspect, confers acidic properties to the glycosylated compound. Such site(s) may be incorporated in the linker of the compounds of this invention and are preferably glycosylated by a cell during recombinant production of the polypeptide compounds (*e.g*., in mammalian cells such as CHO, BHK, COS). In other aspects, such sites are glycosylated by synthetic or semi- synthetic procedures known in the art.

In some embodiments, sialysation (also referred to as sialylation), can be performed on the column as part of the purification procedures described herein (including the anion exchange, cation exchange, size exclusion, and/or immunoaffinity methods). In some embodiments, the sialylation results in increased stability of the rVWF as compared to rVWF that has not undergone sialylation. In some embodiments, the sialylation results in increased stability of the rVWF in blood circulation (for example, after administration to a subject) as compared to rVWF that has not undergone sialylation. In some embodiments, the increased stability of sialylated rVWF results in an increase of 10%, 20%, 30%, 40%, 50%, 60%, 70%, 80%, 90%, or more as compared rVWF that has not undergone sialylation. In some embodiments, the sialylation results in increased half-life for the rVWF as compared to rVWF that has not undergone sialylation. In some embodiments, the sialylation results in increased half-life for the rVWF in blood circulation (for example, after administration to a subject) as compared to rVWF that has not undergone sialylation. In some embodiments, the increased half-life of sialylated rVWF results in an increase of 10%, 20%, 30%, 40%, 50%, 60%, 70%, 80%, 90%, or more as compared rVWF that has not undergone sialylation. In some embodiments, the increased half-life of sialylated rVWF results in rVWF that is stable for 1 hour, 2 hours, 3 hours, 4 hours, 6 hours, 12 hours, 24 hours or more in blood circulation (for example, after administration to a subject) as compared to rVWF that has not undergone sialylation. In some embodiments, sialylation increases the number of 2,3 sialylation and/or 2,6 sialylation. In some embodiments, sialylation is increased by the addition of 2,3 sialyltransferase and/or 2,6 sialyltransferase and CMP-NANA (Cytidine-5'-monophospho-N-acetylneuraminic acid sodium salt) as an additional buffer step. In some embodiments, sialylation is increased by the addition of 2,3 sialyltransferase and CMP-NANA (Cytidine-5'-monophospho-N-acetylneuraminic acid sodium salt) as an additional buffer step. In some embodiments, 2,3 sialylation is increased by the addition of 2,3 sialyltransferase and CMP-NANA (Cytidine-5'-monophospho-N-acetylneuraminic acid sodium salt) as an additional buffer step.

In some embodiments, 2,6 sialylation is increased by the addition of 2,6 sialyltransferase and CMP-NANA (Cytidine-5'-monophospho-N-acetylneuraminic acid sodium salt) as an additional buffer step. In some embodiments, 2,3 sialylation and/or 2,6 sialylation are increased by the addition of 2,3 sialyltransferase and/or 2,6 sialyltransferase and CMP-NANA (Cytidine-5'-monophospho-N-acetylneuraminic acid sodium salt) as an additional buffer step. In some embodiments, CMP-NANA is chemically or enzymatic modified to transfer modified sialic acid to potential free position. In some embodiments, sialylation is performed by loading rVWF onto the resin, washing with one or more buffers as described herein to deplete unwanted impurities, apply one or more buffers containing sialyltransferase and CMP-NANA at conditions that allow additional sialylation, and washing with one or more buffers to deplete excess of the sialylation reagents, and eluting with one or more buffers the enhanced rVWF (e.g., the rVWF with increased sialylation). In some embodiments, the sialylation process is performed as part of a cation exchange method, an anion exchange method, a size exclusion method, or an immunoaffinity purification method, as described herein.

Alternatively, the compounds are made by synthetic methods using, for example, solid phase synthesis techniques. Suitable techniques are well known in the art, and include those described in Merrifield (1973), Chem. Polypeptides, pp. 335-61 (Katsoyannis and Panayotis eds.); Merrifield (1963), J. Am. Chem. Soc. 85: 2149; Davis et al. (1985), Biochem. Intl. 10: 394-414; Stewart and Young (1969), Solid Phase Peptide Synthesis; US 3,941,763; Finn et al. (1976), The Proteins (3rd ed.) 2: 105-253; and Erickson et al. (1976), The Proteins (3rd ed.) 2: 257-527. Solid phase synthesis is the preferred technique of making individual peptides since it is the most cost-effective method of making small peptides.

Fragments, variants and analogs of VWF can be produced according to methods that are well-known in the art. Fragments of a polypeptide can be prepared using, without limitation, enzymatic cleavage (e.g., cleavage by trypsin or chymotrypsin) and also using recombinant means to generate a polypeptide fragments having a specific amino acid sequence. Polypeptide fragments may be generated comprising a region of the protein having a particular activity, such as a multimerization domain or any other identifiable VWF domain known in the art.

Methods of making polypeptide analogs are also well-known. Amino acid sequence analogs of a polypeptide can be substitutional, insertional, addition or deletion analogs. Deletion analogs, including fragments of a polypeptide, lack one or more residues of the native protein which are not essential for function or immunogenic activity. Insertional analogs involve the addition of, *e.g.,* amino acid(s) at a non-terminal point in the polypeptide. This analog may include, for example and without limitation, insertion of an immunoreactive epitope or simply a single residue. Addition analogs, including fragments of a polypeptide, include the addition of one or more amino acids at either or both termini of a protein and include, for example, fusion proteins. Combinations of the aforementioned analogs are also contemplated.

Substitutional analogs typically exchange one amino acid of the wild-type for another at one or more sites within the protein, and may be designed to modulate one or more properties of the polypeptide without the complete loss of other functions or properties. In one aspect, substitutions are conservative substitutions. "Conservative amino acid substitution" is substitution of an amino acid with an amino acid having a side chain or a similar chemical character. Similar amino acids for making conservative substitutions include those having an acidic side chain (glutamic acid, aspartic acid); a basic side chain (arginine, lysine, histidine); a polar amide side chain (glutamine, asparagine); a hydrophobic, aliphatic side chain (leucine, isoleucine, valine, alanine, glycine); an aromatic side chain (phenylalanine, tryptophan, tyrosine); a small side chain (glycine, alanine, serine, threonine, methionine); or an aliphatic hydroxyl side chain (serine, threonine).

In one aspect, analogs are substantially homologous or substantially identical to the recombinant VWF from which they are derived. Analogs include those which retain at least some of the biological activity of the wild-type polypeptide, *e.g.* blood clotting activity.

Polypeptide variants contemplated include, without limitation, polypeptides chemically modified by such techniques as ubiquitination, glycosylation, including polysialation (or polysialylation), conjugation to therapeutic or diagnostic agents, labeling, covalent polymer attachment such as pegylation (derivatization with polyethylene glycol), introduction of non-hydrolyzable bonds, and insertion or substitution by chemical synthesis of amino acids such as ornithine, which do not normally occur in human proteins. Variants retain the same or essentially the same binding properties of non-modified molecules of the invention. Such chemical modification may include direct or indirect (*e.g.,* via a linker) attachment of an agent to the VWF polypeptide. In the case of indirect attachment, it is contemplated that the linker may be hydrolyzable or non-hydrolyzable.

Preparing pegylated polypeptide analogs will in one aspect comprise the steps of (a) reacting the polypeptide with polyethylene glycol (such as a reactive ester or aldehyde derivative of PEG) under conditions whereby the binding construct polypeptide becomes attached to one or more PEG groups, and (b) obtaining the reaction product(s). In general, the optimal reaction conditions for the acylation reactions are determined based on known parameters and the desired result. For example, the larger the ratio of PEG: protein, the greater the percentage of polypegylated product. In some embodiments, the binding construct has a single PEG moiety at the N-terminus. Polyethylene glycol (PEG) may be attached to the blood clotting factor to, for example, provide a longer half-life in vivo. The PEG group may be of any convenient molecular weight and is linear or branched. The average molecular weight of the PEG ranges from about 2 kiloDalton ("kD") to about 100 kDa, from about 5 kDa to about 50 kDa, or from about 5 kDa to about 10 kDa. In certain aspects, the PEG groups are attached to the blood clotting factor via acylation or reductive alkylation through a natural or engineered reactive group on the PEG moiety (e.g., an aldehyde, amino, thiol, or ester group) to a reactive group on the blood clotting factor (*e.g.,* an aldehyde, amino, or ester group) or by any other technique known in the art.

Methods for preparing polysialylated polypeptide are described in US 2006/160948, Fernandes et Gregoriadis; Biochim. Biophys. Acta 1341: 26-34, 1997, and Saenko et al., Haemophilia 12:42-51, 2006. Briefly, a solution of colominic acid (CA) containing 0.1 M NaIO₄ is stirred in the dark at room temperature to oxidize the CA. The activated CA solution is dialyzed against, *e.g.,* 0.05 M sodium phosphate buffer, pH 7.2 in the dark and this solution was added to a rVWF solution and incubated for 18 h at room temperature in the dark under gentle shaking. Free reagents are optionally be separated from the rVWF-polysialic acid conjugate by, for example, ultrafiltration/diafiltration. Conjugation of rVWF with polysialic acid is achieved using glutaraldehyde as cross-linking reagent (Migneault et al., Biotechniques 37: 790-796, 2004).

It is also contemplated in another aspect that prepro-VWF and pro-VWF polypeptides will provide a therapeutic benefit in the formulations of the present invention. For example, US Patent No. 7,005,502 describes a pharmaceutical preparation comprising substantial amounts of pro-VWF that induces thrombin generation in vitro. In addition to recombinant, biologically active fragments, variants, or other analogs of the naturally-occurring mature VWF, the present invention contemplates the use of recombinant biologically active fragments, variants, or analogs of the prepro-VWF (set out in SEQ ID NO:2) or pro-VWF polypeptides (amino acid residues 23 to 764 of SEQ ID NO: 2) in the formulations described herein.

Polynucleotides encoding fragments, variants and analogs may be readily generated by a worker of skill to encode biologically active fragments, variants, or analogs of the naturally-occurring molecule that possess the same or similar biological activity to the naturally-occurring molecule. In various aspects, these polynucleotides are prepared using PCR techniques, digestion/ligation of DNA encoding molecule, and the like. Thus, one of skill in the art will be able to generate single base changes in the DNA strand to result in an altered codon and a missense mutation, using any method known in the art, including, but not limited to site- specific mutagenesis. As used herein, the phrase "moderately stringent hybridization conditions" means, for example, hybridization at 42 ° C in 50% formamide and washing at 60°C in 0.1 x SSC, 0.1% SDS. It is understood by those of skill in the art that variation in these conditions occurs based on the length and GC nucleotide base content of the sequences to be hybridized. Formulas standard in the art are appropriate for determining exact hybridization conditions. See Sambrook et al., 9.47-9.51 in Molecular Cloning, Cold Spring Harbor Laboratory Press, Cold Spring Harbor, New York (1989).

### A. VWF Multimers

In one embodiment, an rVWF composition used in the methods described herein has a distribution of rVWF oligomers characterized in that 95% of the oligomers have between 6 subunits and 20 subunits. In some embodiments, the distribution of rVWF oligomers is characterized in that 95% of the oligomers have between 6 subunits and 10 subunits. In some embodiments, the distribution of rVWF oligomers is characterized in that 95% of the oligomers have between 10 subunits and 15 subunits. In some embodiments, the distribution of rVWF oligomers is characterized in that 95% of the oligomers have between 15 subunits and 20 subunits. In other embodiments, the rVWF composition has a distribution of rVWF oligomers characterized in that 95% of the oligomers have a range of subunits selected from variations 458 to 641 found in Table 2 below which is a reproduction of Table 2 of WO 2012/171031.

In some embodiments, the rVWF composition prepared by the purification method described herein has a distribution of rVWF oligomers characterized in that 95% of the oligomers have between 6 subunits and 20 subunits. In some embodiments, the rVWF composition has a distribution of rVWF oligomers characterized in that 95% of the oligomers have a range of subunits selected from variations 458 to 641 found in Table 2 below which is reproduced from Table 2 of WO2012/171031.

**Table 2. Exemplary embodiments for the distribution of rVWF oligomers found in the compositions and used in the methods provided herein.**

| **Subunits** | | **Subunits** | | **Subunits** | | **Subunits** | |
|---|---|---|---|---|---|---|---|
| 2-40 | Var. 458 | 6-16 | Var. 504 | 12-20 | Var. 550 | 20-28 | Var. 596 |
| 2-38 | Var. 459 | 6-14 | Var. 505 | 12-18 | Var. 551 | 20-26 | Var. 597 |
| 2-36 | Var. 460 | 6-12 | Var. 506 | 12-16 | Var. 552 | 20-24 | Var. 598 |
| 2-34 | Var. 461 | 6-10 | Var. 507 | 12-14 | Var. 553 | 20-22 | Var. 599 |
| 2-32 | Var. 462 | 6-8 | Var. 508 | 14-40 | Var. 554 | 22-40 | Var. 600 |
| 2-30 | Var. 463 | 8-40 | Var. 509 | 14-38 | Var. 555 | 22-38 | Var. 601 |
| 2-28 | Var. 464 | 8-38 | Var. 510 | 14-36 | Var. 556 | 22-36 | Var. 602 |
| 2-26 | Var. 465 | 8-36 | Var. 511 | 14-34 | Var. 557 | 22-34 | Var. 603 |
| 2-24 | Var. 466 | 8-34 | Var. 512 | 14-32 | Var. 558 | 22-32 | Var. 604 |
| 2-22 | Var. 467 | 8-32 | Var. 513 | 14-30 | Var. 559 | 22-30 | Var. 605 |
| 2-20 | Var. 468 | 8-30 | Var. 514 | 14-28 | Var. 560 | 22-28 | Var. 606 |
| 2-18 | Var. 469 | 8-28 | Var. 515 | 14-26 | Var. 561 | 22-26 | Var. 607 |
| 2-16 | Var. 470 | 8-26 | Var. 516 | 14-24 | Var. 562 | 22-24 | Var. 608 |
| 2-14 | Var. 471 | 8-24 | Var. 517 | 14-22 | Var. 563 | 24-40 | Var. 609 |
| 2-12 | Var. 472 | 8-22 | Var. 518 | 14-20 | Var. 564 | 24-38 | Var. 610 |
| 2-10 | Var. 473 | 8-20 | Var. 519 | 14-18 | Var. 565 | 24-36 | Var. 611 |
| 2-8 | Var. 474 | 8-18 | Var. 520 | 14-16 | Var. 566 | 24-34 | Var. 612 |
| 4-40 | Var. 475 | 8-16 | Var. 521 | 16-40 | Var. 567 | 24-32 | Var. 613 |
| 4-38 | Var. 476 | 8-14 | Var. 522 | 16-38 | Var. 568 | 24-30 | Var. 614 |
| 4-36 | Var. 477 | 8-12 | Var. 523 | 16-36 | Var. 569 | 24-28 | Var. 615 |
| 4-34 | Var. 478 | 8-10 | Var. 524 | 16-34 | Var. 570 | 24-26 | Var. 616 |
| 4-32 | Var. 479 | 10-40 | Var. 525 | 16-32 | Var. 571 | 26-40 | Var. 617 |
| 4-30 | Var. 480 | 10-38 | Var. 526 | 16-30 | Var. 572 | 26-38 | Var. 618 |
| 4-28 | Var. 481 | 10-36 | Var. 527 | 16-28 | Var. 573 | 26-36 | Var. 619 |
| 4-26 | Var. 482 | 10-34 | Var. 528 | 16-26 | Var. 574 | 26-34 | Var. 620 |
| 4-24 | Var. 483 | 10-32 | Var. 529 | 16-24 | Var. 575 | 26-32 | Var. 621 |
| 4-22 | Var. 484 | 10-30 | Var. 530 | 16-22 | Var. 576 | 26-30 | Var. 622 |
| 4-20 | Var. 485 | 10-28 | Var. 531 | 16-20 | Var. 577 | 26-28 | Var. 623 |
| 4-18 | Var. 486 | 10-26 | Var. 532 | 16-18 | Var. 578 | 28-40 | Var. 624 |
| 4-16 | Var. 487 | 10-24 | Var. 533 | 18-40 | Var. 579 | 28-38 | Var. 625 |
| 4-14 | Var. 488 | 10-22 | Var. 534 | 18-38 | Var. 580 | 28-36 | Var. 626 |
| 4-12 | Var. 489 | 10-20 | Var. 535 | 18-36 | Var. 581 | 28-34 | Var. 627 |
| 4-10 | Var. 490 | 10-18 | Var. 536 | 18-34 | Var. 582 | 28-32 | Var. 628 |
| 4-8 | Var. 491 | 10-16 | Var. 537 | 18-32 | Var. 583 | 28-30 | Var. 629 |
| 6-40 | Var. 492 | 10-14 | Var. 538 | 18-30 | Var. 584 | 30-40 | Var. 630 |
| 6-38 | Var. 493 | 10-12 | Var. 539 | 18-28 | Var. 585 | 30-38 | Var. 631 |
| 6-36 | Var. 494 | 12-40 | Var. 540 | 18-26 | Var. 586 | 30-36 | Var. 632 |
| 6-34 | Var. 495 | 12-38 | Var. 541 | 18-24 | Var. 587 | 30-34 | Var. 633 |
| 6-32 | Var. 496 | 12-36 | Var. 542 | 18-22 | Var. 588 | 30-32 | Var. 634 |
| 6-30 | Var. 497 | 12-34 | Var. 543 | 18-20 | Var. 589 | 32-40 | Var. 635 |
| 6-28 | Var. 498 | 12-32 | Var. 544 | 20-40 | Var. 590 | 32-38 | Var. 636 |
| 6-26 | Var. 499 | 12-30 | Var. 545 | 20-38 | Var. 591 | 32-36 | Var. 637 |
| 6-24 | Var. 500 | 12-28 | Var. 546 | 20-36 | Var. 592 | 32-34 | Var. 638 |
| 6-22 | Var. 501 | 12-26 | Var. 547 | 20-34 | Var. 593 | 34-40 | Var. 639 |
| 6-20 | Var. 502 | 12-24 | Var. 548 | 20-32 | Var. 594 | 36-38 | Var. 640 |
| 6-18 | Var. 503 | 12-22 | Var. 549 | 20-30 | Var. 595 | 38-40 | Var. 641 |

| | | | | | | | |
|---|---|---|---|---|---|---|---|
| Var. = Variation | | | | | | | |

In some embodiments, the distribution of rVWF oligomers is characterized in that 95% of the oligomers have between 460 subunits and 500 subunits. In some embodiments, the distribution of rVWF oligomers is characterized in that 95% of the oligomers have between 500 subunits and 550 subunits. In some embodiments, the distribution of rVWF oligomers is characterized in that 95% of the oligomers have between 550 subunits and 600 subunits. In some embodiments, the distribution of rVWF oligomers is characterized in that 95% of the oligomers have between 600 subunits and 640 subunits.

In one embodiment, an rVWF composition can be characterized according to the percentage of rVWF molecules that are present in a particular higher order rVWF multimer or larger multimer. For example, in one embodiment, at least 20% of rVWF molecules in a rVWF composition used in the methods described herein are present in an oligomeric complex of at least 10 subunits. In another embodiment, at least 20% of rVWF molecules in a rVWF composition used in the methods described herein are present in an oligomeric complex of at least 12 subunits. In yet other embodiments, a rVWF composition used in the methods provided herein has a minimal percentage (*e.g.*, has at least X %) of rVWF molecules present in a particular higher-order rVWF multimer or larger multimer (*e.g.,* a multimer of at least Y subunits) according to any one of variations 134 to 457 found in Tables 3-5 below which are reproductions of Table 3 to Table 5 of WO2012/171031.

In some embodiments, the rVWF composition prepared by the methods provided herein can be characterized according to the percentage of rVWF molecules that are present in a particular higher order rVWF multimer or larger multimer. For example, in one embodiment, at least 20% of rVWF molecules in a rVWF composition used in the methods described herein are present in an oligomeric complex of at least 10 subunits. In another embodiment, at least 20% of rVWF molecules in a rVWF composition used in the methods described herein are present in an oligomeric complex of at least 12 subunits. In yet other embodiments, a rVWF composition used in the methods provided herein has a minimal percentage (*e.g.,* has at least X%) of rVWF molecules present in a particular higher-order rVWF multimer or larger multimer (*e.g.,* a multimer of at least Y subunits) according to any one of variations 134 to 457 found in Tables 3 to 5 below which is reproduced from Tables 3-5 of WO2012/171031.

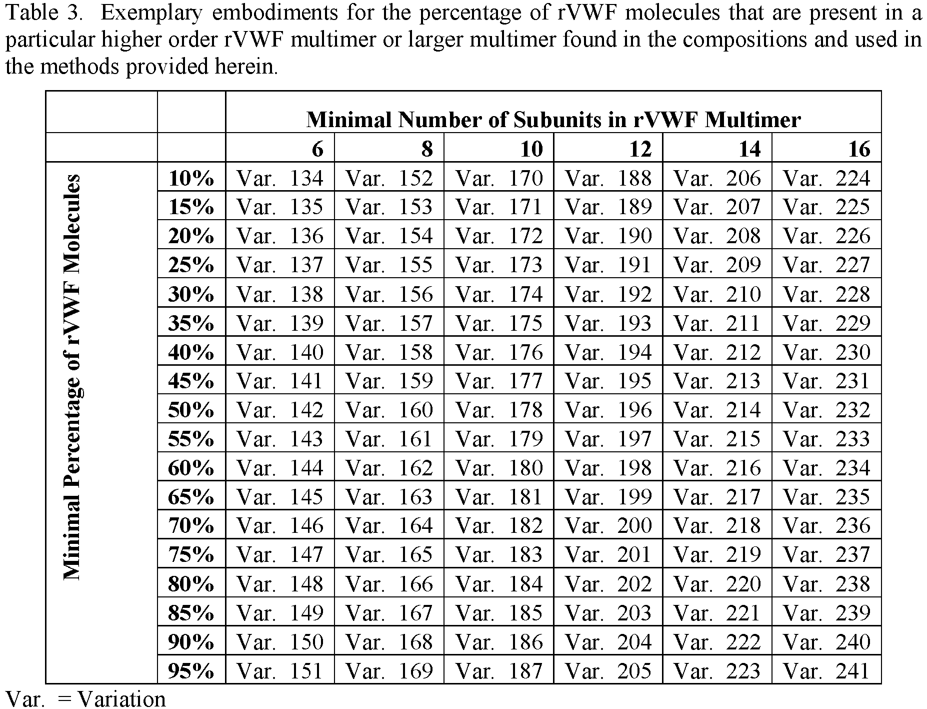

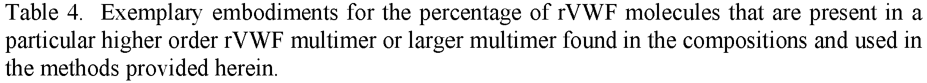

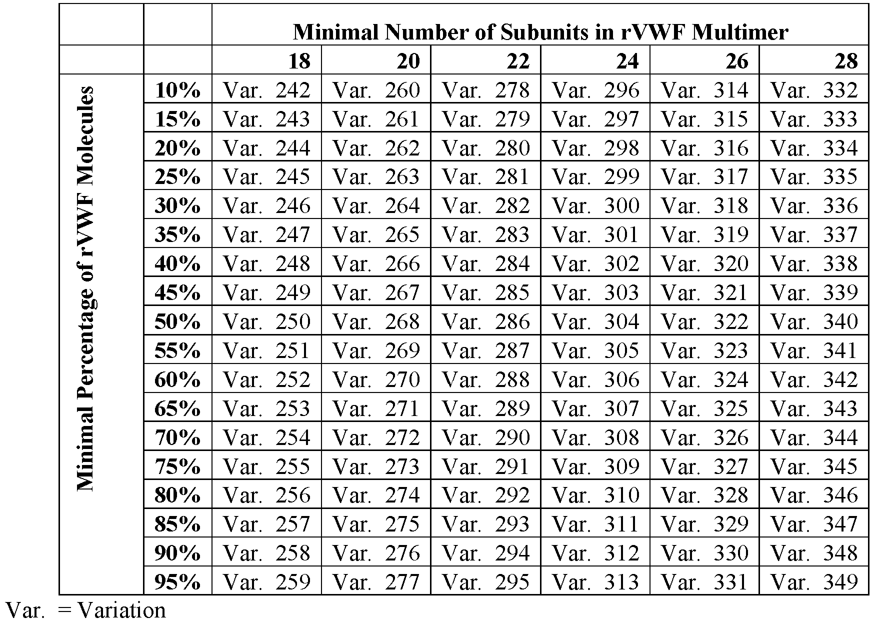

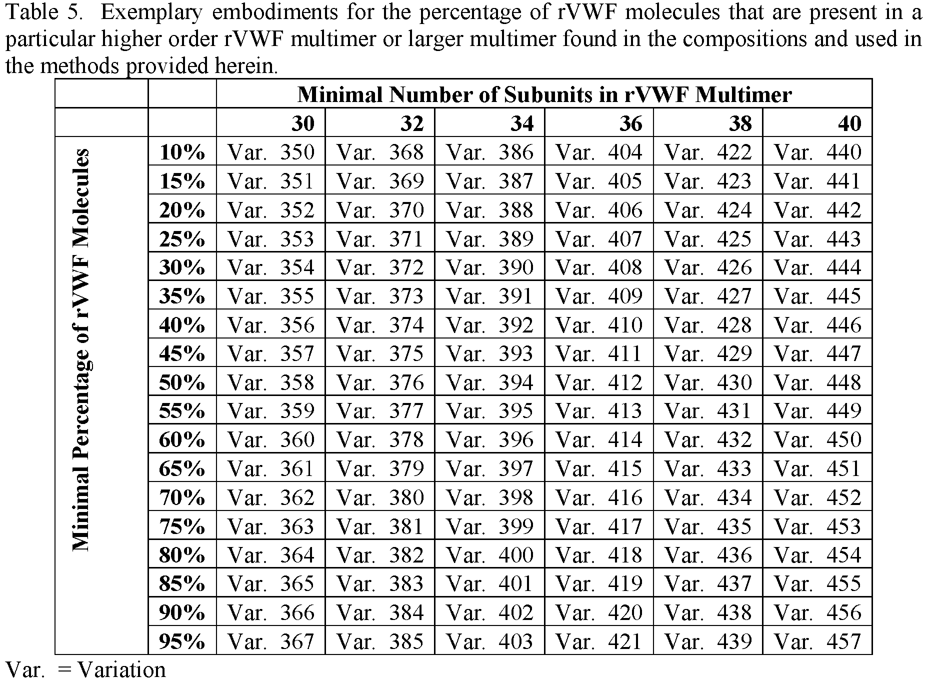

In accordance with the above, the rVWF composition administered to the subject (with or without FVIII) generally comprises a significant percentage of high molecular weight (HMW) rVWF multimers. In further embodiments, the HMW rVWF multimer composition comprises at least 10%-80% rVWF decamers or higher order multimers. In further embodiments, the composition comprises about 10-95%, 20-90%, 30-85%, 40-80%, 50-75%, 60-70% decamers or higher order multimers. In further embodiments, the HMW rVWF multimer composition comprises at least about 10%, 20%, 30%, 40%, 50%, 60%, 70%, 80%, 90% decamers or higher order multimers.

In further embodiments, higher order rVWF multimers of the invention are stable for about 1 to about 90 hours post-administration. In still further embodiments, the higher order rVWF multimers are stable for about 5-80, 10-70, 15-60, 20-50, 25-40, 30-35 hours post-administration. In yet further embodiments, the higher order rVWF multimers are stable for at least 3, 6, 12, 18, 24, 36, 48, 72 hours post-administration. In certain embodiments, the stability of the rVWF multimers is assessed in vitro.

The rVWF is highly multimeric comprising about 10 to about 40 subunits. In further embodiments, the multimeric rVWF produced using methods of the present invention comprise about 10-30, 12-28, 14-26, 16-24, 18-22, 20-21 subunits. In some embodiments, the rVWF is present in multimers varying in size from dimers to multimers of over 40 subunits (> 10 million Daltons). The largest multimers provide multiple binding sites that can interact with both platelet receptors and subendothelial matrix sites of injury and are the most hemostatically active form of VWF. In some embodiments, the rVWF of the present invention comprises ultralarge multimers (ULMs). Generally, high and ultralarge multimers are considered to be hemostatically most effective (*see,* for example, Turecek, P., Hämostaseologie, (Vol. 37): Supplement 1, pages S15-S25 (2017)). In some embodiments, the rVWF is between 500 kDa and 20,000 kDa. In some embodiments, any desired multimer pattern can be obtained using the methods described. In some embodiments, when anion exchange and/or cation exchanger methods are employed, the pH, conductivity, and/or counterion concentration of the buffers in the one or more wash step(s) or the gradient buffers can be manipulated to obtain the desired multimer pattern. In some embodiments, then size exclusion chromatography methods are employed, the collection criteria can be employed to obtain the desired multimer pattern. In some embodiments, the described multimer pattern comprises ultralarge multimers. In some embodiments, the ultralarge multimers are at least 10,000 kDa, at least 11,000 kDa, at least 12,000 kDa, at least 13,000 kDa, at least 14,000 kDa, at least 15,000 kDa, at least 16,000 kDa, at least 17,000 kDa, at least 18,000 kDa, at least 19,000 kDa, at least 20,000 kDa. In some embodiments, the ultralarge multimers are between about 10,000 kDa and 20,000 kDa. In some embodiments, the ultralarge multimers are between about 11,000 kDa and 20,000 kDa. In some embodiments, the ultralarge multimers are between about 12,000 kDa and 20,000 kDa. In some embodiments, the ultralarge multimers are between about 13,000 kDa and 20,000 kDa. In some embodiments, the ultralarge multimers are between about 14,000 kDa and 20,000 kDa. In some embodiments, the ultralarge multimers are between about 15,000 kDa and 20,000 kDa. In some embodiments, the ultralarge multimers are between about 16,000 kDa and 20,000 kDa. In some embodiments, the ultralarge multimers are between about 17,000 kDa and 20,000 kDa. In some embodiments, the ultralarge multimers are between about 18,000 kDa and 20,000 kDa. In some embodiments, the ultralarge multimers are between about 19,000 kDa and 20,000 kDa. In some embodiments, the rVWF obtained using the present methods includes any multimer pattern present in the loading sample of the rVWF. In some embodiments, the rVWF obtained using the present methods includes physiological occurring multimer patters as well as ultra large VWF-multimer patterns.

In accordance with the above, the rVWF comprises a significant percentage of high molecular weight (HMW) rVWF multimers. In further embodiments, the HMW rVWF multimer composition comprises at least 10% - 80% rVWF decamers or higher order multimers. In further embodiments, the composition comprises about 10-95%, 20-90%, 30-85%, 40-80%, 50-75%, 60-70% decamers or higher order multimers. In further embodiments, the HMW rVWF multimer composition comprises at least about 10%, 20%, 30%, 40%, 50%, 60%, 70%, 80%, 90% decamers or higher order multimers.

In further embodiments, higher order rVWF multimers of the invention are stable for about 1 to about 90 hours post-administration. In still further embodiments, the higher order rVWF multimers are stable for about 5-80, 10-70, 15-60, 20-50, 25-40, 30-35 hours post-administration. In yet further embodiments, the higher order rVWF multimers are stable for at least 3, 6, 12, 18, 24, 36, 48, 72 hours post-administration. In certain embodiments, the stability of the rVWF multimers is assessed *in vitro.*

In one embodiment, higher order rVWF multimers used in the compositions and methods provided herein have a half-life of at least 12 hour post administration. In another embodiment, the higher order rVWF multimers have a half-life of at least 24 hour post administration. In yet other embodiments, the higher order rVWF multimers have a half-life selected from variations 642 to 1045 found in Table 6 below which is a copy of Table 6 of WO2012/171031.

**Table 6. Exemplary embodiments for the half-life of higher order rVWF multimers found in the compositions prepared by the methods provided herein.**

| **Hours** | | **Hours** | | **Hours** | | **Hours** | |
|---|---|---|---|---|---|---|---|
| at least 1 | Var. 642 | 4-22 | Var. 743 | 14-78 | Var. 844 | 24-30 | Var. 945 |
| at least 2 | Var. 643 | 4-20 | Var. 744 | 14-72 | Var. 845 | 24-27 | Var. 946 |
| at least 3 | Var. 644 | 4-18 | Var. 745 | 14-66 | Var. 846 | 27-90 | Var. 947 |
| at least 4 | Var. 645 | 4-16 | Var. 746 | 14-60 | Var. 847 | 27-84 | Var. 948 |
| at least 5 | Var. 646 | 4-14 | Var. 747 | 14-54 | Var. 848 | 27-78 | Var. 949 |
| at least 6 | Var. 647 | 4-12 | Var. 748 | 14-48 | Var. 849 | 27-72 | Var. 950 |
| at least 7 | Var. 648 | 4-10 | Var. 749 | 14-45 | Var. 850 | 27-66 | Var. 951 |
| at least 8 | Var. 649 | 4-8 | Var. 750 | 14-42 | Var. 851 | 27-60 | Var. 952 |
| at least 9 | Var. 650 | 4-6 | Var. 751 | 14-39 | Var. 852 | 27-54 | Var. 953 |
| at least 10 | Var. 651 | 6-90 | Var. 752 | 14-36 | Var. 853 | 27-48 | Var. 954 |
| at least 11 | Var. 652 | 6-84 | Var. 753 | 14-33 | Var. 854 | 30-90 | Var. 955 |
| at least 12 | Var. 653 | 6-78 | Var. 754 | 14-30 | Var. 855 | 30-84 | Var. 956 |
| at least 14 | Var. 654 | 6-72 | Var. 755 | 14-27 | Var. 856 | 30-78 | Var. 957 |
| at least 16 | Var. 655 | 6-66 | Var. 756 | 14-24 | Var. 857 | 30-72 | Var. 958 |
| at least 18 | Var. 656 | 6-60 | Var. 757 | 14-22 | Var. 858 | 30-66 | Var. 959 |
| at least 20 | Var. 657 | 6-54 | Var. 758 | 14-20 | Var. 859 | 30-60 | Var. 960 |
| at least 22 | Var. 658 | 6-48 | Var. 759 | 14-18 | Var. 860 | 30-54 | Var. 961 |
| at least 24 | Var. 659 | 6-45 | Var. 760 | 14-16 | Var. 861 | 30-48 | Var. 962 |
| at least 27 | Var. 660 | 6-42 | Var. 761 | 16-90 | Var. 862 | 30-45 | Var. 963 |
| at least 30 | Var. 661 | 6-39 | Var. 762 | 16-84 | Var. 863 | 30-42 | Var. 964 |
| at least 33 | Var. 662 | 6-36 | Var. 763 | 16-78 | Var. 864 | 30-39 | Var. 965 |
| at least 36 | Var. 663 | 6-33 | Var. 764 | 16-72 | Var. 865 | 30-36 | Var. 966 |
| at least 39 | Var. 664 | 6-30 | Var. 765 | 16-66 | Var. 866 | 30-33 | Var. 967 |
| at least 42 | Var. 665 | 6-27 | Var. 766 | 16-60 | Var. 867 | 33-90 | Var. 968 |
| at least 45 | Var. 666 | 6-24 | Var. 767 | 16-54 | Var. 868 | 33-84 | Var. 969 |
| at least 48 | Var. 667 | 6-22 | Var. 768 | 16-48 | Var. 869 | 33-78 | Var. 970 |
| at least 54 | Var. 668 | 6-20 | Var. 769 | 16-45 | Var. 870 | 33-72 | Var. 971 |
| at least 60 | Var. 669 | 6-18 | Var. 770 | 16-42 | Var. 871 | 33-66 | Var. 972 |
| at least 66 | Var. 670 | 6-16 | Var. 771 | 16-39 | Var. 872 | 33-60 | Var. 973 |
| at least 72 | Var. 671 | 6-14 | Var. 772 | 16-36 | Var. 873 | 33-54 | Var. 974 |
| at least 78 | Var. 672 | 6-12 | Var. 773 | 16-33 | Var. 874 | 33-48 | Var. 975 |
| at least 84 | Var. 673 | 6-10 | Var. 774 | 16-30 | Var. 875 | 33-45 | Var. 976 |
| at least 90 | Var. 674 | 6-8 | Var. 775 | 16-27 | Var. 876 | 33-42 | Var. 977 |
| 2-90 | Var. 675 | 8-90 | Var. 776 | 16-24 | Var. 877 | 33-29 | Var. 978 |
| 2-84 | Var. 676 | 8-84 | Var. 777 | 16-22 | Var. 878 | 33-36 | Var. 979 |
| 2-78 | Var. 677 | 8-78 | Var. 778 | 16-20 | Var. 879 | 36-90 | Var. 980 |
| 2-72 | Var. 678 | 8-72 | Var. 779 | 16-18 | Var. 880 | 36-84 | Var. 981 |
| 2-66 | Var. 679 | 8-66 | Var. 780 | 18-90 | Var. 881 | 36-78 | Var. 982 |
| 2-60 | Var. 680 | 8-60 | Var. 781 | 18-84 | Var. 882 | 36-72 | Var. 983 |
| 2-54 | Var. 681 | 8-54 | Var. 782 | 18-78 | Var. 883 | 36-66 | Var. 984 |
| 2-48 | Var. 682 | 8-48 | Var. 783 | 18-72 | Var. 884 | 36-60 | Var. 985 |
| 2-45 | Var. 683 | 8-45 | Var. 784 | 18-66 | Var. 885 | 36-54 | Var. 986 |
| 2-42 | Var. 684 | 8-42 | Var. 785 | 18-60 | Var. 886 | 36-48 | Var. 987 |
| 2-39 | Var. 685 | 8-39 | Var. 786 | 18-54 | Var. 887 | 36-45 | Var. 988 |
| 2-36 | Var. 686 | 8-36 | Var. 787 | 18-48 | Var. 888 | 36-42 | Var. 989 |
| 2-33 | Var. 687 | 8-33 | Var. 788 | 18-45 | Var. 889 | 36-39 | Var. 990 |
| 2-30 | Var. 688 | 8-30 | Var. 789 | 18-42 | Var. 890 | 39-90 | Var. 991 |
| 2-27 | Var. 689 | 8-27 | Var. 790 | 18-39 | Var. 891 | 39-84 | Var. 992 |
| 2-24 | Var. 690 | 8-24 | Var. 791 | 18-36 | Var. 892 | 39-78 | Var. 993 |
| 2-22 | Var. 691 | 8-22 | Var. 792 | 18-33 | Var. 893 | 39-72 | Var. 994 |
| 2-20 | Var. 692 | 8-20 | Var. 793 | 18-30 | Var. 894 | 39-66 | Var. 995 |
| 2-18 | Var. 693 | 8-18 | Var. 794 | 18-27 | Var. 895 | 39-60 | Var. 996 |
| 2-16 | Var. 694 | 8-16 | Var. 795 | 18-24 | Var. 896 | 39-54 | Var. 997 |
| 2-14 | Var. 695 | 8-14 | Var. 796 | 18-22 | Var. 897 | 39-48 | Var. 998 |
| 2-12 | Var. 696 | 8-12 | Var. 797 | 18-20 | Var. 898 | 39-45 | Var. 999 |
| 2-10 | Var. 697 | 8-10 | Var. 798 | 20-90 | Var. 899 | 39-42 | Var. 1000 |
| 2-8 | Var. 698 | 10-90 | Var. 799 | 20-84 | Var. 900 | 42-90 | Var. 1001 |
| 2-6 | Var. 699 | 10-84 | Var. 800 | 20-78 | Var. 901 | 42-84 | Var. 1002 |
| 2-4 | Var. 700 | 10-78 | Var. 801 | 20-72 | Var. 902 | 42-78 | Var. 1003 |
| 3-90 | Var. 701 | 10-72 | Var. 802 | 20-66 | Var. 903 | 42-72 | Var. 1004 |
| 3-84 | Var. 702 | 10-66 | Var. 803 | 20-60 | Var. 904 | 42-66 | Var. 1005 |
| 3-78 | Var. 703 | 10-60 | Var. 804 | 20-54 | Var. 905 | 42-60 | Var. 1006 |
| 3-72 | Var. 704 | 10-54 | Var. 805 | 20-48 | Var. 906 | 42-54 | Var. 1007 |
| 3-66 | Var. 705 | 10-48 | Var. 806 | 20-45 | Var. 907 | 42-48 | Var. 1008 |
| 3-60 | Var. 706 | 10-45 | Var. 807 | 20-42 | Var. 908 | 42-45 | Var. 1009 |
| 3-54 | Var. 707 | 10-42 | Var. 808 | 20-39 | Var. 909 | 45-90 | Var. 1010 |
| 3-48 | Var. 708 | 10-39 | Var. 809 | 20-36 | Var. 910 | 45-84 | Var. 1011 |
| 3-45 | Var. 709 | 10-36 | Var. 810 | 20-33 | Var. 911 | 45-78 | Var. 1012 |
| 3-42 | Var. 710 | 10-33 | Var. 811 | 20-30 | Var. 912 | 45-72 | Var. 1013 |
| 3-39 | Var. 711 | 10-30 | Var. 812 | 20-27 | Var. 913 | 45-66 | Var. 1014 |
| 3-36 | Var. 712 | 10-27 | Var. 813 | 20-24 | Var. 914 | 45-60 | Var. 1015 |
| 3-33 | Var. 713 | 10-24 | Var. 814 | 20-22 | Var. 915 | 45-54 | Var. 1016 |
| 3-30 | Var. 714 | 10-22 | Var. 815 | 22-90 | Var. 916 | 45-48 | Var. 1017 |
| 3-27 | Var. 715 | 10-20 | Var. 816 | 22-84 | Var. 917 | 48-90 | Var. 1018 |
| 3-24 | Var. 716 | 10-18 | Var. 817 | 22-78 | Var. 918 | 48-84 | Var. 1019 |
| 3-22 | Var. 717 | 10-16 | Var. 818 | 22-72 | Var. 919 | 48-78 | Var. 1020 |
| 3-20 | Var. 718 | 10-14 | Var. 819 | 22-66 | Var. 920 | 48-72 | Var. 1021 |
| 3-18 | Var. 719 | 10-12 | Var. 820 | 22-60 | Var. 921 | 48-66 | Var. 1022 |
| 3-16 | Var. 720 | 12-90 | Var. 821 | 22-54 | Var. 922 | 48-60 | Var. 1023 |
| 3-14 | Var. 721 | 12-84 | Var. 822 | 22-48 | Var. 923 | 48-54 | Var. 1024 |
| 3-12 | Var. 722 | 12-78 | Var. 823 | 22-45 | Var. 924 | 54-90 | Var. 1025 |
| 3-10 | Var. 723 | 12-72 | Var. 824 | 22-42 | Var. 925 | 54-84 | Var. 1026 |
| 3-8 | Var. 724 | 12-66 | Var. 825 | 22-39 | Var. 926 | 54-78 | Var. 1027 |
| 3-6 | Var. 725 | 12-60 | Var. 826 | 22-36 | Var. 927 | 54-72 | Var. 1028 |
| 3-4 | Var. 726 | 12-54 | Var. 827 | 22-33 | Var. 928 | 54-66 | Var. 1029 |
| 4-90 | Var. 727 | 12-48 | Var. 828 | 22-30 | Var. 929 | 54-60 | Var. 1030 |
| 4-84 | Var. 728 | 12-45 | Var. 829 | 22-27 | Var. 930 | 60-90 | Var. 1031 |
| 4-78 | Var. 729 | 12-42 | Var. 830 | 22-24 | Var. 931 | 60-84 | Var. 1032 |
| 4-72 | Var. 730 | 12-39 | Var. 831 | 24-90 | Var. 932 | 60-78 | Var. 1033 |
| 4-66 | Var. 731 | 12-36 | Var. 832 | 24-84 | Var. 933 | 60-72 | Var. 1034 |
| 4-60 | Var. 732 | 12-33 | Var. 833 | 24-78 | Var. 934 | 60-66 | Var. 1035 |
| 4-54 | Var. 733 | 12-30 | Var. 834 | 24-72 | Var. 935 | 66-90 | Var. 1036 |
| 4-48 | Var. 734 | 12-27 | Var. 835 | 24-66 | Var. 936 | 66-84 | Var. 1037 |
| 4-45 | Var. 735 | 12-24 | Var. 836 | 24-60 | Var. 937 | 66-78 | Var. 1038 |
| 4-42 | Var. 736 | 12-22 | Var. 837 | 24-54 | Var. 938 | 66-72 | Var. 1039 |
| 4-39 | Var. 737 | 12-20 | Var. 838 | 24-48 | Var. 939 | 72-90 | Var. 1040 |
| 4-36 | Var. 738 | 12-18 | Var. 839 | 24-45 | Var. 940 | 72-84 | Var. 1041 |
| 4-33 | Var. 739 | 12-16 | Var. 840 | 24-42 | Var. 941 | 72-78 | Var. 1042 |
| 4-30 | Var. 740 | 12-14 | Var. 841 | 24-39 | Var. 942 | 78-90 | Var. 1043 |
| 4-27 | Var. 741 | 14-90 | Var. 842 | 24-36 | Var. 943 | 78-84 | Var. 1044 |
| 4-24 | Var. 742 | 14-84 | Var. 843 | 24-33 | Var. 944 | 84-90 | Var. 1045 |

| | | | | | | | |
|---|---|---|---|---|---|---|---|
| Var. = Variation | | | | | | | |

Assessment of the number and percentage of rVWF multimers can be conducted using methods known in the art, including without limitation methods using electrophoresis and size exclusion chromatography methods to separate VWF multimers by size, for example as discussed by Cumming et al., (J Clin Pathol., 1993 May; 46(5): 470-473, which is hereby incorporated by reference in its entirety for all purposes and in particular for all teachings related to assessment of VWF multimers). Such techniques may further include immunoblotting techniques (such as Western Blot), in which the gel is immunoblotted with a radiolabeled antibody against VWF followed by chemiluminescent detection (see, for example, Wen et al., J. Clin. Lab. Anal., 1993, 7: 317-323, which is hereby incorporated by reference in its entirety for all purposes and in particular for all teachings related to assessment of VWF multimers). In some embodiments, the rVWF obtained using the present methods includes any multimer pattern present in the loading sample of the rVWF. In some embodiments, the rVWF obtained using the present methods includes physiological occurring multimer patters as well as ultralarge VWF-multimer patterns.

### b. VWF Assays

In primary hemostasis, VWF serves as a bridge between platelets and specific components of the extracellular matrix, such as collagen. The biological activity of VWF in this process can be measured by different in vitro assays (Turecek et al., Semin. Thromb. Hemost. 28: 149-160, 2002). The ristocetin cofactor assay is based on the agglutination of fresh or formalin-fixed platelets induced by the antibiotic ristocetin in the presence of VWF. The degree of platelet agglutination depends on the VWF concentration and can be measured by the turbidimetric method, *e.g.* by use of an aggregometer (Weiss et al., J. Clin. Invest. 52: 2708-2716, 1973; Macfarlane et al., Thromb. Diath. Haemorrh. 34: 306-308, 1975). As provided herein, the specific Ristocetin Cofactor activity of the VWF (VWF:RCo) of the present invention is generally described in terms of mU/µg of VWF or IU/mg of VWF protein, as measured using in vitro assays.

The second method is the VWF collagen binding (VWF:CB) assay, which is based on ELISA technology (Brown et Bosak, Thromb. Res. 43: 303-311, 1986; Favaloro, Thromb. Haemost. 83: 127-135, 2000). A microtiter plate is coated with type I or III collagen. Then, the VWF is bound to the collagen surface and subsequently detected with an enzyme-labeled polyclonal antibody. The last step is the substrate reaction, which can be photometrically monitored with an ELISA reader. In some embodiments, VWF collagen binding (VWF:CB)/VWF:Ag, also referred to as the ratio of VWF collagen binding to antigen, is used to assess VWF and/or VWD diagnosis.

Further assays for VWF include VWF:Antigen (VWF:Ag), VWF:Ristocetin Cofactor (VWF:RCo), and VWF:Collagen Binding Activity assay (VWF:CB), which are often used for diagnosis and classification of Von Willebrand Disease. Additional useful methods for assessing VWF assays including assays for VWF glycoprotein IB binding, VWF collagen binding, VWF multimers, DDAVP challenge trials are known to those skilled in the art and are described in, for example, Patzke and Favaloro, Methods Mol Biol, 2017, 1646:453-460; Higgins and Goodwin, Am J Hematol, 2019, 94:496-503; Stufano et al., Haemophilia, 2020, 26(2):298-305; Ni et al., Int J Lab Hematol, 2013, 35(2):170-176; Michiels et al., J Hematol Thrombo Dis, 2019, 6:299; Michiels et al., Clinical and Applied Thrombosis/Hemostatis, 2017, 23(6):518-531; Mohammed and Favaloro, Methods Mol Biol, 2017, 1646:461-472; Favaloro et al., Pathology, 1997, 29(4): 341-456, the contents are hereby incorporated by reference in their entirety including all teachings related to assays for VWF.

In some embodiments, VWF collagen binding (VWF:CB)/VWF:Ag, also referred to as the ratio of VWF collagen binding to antigen, is used to assess VWF and/or VWD diagnosis.

In some embodiments, the ratio of rFVIII procoagulant activity (IU rFVIII:C) to rVWF Ristocetin cofactor activity (IU rVWF:RCo) for the rVWF prepared according to the methods of the present invention is between 3:1 and 1:5. In further embodiments, the ratio is between 2:1 and 1:4. In still further embodiments, the ratio is between 5:2 and 1:4. In further embodiments, the ratio is between 3:2 and 1:3. In still further embodiments, the ratio is about 1:1, 1:2, 1:3, 1:4, 1:5, 2:1, 2:3, 2:4, 2:5, 3:1, 3:2, 3:4, or 3:5. In further embodiments, the ratio is between 1:1 and 1:2. In yet further embodiments, the ratio is 1.1:1, 1.2:1, 1.3:1, 1.4:1, 1.5:1, 1.6:1, 1.7:1, 1.8:1, 1.9:1, or 2:1. In certain embodiments, the ratio of rFVIII procoagulant activity (IU rFVIII:C) to rVWF Ristocetin cofactor activity (IU rVWF:RCo) in a composition useful for a method described herein is selected from variations 1988 to 2140 found in Table 7 below which is a copy of Table 7 of WO2012/171031.

**Table 7. Exemplary embodiments for the ratio of rFVIII procoagulant activity (IU rFVIII:C) to rVWF Ristocetin cofactor activity (IU rVWF:RCo) in compositions and used in methods provided herein.**

| **(IU rFVIII:C) to (IU rVWF:RCo)** | | **(IU rFVIII:C) to (IU rVWF:RCo)** | | **(IU rFVIII:C) to (IU rVWF:RCo)** | | **(IU rFVIII:C) to (IU rVWF:RCo)** | |
|---|---|---|---|---|---|---|---|
| 4:1 | Var. 1988 | 3:1-3:5 | Var. 2027 | 4:3-1:4 | Var. 2065 | 4:5-2:3 | Var. 2103 |
| 3:1 | Var. 1989 | 3:1-2:3 | Var. 2028 | 4:3-1:3 | Var. 2066 | 4:5-3:4 | Var. 2104 |
| 2:1 | Var. 1990 | 3:1-3:4 | Var. 2029 | 4:3-2:5 | Var. 2067 | 3:4-1:6 | Var. 2105 |
| 3:2 | Var. 1991 | 3:1-4:5 | Var. 2030 | 4:3-1:2 | Var. 2068 | 3:4-1:5 | Var. 2106 |
| 4:3 | Var. 1992 | 3:1-5:6 | Var. 2031 | 4:3-3:5 | Var. 2069 | 3:4-1:4 | Var. 2107 |
| 1:1 | Var. 1993 | 3:1-1:1 | Var. 2032 | 4:3-2:3 | Var. 2070 | 3:4-1:3 | Var. 2108 |
| 5:6 | Var. 1994 | 3:1-4:3 | Var. 2033 | 4:3-3:4 | Var. 2071 | 3:4-2:5 | Var. 2109 |
| 4:5 | Var. 1995 | 3:1-3:2 | Var. 2034 | 4:3-4:5 | Var. 2072 | 3:4-1:2 | Var. 2110 |
| 3:4 | Var. 1996 | 3:1-2:1 | Var. 2035 | 4:3-5:6 | Var. 2073 | 3:4-3:5 | Var. 2111 |
| 2:3 | Var. 1997 | 2:1-1:6 | Var. 2036 | 4:3-1:1 | Var. 2074 | 3:4-2:3 | Var. 2112 |
| 3:5 | Var. 1998 | 2:1-1:5 | Var. 2037 | 1:1-1:6 | Var. 2075 | 2:3-1:6 | Var. 2113 |
| 1:2 | Var. 1999 | 2:1-1:4 | Var. 2038 | 1:1-1:5 | Var. 2076 | 2:3-1:5 | Var. 2114 |
| 2:5 | Var. 2000 | 2:1-1:3 | Var. 2039 | 1:1-1:4 | Var. 2077 | 2:3-1:4 | Var. 2115 |
| 1:3 | Var. 2001 | 2:1-2:5 | Var. 2040 | 1:1-1:3 | Var. 2078 | 2:3-1:3 | Var. 2116 |
| 1:4 | Var. 2002 | 2:1-1:2 | Var. 2041 | 1:1-2:5 | Var. 2079 | 2:3-2:5 | Var. 2117 |
| 1:5 | Var. 2003 | 2:1-3:5 | Var. 2042 | 1:1-1:2 | Var. 2080 | 2:3-1:2 | Var. 2118 |
| 1:6 | Var. 2004 | 2:1-2:3 | Var. 2043 | 1:1-3:5 | Var. 2081 | 2:3-3:5 | Var. 2119 |
| 4:1-1:6 | Var. 2005 | 2:1-3:4 | Var. 2044 | 1:1-2:3 | Var. 2082 | 3:5-1:6 | Var. 2120 |
| 4:1-1:5 | Var. 2006 | 2:1-4:5 | Var. 2045 | 1:1-3:4 | Var. 2083 | 3:5-1:5 | Var. 2121 |
| 4:1-1:4 | Var. 2007 | 2:1-5:6 | Var. 2046 | 1:1-4:5 | Var. 2084 | 3:5-1:4 | Var. 2122 |
| 4:1-1:3 | Var. 2008 | 2:1-1:1 | Var. 2047 | 1:1-5:6 | Var. 2085 | 3:5-1:3 | Var. 2123 |
| 4:1-2:5 | Var. 2009 | 2:1-4:3 | Var. 2048 | 5:6-1:6 | Var. 2086 | 3:5-2:5 | Var. 2124 |
| 4:1-1:2 | Var. 2010 | 2:1-3:2 | Var. 2049 | 5:6-1:5 | Var. 2087 | 3:5-1:2 | Var. 2125 |
| 4:1-3:5 | Var. 2011 | 3:2-1:6 | Var. 2050 | 5:6-1:4 | Var. 2088 | 1:2-1:6 | Var. 2126 |
| 4:1-2:3 | Var. 2012 | 3:2-1:5 | Var. 2051 | 5:6-1:3 | Var. 2089 | 1:2-1:5 | Var. 2127 |
| 4:1-3:4 | Var. 2013 | 3:2-1:4 | Var. 2052 | 5:6-2:5 | Var. 2090 | 1:2-1:4 | Var. 2128 |
| 4:1-4:5 | Var. 2014 | 3:2-1:3 | Var. 2053 | 5:6-1:2 | Var. 2091 | 1:2-1:3 | Var. 2129 |
| 4:1-5:6 | Var. 2015 | 3:2-2:5 | Var. 2054 | 5:6-3:5 | Var. 2092 | 1:2-2:5 | Var. 2130 |
| 4:1-1:1 | Var. 2016 | 3:2-1:2 | Var. 2055 | 5:6-2:3 | Var. 2093 | 2:5-1:6 | Var. 2131 |
| 4:1-4:3 | Var. 2017 | 3:2-3:5 | Var. 2056 | 5:6-3:4 | Var. 2094 | 2:5-1:5 | Var. 2132 |
| 4:1-3:2 | Var. 2018 | 3:2-2:3 | Var. 2057 | 5:6-4:5 | Var. 2095 | 2:5-1:4 | Var. 2133 |
| 4:1-2:1 | Var. 2019 | 3:2-3:4 | Var. 2058 | 4:5-1:6 | Var. 2096 | 2:5-1:3 | Var. 2134 |
| 4:1-3:1 | Var. 2020 | 3:2-4:5 | Var. 2059 | 4:5-1:5 | Var. 2097 | 1:3-1:6 | Var. 2135 |
| 3:1-1:6 | Var. 2021 | 3:2-5:6 | Var. 2060 | 4:5-1:4 | Var. 2098 | 1:3-1:5 | Var. 2136 |
| 3:1-1:5 | Var. 2022 | 3:2-1:1 | Var. 2061 | 4:5-1:3 | Var. 2099 | 1:3-1:4 | Var. 2137 |
| 3:1-1:4 | Var. 2023 | 3:2-4:3 | Var. 2062 | 4:5-2:5 | Var. 2100 | 1:4-1:6 | Var. 2138 |
| 3:1-1:3 | Var. 2024 | 4:3-1:6 | Var. 2063 | 4:5-1:2 | Var. 2101 | 1:4-1:5 | Var. 2139 |
| 3:1-2:5 | Var. 2025 | 4:3-1:5 | Var. 2064 | 4:5-3:5 | Var. 2102 | 1:5-1:6 | Var. 2140 |
| 3:1-1:2 | Var. 2026 | | | | | | |

| | | | | | | | |
|---|---|---|---|---|---|---|---|
| Var. = Variation | | | | | | | |

In some embodiments, the pro-VWF and/or purified rVWF purified in accordance with the present invention is not modified with any conjugation, post-translation or covalent modifications. In particular embodiments, the pro-VWF and/or purified rVWF of the present invention is not modified with a water soluble polymer, including without limitation, a polyethylene glycol (PEG), a polypropylene glycol, a polyoxyalkylene, a polysialic acid, hydroxyl ethyl starch, a poly-carbohydrate moiety, and the like.

In some embodiments, the pro-VWF and/or purified rVWF purified in accordance with the present invention is modified through conjugation, post-translation modification, or covalent modification, including modifications of the N- or C- terminal residues as well as modifications of selected side chains, for example, at free sulfhydryl-groups, primary amines, and hydroxyl-groups. In one embodiment, a water-soluble polymer is linked to the protein (directly or via a linker) by a lysine group or other primary amine. In some embodiments, the pro-VWF and/or purified rVWF of the present invention may be modified by conjugation of a water soluble polymer, including without limitation, a polyethylene glycol (PEG), a polypropylene glycol, a polyoxyalkylene, a polysialic acid, hydroxyl ethyl starch, a poly-carbohydrate moiety, and the like.

Water-soluble polymers that may be used to modify the pro-VWF and/or purified rVWF include linear and branched structures. The conjugated polymers may be attached directly to the coagulation proteins of the invention, or alternatively may be attached through a linking moiety. Non-limiting examples of protein conjugation with water soluble polymers can be found in U.S. Patent Nos. 4,640,835; 4,496,689; 4,301,144; 4,670,417; 4,791,192, and 4,179,337, as well as in Abuchowski and Davis "Enzymes as Drugs," Holcenberg and Roberts, Eds., pp. 367 383, John Wiley and Sons, New York (1981), and Hermanson G., Bioconjugate Techniques 2nd Ed., Academic Press, Inc. 2008.

Protein conjugation may be performed by a number of well-known techniques in the art, for example, see Hermanson G., Bioconjugate Techniques 2nd Ed., Academic Press, Inc. 2008. Examples include linkage through the peptide bond between a carboxyl group on one of either the coagulation protein or water-soluble polymer moiety and an amine group of the other, or an ester linkage between a carboxyl group of one and a hydroxyl group of the other. Another linkage by which a coagulation protein of the invention could be conjugated to a water-soluble polymer compound is via a Schiff base, between a free amino group on the polymer moiety being reacted with an aldehyde group formed at the non-reducing end of the polymer by periodate oxidation (Jennings and Lugowski, J. Immunol. 1981; 127:1011-8; Femandes and Gregonradis, Biochim Biophys Acta. 1997; 1341; 26-34). The generated Schiff Base can be stabilized by specific reduction with NaCNBH₃ to form a secondary amine. An alternative approach is the generation of terminal free amino groups on the polymer by reductive amination with NH₄Cl after prior oxidation. Bifunctional reagents can be used for linking two amino or two hydroxyl groups. For example, a polymer containing an amino group can be coupled to an amino group of the coagulation protein with reagents like BS3 (Bis(sulfosuccinimidyl)suberate/Pierce, Rockford, Ill.). In addition, heterobifunctional cross-linking reagents like Sulfo-EMCS (N-ε-Maleimidocaproyloxy) sulfosuccinimide ester/Pierce) can be used for instance to link amine and thiol groups. In other embodiments, an aldehyde reactive group, such as PEG alkoxide plus diethyl acetal of bromoacetaldehyde; PEG plus DMSO and acetic anhydride, and PEG chloride plus the phenoxide of 4-hydroxybenzaldehyde, succinimidyl active esters, activated dithiocarbonate PEG, 2,4,5-trichlorophenylchloroformate and P-nitrophenylchloroformate activated PEG, may be used in the conjugation of a coagulation protein.

Another method for measuring the biological activity of VWF is the collagen binding assay, which is based on ELISA technology (Brown and Bosak, Thromb. Res., 1986, 43:303-311; Favaloro, Thromb. Haemost., 2000, 83 127-135). A microtiter plate is coated with type I or III collagen. Then the VWF is bound to the collagen surface and subsequently detected with an enzyme-labeled polyclonal antibody. The last step is a substrate reaction, which can be photometrically monitored with an ELISA reader.

Immunological assays of von Willebrand factors (VWF:Ag) are immunoassays that measure the concentration of the VWF protein in plasma. They give no indication as to VWF function. A number of methods exist for measuring VWF:Ag and these include both enzymelinked immunosorbent assay (ELISA) or automated latex immunoassays (LIA.) Many laboratories now use a fully automated latex immunoassay. Historically laboratories used a variety of techniques including Laurell electroimmunoassay 'Laurell Rockets' but these are rarely used in most labs today.

### V. Kits

As an additional aspect, the invention includes kits which comprise one or more lyophilized compositions packaged in a manner which facilitates their use for administration to subjects. In one embodiment, such a kit includes pharmaceutical formulation described herein (*e.g.,* a composition comprising a therapeutic protein or peptide), packaged in a container such as a sealed bottle or vessel, with a label affixed to the container or included in the package that describes use of the compound or composition in practicing the method. In one embodiment, the pharmaceutical formulation is packaged in the container such that the amount of headspace in the container (*e.g.*, the amount of air between the liquid formulation and the top of the container) is very small. Preferably, the amount of headspace is negligible (*e.g*., almost none). In one embodiment, the kit contains a first container having a therapeutic protein or peptide composition and a second container having a physiologically acceptable reconstitution solution for the composition. In one aspect, the pharmaceutical formulation is packaged in a unit dosage form. The kit may further include a device suitable for administering the pharmaceutical formulation according to a specific route of administration. Preferably, the kit contains a label that describes use of the pharmaceutical formulations.

In certain embodiments, the compositions provided are liquid formulations for administration with the use of a syringe or other storage vessel. In further embodiments, these liquid formulations are produced from lyophilized material described herein reconstituted as an aqueous solution.

### VI. rVWF for Methods of Treating Menorrhagia in Patient with Severe VWD

One of the advantages of administering rVWF to subjects with severe VWD is that the higher specific activity of rVWF as compared to pdVWF allows flexibility in the amount of rVWF administered and the number of times the subject is re-dosed. As will be appreciated and as is discussed in further detail herein, the co-administered FVIII may be recombinant or plasma derived.

Single or multiple administrations of rVWF are carried out with the dose levels and pattern being selected by the treating physician. For the prevention or treatment of disease, the appropriate dosage depends on the type of disease to be treated (e.g., von Willebrand disease), the severity and course of the disease, whether drug is administered for preventive or therapeutic purposes, previous therapy, the patient's clinical history and response to the drug, and the discretion of the attending physician.

In some aspects, rVWF is administered to a subject at a range from 20-90 IU/kg, *e.g.,* 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 20-90, 30-60, 30-70, 35-70, 20-40, 35-60, 40-50, 40-60, 40-85, 45-60, 45-55, 45-50, 50-55, 50-60, 50-75, 50-90, 55-60, or 60-80 IU/kg.

In some embodiments, rVWF is administered to the subject at a range of -90 IU/kg, *e.g.,* 5-90, 5-50, 10-90, 15-90, 20-90, 30-60, 30-70, 30-90, 40-50, 40-60, 40-85, 40-90, 50-75, 50-90, 60-80, 60-90, 70-90, 80-90, 5-80, 10-70, 20-60, 30-50, 35-60, 5-50, 5-40, 5-30. 5-20, 10-90, 10-50, or 20-40 IU/kg. In other embodiments, rVWF is administered at a dose of 70-200 IU/kg, *e.g.,* 70-200, 80-200-, 90-200, 100-200, 110-200, 120-200, 130-200, 130-200, 140-200, 150-200, 160-200, 170-200, 180-200, 190-200, 70-170, 80-180, 60-160, 50-150, 40-140, 30, 130, 20-120, 10-110, 70-100, or 70-90 IU/kg.

In some embodiments, the subject is administered 30-60 IU/kg rVWF. In some embodiments, the subject is administered 40-50 IU/kg rVWF.

In some embodiments, the subject is administered 40-85 IU/kg rVWF. In some embodiments, the subject is administered 50-75 IU/kg rVWF. In other embodiments, the subject is administered 55-70 IU/kg rVWF.

In some embodiments, the subject is administered 30-70 IU/kg rVWF. In some embodiments, the subject is administered 40-60 IU/kg rVWF. In other embodiments, the subject is administered 45-55 IU/kg rVWF.

In some embodiments, the subject is administered 50-90 IU/kg rVWF. In some embodiments, the subject is administered 60-80 IU/kg rVWF. In other embodiments, the subject is administered 65-85 IU/kg rVWF.

Compositions of rVWF can be contained in pharmaceutical formulations, as described herein. Such formulations can be administered orally, topically, transdermally, parenterally, by inhalation spray, vaginally, rectally, or by intracranial injection. The term parenteral as used herein includes subcutaneous injections, intravenous, intramuscular, intracisternal injection, or infusion techniques. Administration by intravenous, intradermal, intramuscular, intramammary, intraperitoneal, intrathecal, retrobulbar, intrapulmonary injection and or surgical implantation at a particular site is contemplated as well. Generally, compositions are essentially free of pyrogens, as well as other impurities that could be harmful to the recipient.

In one aspect, formulations of the invention are administered by an initial bolus followed by a continuous infusion to maintain therapeutic circulating levels of drug product. As another example, the inventive compound is administered as a one-time dose. Those of ordinary skill in the art will readily optimize effective dosages and administration regimens as determined by good medical practice and the clinical condition of the individual patient. The route of administration can be, but is not limited to, by intravenous, intraperitoneal, subcutaneous, or intramuscular administration. The frequency of dosing depends on the pharmacokinetic parameters of the agents and the route of administration. The optimal pharmaceutical formulation is determined by one skilled in the art depending upon the route of administration and desired dosage. See for example, Remington's Pharmaceutical Sciences, 18th Ed., 1990, Mack Publishing Co., Easton, Pa. 18042 pages 1435-1712, the disclosure of which is hereby incorporated by reference in its entirety for all purposes and in particular for all teachings related to formulations, routes of administration and dosages for pharmaceutical products. Such formulations influence the physical state, stability, rate of *in vivo* release, and rate of *in vivo* clearance of the administered agents. Depending on the route of administration, a suitable dose is calculated according to body weight, body surface area or organ size. Appropriate dosages may be ascertained through use of established assays for determining blood level dosages in conjunction with appropriate dose-response data. The final dosage regimen is determined by the attending physician, considering various factors which modify the action of drugs, *e.g.* the drug's specific activity, the severity of the damage and the responsiveness of the patient, the age, condition, body weight, sex and diet of the patient, the severity of any infection, time of administration and other clinical factors. By way of example, a typical dose of a recombinant VWF of the present invention is approximately 50 IU/kg, equal to 500 µg/kg. As studies are conducted, further information will emerge regarding the appropriate dosage levels and duration of treatment for various diseases and conditions.

### a. Lyophilized VWF Formulations

The present method also provides formulations of rVWF for use in the treatment methods provided herein. In some embodiments, the rVWF composition is used for the production of a pharmaceutical composition. In some embodiments, the rVWF can be formulated into a lyophilized formulation.

In some embodiments, the formulations comprising a VWF polypeptide of the invention are lyophilized after purification and prior to administration to a subject. Lyophilization is carried out using techniques common in the art and should be optimized for the composition being developed (Tang et al., Pharm Res. 21:191-200, (2004) and Chang et al., Pharm Res. 13:243-9 (1996)).

A lyophilization cycle is, in one aspect, composed of three steps: freezing, primary drying, and secondary drying (A. P. Mackenzie, Phil Trans R Soc London, Ser B, Biol 278:167 (1977)). In the freezing step, the solution is cooled to initiate ice formation. Furthermore, this step induces the crystallization of the bulking agent. The ice sublimes in the primary drying stage, which is conducted by reducing chamber pressure below the vapor pressure of the ice, using a vacuum and introducing heat to promote sublimation. Finally, adsorbed or bound water is removed at the secondary drying stage under reduced chamber pressure and at an elevated shelf temperature. The process produces a material known as a lyophilized cake. Thereafter the cake can be reconstituted with either sterile water or suitable diluent for injection.

The lyophilization cycle not only determines the final physical state of excipients but also affects other parameters such as reconstitution time, appearance, stability and final moisture content. The composition structure in the frozen state proceeds through several transitions (*e.g.*, glass transitions, wettings, and crystallizations) that occur at specific temperatures and the structure may be used to understand and optimize the lyophilization process. The glass transition temperature (Tg and/or Tg') can provide information about the physical state of a solute and can be determined by differential scanning calorimetry (DSC). Tg and Tg' are an important parameter that must be taken into account when designing the lyophilization cycle. For example, Tg' is important for primary drying. Furthermore, in the dried state, the glass transition temperature provides information on the storage temperature of the final product.

### b. Pharmaceutical Formulations and Excipients in General

Excipients are additives that either impart or enhance the stability and delivery of a drug product (*e.g*., protein). Regardless of the reason for their inclusion, excipients are an integral component of a formulation and therefore need to be safe and well tolerated by patients. For protein drugs, the choice of excipients is particularly important because they can affect both efficacy and immunogenicity of the drug. Hence, protein formulations need to be developed with appropriate selection of excipients that afford suitable stability, safety, and marketability.

A lyophilized formulation is, in one aspect, at least comprised of one or more of a buffer, a bulking agent, and a stabilizer. In this aspect, the utility of a surfactant is evaluated and selected in cases where aggregation during the lyophilization step or during reconstitution becomes an issue. An appropriate buffering agent is included to maintain the formulation within stable zones of pH during lyophilization. A comparison of the excipient components contemplated for liquid and lyophilized protein formulations is provided in Table 8.

**Table 8: Excipient components of lyophilized protein formulations**

| **Excipient component** | **Function in lyophilized formulation** | |
|---|---|---|
| Buffer | | ∘ Maintain pH of formulation during lyophilization and upon reconstitution |
| Tonicity agent/stabilizer | | ∘ Stabilizers include cryo and lycoprotectants |
| | | ∘ Examples include polyols, sugars and polymers |
| | | ∘ Cryoprotectants protect proteins from freezing stresses |
| | | ∘ Lyoprotectants stabilize proteins in the freeze-dried state |
| Bulking agent | | ∘ Used to enhance product elegance and to prevent blowout |
| | | ∘ Provides structural strength to the lyo cake |
| | | ∘ Examples include mannitol and glycine |
| Surfactant | | ∘ Employed if aggregation during the lyophilization process is an issue |
| | | ∘ May serve to reduce reconstitution times |
| | | ∘ Examples include polysorbate 20 and 80 |
| Anti-oxidant | | ∘ Usually not employed, molecular reactions in the lyo cake are generally retarded |
| Metal ions/chelating agent | | ∘ May be included if a specific metal ion is included only as a co-factor of where the metal is required for protease activity |
| | | ∘ Chelating agents are generally not needed in lyo formulations |
| Preservative | | ∘ For multi-dose formulations only |
| | | ∘ Provides protection against microbial growth in formulation |
| | | ∘ Is usually included in the reconstitution diluent (*e*.*g*., bWFI) |

The principal challenge in developing formulations for proteins is stabilizing the product against the stresses of manufacturing, shipping and storage. The role of formulation excipients is to provide stabilization against these stresses. Excipients are also be employed to reduce viscosity of high concentration protein formulations in order to enable their delivery and enhance patient convenience. In general, excipients can be classified on the basis of the mechanisms by which they stabilize proteins against various chemical and physical stresses. Some excipients are used to alleviate the effects of a specific stress or to regulate a particular susceptibility of a specific protein. Other excipients have more general effects on the physical and covalent stabilities of proteins. The excipients described herein are organized either by their chemical type or their functional role in formulations. Brief descriptions of the modes of stabilization are provided when discussing each excipient type.

Given the teachings and guidance provided herein, those skilled in the art will know what amount or range of excipient can be included in any particular formulation to achieve a biopharmaceutical formulation of the invention that promotes retention in stability of the biopharmaceutical (*e.g*., a protein). For example, the amount and type of a salt to be included in a biopharmaceutical formulation of the invention is selected based on the desired osmolality (*e.g.*, isotonic, hypotonic or hypertonic) of the final solution as well as the amounts and osmolality of other components to be included in the formulation.

By way of example, inclusion of about 5% sorbitol can achieve isotonicity while about 9% of a sucrose excipient is needed to achieve isotonicity. Selection of the amount or range of concentrations of one or more excipients that can be included within a biopharmaceutical formulation of the invention has been exemplified above by reference to salts, polyols and sugars. However, those skilled in the art will understand that the considerations described herein and further exemplified by reference to specific excipients are equally applicable to all types and combinations of excipients including, for example, salts, amino acids, other tonicity agents, surfactants, stabilizers, bulking agents, cryoprotectants, lyoprotectants, anti-oxidants, metal ions, chelating agents and/or preservatives.

Further, where a particular excipient is reported in molar concentration, those skilled in the art will recognize that the equivalent percent (%) w/v (*e.g.,* (grams of substance in a solution sample/mL of solution) X 100%) of solution is also contemplated.

Of course, a person having ordinary skill in the art would recognize that the concentrations of the excipients described herein share an interdependency within a particular formulation. By way of example, the concentration of a bulking agent may be lowered where, *e.g.,* there is a high protein concentration or where, *e.g.,* there is a high stabilizing agent concentration. In addition, a person having ordinary skill in the art would recognize that, in order to maintain the isotonicity of a particular formulation in which there is no bulking agent, the concentration of a stabilizing agent would be adjusted accordingly (*e.g.*, a "tonicifying" amount of stabilizer would be used). Common excipients are known in the art and can be found in Powell et al., Compendium of Excipients fir Parenteral Formulations (1998), PDA J. Pharm. Sci. Technology, 52:238-311.

### c. Pharmaceutical Buffers and Buffering Agents

The stability of a pharmacologically active protein formulation is usually observed to be maximal in a narrow pH range. This pH range of optimal stability needs to be identified early during pre-formulation studies. Several approaches, such as accelerated stability studies and calorimetric screening studies, are useful in this endeavor (Remmele R.L. Jr., et al., Biochemistry, 38(16): 5241-7 (1999)). Once a formulation is finalized, the protein must be manufactured and maintained throughout its shelf-life. Hence, buffering agents are almost always employed to control pH in the formulation.

The buffer capacity of the buffering species is maximal at a pH equal to the pKa and decreases as pH increases or decreases away from this value. Ninety percent of the buffering capacity exists within one pH unit of its pKa. Buffer capacity also increases proportionally with increasing buffer concentration.

Several factors need to be considered when choosing a buffer. First and foremost, the buffer species and its concentration need to be defined based on its pKa and the desired formulation pH. Equally important is to ensure that the buffer is compatible with the protein and other formulation excipients, and does not catalyze any degradation reactions. A third important aspect to be considered is the sensation of stinging and irritation the buffer may induce upon administration. For example, citrate is known to cause stinging upon injection (Laursen T, et al., Basic Clin Pharmacol Toxicol., 98(2): 218-21 (2006)). The potential for stinging and irritation is greater for drugs that are administered via the subcutaneous (SC) or intramuscular (IM) routes, where the drug solution remains at the site for a relatively longer period of time than when administered by the IV route where the formulation gets diluted rapidly into the blood upon administration. For formulations that are administered by direct IV infusion, the total amount of buffer (and any other formulation component) needs to be monitored. One has to be particularly careful about potassium ions administered in the form of the potassium phosphate buffer, which can induce cardiovascular effects in a patient (Hollander-Rodriguez JC, et al., Am. Fam. Physician., 73(2): 283-90 (2006)).

Buffers for lyophilized formulations need additional consideration. Some buffers like sodium phosphate can crystallize out of the protein amorphous phase during freezing resulting in shifts in pH. Other common buffers such as acetate and imidazole may sublime or evaporate during the lyophilization process, thereby shifting the pH of formulation during lyophilization or after reconstitution.

The buffer system present in the compositions is selected to be physiologically compatible and to maintain a desired pH of the pharmaceutical formulation. In one embodiment, the pH of the solution is between pH 2.0 and pH 12.0. For example, the pH of the solution may be 2.0, 2.3, 2.5, 2.7, 3.0, 3.3, 3.5, 3.7, 4.0, 4.3, 4.5, 4.7, 5.0, 5.3, 5.5, 5.7, 6.0, 6.3, 6.5, 6.7, 7.0, 7.3, 7.5, 7.7, 8.0, 8.3, 8.5, 8.7, 9.0, 9.3, 9.5, 9.7, 10.0, 10.3, 10.5, 10.7, 11.0, 11.3, 11.5, 11.7, or 12.0.

The pH buffering compound may be present in any amount suitable to maintain the pH of the formulation at a predetermined level. In one embodiment, the pH buffering concentration is between 0.1 mM and 500 mM. For example, it is contemplated that the pH buffering agent is at least 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 25, 30, 40, 50, 60, 70, 80, 90, 100, 200, or 500 mM.

Exemplary pH buffering agents used to buffer the formulation as set out herein include, but are not limited to organic acids, glycine, histidine, glutamate, succinate, phosphate, acetate, citrate, Tris, HEPES, and amino acids or mixtures of amino acids, including, but not limited to aspartate, histidine, and glycine. In one embodiment of the present invention, the buffering agent is citrate.

### d. Pharmaceutical Stabilizers and Bulking Agents

In one aspect of the present pharmaceutical formulations, a stabilizer (or a combination of stabilizers) is added to prevent or reduce storage-induced aggregation and chemical degradation. A hazy or turbid solution upon reconstitution indicates that the protein has precipitated or at least aggregated. The term "stabilizer" means an excipient capable of preventing aggregation or physical degradation, including chemical degradation (for example, autolysis, deamidation, oxidation, etc.) in an aqueous state. Stabilizers contemplated include, but are not limited to, sucrose, trehalose, mannose, maltose, lactose, glucose, raffinose, cellobiose, gentiobiose, isomaltose, arabinose, glucosamine, fructose, mannitol, sorbitol, glycine, arginine HCL, poly-hydroxy compounds, including polysaccharides such as dextran, starch, hydroxyethyl starch, cyclodextrins, N-methyl pyrrolidene, cellulose and hyaluronic acid, sodium chloride, (Carpenter et al., Develop. Biol. Standard 74:225, (1991)). In the present formulations, the stabilizer is incorporated in a concentration of about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, 200, 500, 700, 900, or 1000 mM. In one embodiment of the present invention, mannitol and trehalose are used as stabilizing agents.

If desired, the formulations also include appropriate amounts of bulking and osmolality regulating agents. Bulking agents include, for example and without limitation, mannitol, glycine, sucrose, polymers such as dextran, polyvinylpyrrolidone, carboxymethylcellulose, lactose, sorbitol, trehalose, or xylitol. In one embodiment, the bulking agent is mannitol. The bulking agent is incorporated in a concentration of about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, 200, 500, 700, 900, or 1000 mM.

### e. Pharmaceutical Surfactants

Proteins have a high propensity to interact with surfaces making them susceptible to adsorption and denaturation at air-liquid, vial-liquid, and liquid-liquid (silicone oil) interfaces. This degradation pathway has been observed to be inversely dependent on protein concentration and results in either the formation of soluble and insoluble protein aggregates or the loss of protein from solution via adsorption to surfaces. In addition to container surface adsorption, surface-induced degradation is exacerbated with physical agitation, as would be experienced during shipping and handling of the product.

Surfactants are commonly used in protein formulations to prevent surface-induced degradation. Surfactants are amphipathic molecules with the capability of out-competing proteins for interfacial positions. Hydrophobic portions of the surfactant molecules occupy interfacial positions (*e.g.*, air/liquid), while hydrophilic portions of the molecules remain oriented towards the bulk solvent. At sufficient concentrations (typically around the detergent's critical micellar concentration), a surface layer of surfactant molecules serves to prevent protein molecules from adsorbing at the interface. Thereby, surface-induced degradation is minimized. Surfactants contemplated herein include, without limitation, fatty acid esters of sorbitan polyethoxylates, *e.g.*, polysorbate 20 and polysorbate 80. The two differ only in the length of the aliphatic chain that imparts hydrophobic character to the molecules, C-12 and C-18, respectively. Accordingly, polysorbate-80 is more surface- active and has a lower critical micellar concentration than polysorbate-20.

Detergents can also affect the thermodynamic conformational stability of proteins. Here again, the effects of a given detergent excipient will be protein specific. For example, polysorbates have been shown to reduce the stability of some proteins and increase the stability of others. Detergent destabilization of proteins can be rationalized in terms of the hydrophobic tails of the detergent molecules that can engage in specific binding with partially or wholly unfolded protein states. These types of interactions could cause a shift in the conformational equilibrium towards the more expanded protein states (*e.g.*, increasing the exposure of hydrophobic portions of the protein molecule in complement to binding polysorbate). Alternatively, if the protein native state exhibits some hydrophobic surfaces, detergent binding to the native state may stabilize that conformation.

Another aspect of polysorbates is that they are inherently susceptible to oxidative degradation. Often, as raw materials, they contain sufficient quantities of peroxides to cause oxidation of protein residue side-chains, especially methionine. The potential for oxidative damage arising from the addition of stabilizer emphasizes the point that the lowest effective concentrations of excipients should be used in formulations. For surfactants, the effective concentration for a given protein will depend on the mechanism of stabilization.

Surfactants are also added in appropriate amounts to prevent surface related aggregation phenomenon during freezing and drying (Chang, B, J., Pharm. Sci., 85:1325, (1996)). Thus, exemplary surfactants include, without limitation, anionic, cationic, nonionic, zwitterionic, and amphoteric surfactants including surfactants derived from naturally-occurring amino acids. Anionic surfactants include, but are not limited to, sodium lauryl sulfate, dioctyl sodium sulfo succinate and dioctyl sodium sulfonate, chenodeoxycholic acid, N-lauroylsarcosine sodium salt, lithium dodecyl sulfate, 1-octanesulfonic acid sodium salt, sodium cholate hydrate, sodium deoxycholate, and glycodeoxycholic acid sodium salt. Cationic surfactants include, but are not limited to, benzalkonium chloride or benzethonium chloride, cetylpyridinium chloride monohydrate, and hexadecyltrimethylammonium bromide. Zwitterionic surfactants include, but are not limited to, CHAPS, CHAPSO, SB3-10, and SB3-12. Non-ionic surfactants include, but are not limited to, digitonin, Triton X-100, Triton X-114, TWEEN-20, and TWEEN-80. Surfactants also include, but are not limited to lauromacrogol 400, polyoxyl 40 stearate, polyoxyethylene hydrogenated castor oil 10, 40, 50 and 60, glycerol monostearate, polysorbate 40, 60, 65 and 80, soy lecithin and other phospholipids such as dioleyl phosphatidyl choline (DOPC), dimyristoylphosphatidyl glycerol (DMPG), dimyristoylphosphatidyl choline (DMPC), and (dioleyl phosphatidyl glycerol) DOPG; sucrose fatty acid ester, methyl cellulose and carboxymethyl cellulose. Compositions comprising these surfactants, either individually or as a mixture in different ratios, are therefore further provided. In one embodiment of the present invention, the surfactant is TWEEN-80. In the present formulations, the surfactant is incorporated in a concentration of about 0.01 to about 0.5 g/L. In formulations provided, the surfactant concentration is 0.005, 0.01, 0.02, 0.03, 0.05, 0.06, 0.07, 0.08, 0.09, 0.1, 0.2, 0.3, 0.4, 0.5, 0.6, 0.7, 0.8, 0.9 or 1.0 g/L.

### f. Pharmaceutical Salts

Salts are often added to increase the ionic strength of the formulation, which can be important for protein solubility, physical stability, and isotonicity. Salts can affect the physical stability of proteins in a variety of ways. Ions can stabilize the native state of proteins by binding to charged residues on the protein's surface. Alternatively, salts can stabilize the denatured state by binding to peptide groups along the protein backbone (-CONH-). Salts can also stabilize the protein native conformation by shielding repulsive electrostatic interactions between residues within a protein molecule. Salts in protein formulations can also shield attractive electrostatic interactions between protein molecules that can lead to protein aggregation and insolubility. In formulations provided, the salt concentration is between 0.1, 1, 10, 20, 30, 40, 50, 80, 100, 120, 150, 200, 300, and 500 mM.

### g. Other Common Excipient Components: Pharmaceutical Amino Acids

Amino acids have found versatile use in protein formulations as buffers, bulking agents, stabilizers and antioxidants. Thus, in one aspect histidine and glutamic acid are employed to buffer protein formulations in the pH range of 5.5 - 6.5 and 4.0 - 5.5 respectively. The imidazole group of histidine has a pKa = 6.0 and the carboxyl group of glutamic acid side chain has a pKa of 4.3 which makes these amino acids suitable for buffering in their respective pH ranges. Glutamic acid is particularly useful in such cases. Histidine is commonly found in marketed protein formulations, and this amino acid provides an alternative to citrate, a buffer known to sting upon injection. Interestingly, histidine has also been reported to have a stabilizing effect, with respect to aggregation when used at high concentrations in both liquid and lyophilized presentations (Chen B, et al., Pharm Res., 20(12): 1952-60 (2003)). Histidine was also observed by others to reduce the viscosity of a high protein concentration formulation. However, in the same study, the authors observed increased aggregation and discoloration in histidine containing formulations during freeze-thaw studies of the antibody in stainless steel containers. Another note of caution with histidine is that it undergoes photo-oxidation in the presence of metal ions (Tomita M, et al., Biochemistry, 8(12): 5149-60 (1969)). The use of methionine as an antioxidant in formulations appears promising; it has been observed to be effective against a number of oxidative stresses (Lam XM, et al., J Pharm Sci, 86(11): 1250-5 (1997)).

In various aspects, formulations are provided which include one or more of the amino acids glycine, proline, serine, arginine and alanine have been shown to stabilize proteins by the mechanism of preferential exclusion. Glycine is also a commonly used bulking agent in lyophilized formulations. Arginine has been shown to be an effective agent in inhibiting aggregation and has been used in both liquid and lyophilized formulations. In formulations provided, the amino acid concentration is between 0.1, 1, 10, 20, 30, 40, 50, 80, 100, 120, 150, 200, 300, and 500 mM. In one embodiment of the present invention, the amino acid is glycine.

### h. Other Common Excipient Components: Pharmaceutical Antioxidants

Oxidation of protein residues arises from a number of different sources. Beyond the addition of specific antioxidants, the prevention of oxidative protein damage involves the careful control of a number of factors throughout the manufacturing process and storage of the product such as atmospheric oxygen, temperature, light exposure, and chemical contamination. The invention therefore contemplates the use of the pharmaceutical antioxidants including, without limitation, reducing agents, oxygen/free-radical scavengers, or chelating agents. Antioxidants in therapeutic protein formulations are, in one aspect, water-soluble and remain active throughout the product shelf -life. Reducing agents and oxygen/free-radical scavengers work by ablating active oxygen species in solution. Chelating agents such as EDTA are effective by binding trace metal contaminants that promote free-radical formation. For example, EDTA was utilized in the liquid formulation of acidic fibroblast growth factor to inhibit the metal ion catalyzed oxidation of cysteine residues.

In addition to the effectiveness of various excipients to prevent protein oxidation, the potential for the antioxidants themselves to induce other covalent or physical changes to the protein is of concern. For example, reducing agents can cause disruption of intramolecular disulfide linkages, which can lead to disulfide shuffling. In the presence of transition metal ions, ascorbic acid and EDTA have been shown to promote methionine oxidation in a number of proteins and peptides (Akers MJ, and Defelippis MR. Peptides and Proteins as Parenteral Solutions. In: Pharmaceutical Formulation Development of Peptides and Proteins. Sven Frokjaer, Lars Hovgaard, editors. Pharmaceutical Science. Taylor and Francis, UK (1999)); Fransson J.R., /. Pharm. Sci. 86(9): 4046-1050 (1997); Yin J, et al., Pharm Res., 21(12): 2377-83 (2004)). Sodium thiosulfate has been reported to reduce the levels of light and temperature induced methionine-oxidation in rhuMab HER2; however, the formation of a thiosulfate-protein adduct was also reported in this study (Lam XM, Yang JY, et al., J Pharm Sci. 86(11): 1250-5 (1997)). Selection of an appropriate antioxidant is made according to the specific stresses and sensitivities of the protein. Antioxidants contemplated in certain aspects include, without limitation, reducing agents and oxygen/free-radical scavengers, EDTA, and sodium thiosulfate.

### i. Other Common Excipient Components: Pharmaceutical Metal Ions

In general, transition metal ions are undesired in protein formulations because they can catalyze physical and chemical degradation reactions in proteins. However, specific metal ions are included in formulations when they are co-factors to proteins and in suspension formulations of proteins where they form coordination complexes (*e.g*., zinc suspension of insulin). Recently, the use of magnesium ions (10 -120 mM) has been proposed to inhibit the isomerization of aspartic acid to isoaspartic acid (WO 2004039337).

Two examples where metal ions confer stability or increased activity in proteins are human deoxyribonuclease (rhDNase, Pulmozyme^{®}), and Factor VIII. In the case of rhDNase, Ca⁺² ions (up to 100 mM) increased the stability of the enzyme through a specific binding site (Chen B, et al., / Pharm Sci., 88(4): 477-82 (1999)). In fact, removal of calcium ions from the solution with EGTA caused an increase in deamidation and aggregation. However, this effect was observed only with Ca⁺² ions; other divalent cations Mg⁺², Mn⁺² and Zn⁺² were observed to destabilize rhDNase. Similar effects were observed in Factor VIII. Ca⁺² and Sr⁺² ions stabilized the protein while others like Mg⁺², Mn⁺² and Zn⁺², Cu⁺² and Fe⁺² destabilized the enzyme (Fatouros, A., et al., Int. J. Pharm., 155, 121-131 (1997). In a separate study with Factor VIII, a significant increase in aggregation rate was observed in the presence of Al⁺³ ions (Derrick TS, et al., Pharm. Sci., 93(10): 2549-57 (2004)). The authors note that other excipients like buffer salts are often contaminated with Al⁺³ ions and illustrate the need to use excipients of appropriate quality in formulated products.

### j. Other Common Excipient Components: Pharmaceutical Preservatives

Preservatives are necessary when developing multi-use parenteral formulations that involve more than one extraction from the same container. Their primary function is to inhibit microbial growth and ensure product sterility throughout the shelf- life or term of use of the drug product. Commonly used preservatives include, without limitation, benzyl alcohol, phenol and m-cresol. Although preservatives have a long history of use, the development of protein formulations that includes preservatives can be challenging. Preservatives almost always have a destabilizing effect (aggregation) on proteins, and this has become a major factor in limiting their use in multi-dose protein formulations (Roy S, et al., J Pharm Sci, 94(2): 382-96 (2005)).

To date, most protein drugs have been formulated for single-use only. However, when multi-dose formulations are possible, they have the added advantage of enabling patient convenience, and increased marketability. A good example is that of human growth hormone (hGH) where the development of preserved formulations has led to commercialization of more convenient, multi-use injection pen presentations. At least four such pen devices containing preserved formulations of hGH are currently available on the market. Norditropin^{®} (liquid, Novo Nordisk), Nutropin AQ^{®} (liquid, Genentech) & Genotropin (lyophilized - dual chamber cartridge, Pharmacia & Upjohn) contain phenol while Somatrope^{®} (Eli Lilly) is formulated with m-cresol.

Several aspects need to be considered during the formulation development of preserved dosage forms. The effective preservative concentration in the drug product must be optimized. This requires testing a given preservative in the dosage form with concentration ranges that confer anti-microbial effectiveness without compromising protein stability. For example, three preservatives were successfully screened in the development of a liquid formulation for interleukin-1 receptor (Type I), using differential scanning calorimetry (DSC). The preservatives were rank ordered based on their impact on stability at concentrations commonly used in marketed products (Remmele RL Jr., et al., Pharm Res., 15(2): 200-8 (1998)).

Development of liquid formulations containing preservatives are more challenging than lyophilized formulations. Freeze-dried products can be lyophilized without the preservative and reconstituted with a preservative containing diluent at the time of use. This shortens the time for which a preservative is in contact with the protein significantly minimizing the associated stability risks. With liquid formulations, preservative effectiveness and stability have to be maintained over the entire product shelf-life (about 18-24 months). An important point to note is that preservative effectiveness has to be demonstrated in the final formulation containing the active drug and all excipient components.

Some preservatives can cause injection site reactions, which is another factor that needs consideration when choosing a preservative. In clinical trials that focused on the evaluation of preservatives and buffers in Norditropin, pain perception was observed to be lower in formulations containing phenol and benzyl alcohol as compared to a formulation containing m-cresol (Kappelgaard A.M., Horm Res. 62 Suppl 3:98-103 (2004)). Interestingly, among the commonly used preservative, benzyl alcohol possesses anesthetic properties (Minogue SC, and Sun DA., AnesthAnalg., 100(3): 683-6 (2005)). In various aspects the use of preservatives provide a benefit that outweighs any side effects.

### k. Methods of Preparation of Pharmaceutical Formulations

The present invention further contemplates methods for the preparation of pharmaceutical formulations.

The present methods further comprise one or more of the following steps: adding a stabilizing agent as described herein to the mixture prior to lyophilizing, adding at least one agent selected from a bulking agent, an osmolality regulating agent, and a surfactant, each of which as described herein, to the mixture prior to lyophilization.

The standard reconstitution practice for lyophilized material is to add back a volume of pure water or sterile water for injection (WFI) (typically equivalent to the volume removed during lyophilization), although dilute solutions of antibacterial agents are sometimes used in the production of pharmaceuticals for parenteral administration (Chen, Drug Development and Industrial Pharmacy, 18:1311-1354 (1992)). Accordingly, methods are provided for preparation of reconstituted rVWF compositions comprising the step of adding a diluent to a lyophilized rVWF composition of the invention.

The lyophilized material may be reconstituted as an aqueous solution. A variety of aqueous carriers, *e**.**g.*, sterile water for injection, water with preservatives for multi dose use, or water with appropriate amounts of surfactants (for example, an aqueous suspension that contains the active compound in admixture with excipients suitable for the manufacture of aqueous suspensions). In various aspects, such excipients are suspending agents, for example and without limitation, sodium carboxymethylcellulose, methylcellulose, hydroxypropylmethylcellulose, sodium alginate, polyvinylpyrrolidone, gum tragacanth and gum acacia; dispersing or wetting agents are a naturally-occurring phosphatide, for example and without limitation, lecithin, or condensation products of an alkylene oxide with fatty acids, for example and without limitation, polyoxyethylene stearate, or condensation products of ethylene oxide with long chain aliphatic alcohols, for example and without limitation, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example and without limitation, polyethylene sorbitan monooleate. In various aspects, the aqueous suspensions also contain one or more preservatives, for example and without limitation, ethyl, or n-propyl, p-hydroxybenzoate.

### l. Exemplary rVWF Formulation for Administration

In some embodiments, the present methods provide for an enhanced formulation that allows a final product with high potency (high rVWF concentration and enhanced long term stability) in order to reduce the volume for the treatment (100 IU/ml to 10000 IU/ml). In some embodiments, the rVWF concentration in the formulation for administration is about 100 IU/ml to 10000 IU/ml. In some embodiments, the rVWF concentration in the formulation for administration is about 500 IU/ml to 10000 IU/ml. In some embodiments, the rVWF concentration in the formulation for administration is about 1000 IU/ml to 10000 IU/ml. In some embodiments, the rVWF concentration in the formulation for administration is about 2000 IU/ml to 10000 IU/ml. In some embodiments, the rVWF concentration in the formulation for administration is about 3000 IU/ml to 10000 IU/ml. In some embodiments, the rVWF concentration in the formulation for administration is about 4000 IU/ml to 10000 IU/ml. In some embodiments, the rVWF concentration in the formulation for administration is about 5000 IU/ml to 10000 IU/ml. In some embodiments, the rVWF concentration in the formulation for administration is about 6000 IU/ml to 10000 IU/ml. In some embodiments, the rVWF concentration in the formulation for administration is about 7000 IU/ml to 10000 IU/ml. In some embodiments, the rVWF concentration in the formulation for administration is about 8000 IU/ml to 10000 IU/ml. In some embodiments, the rVWF concentration in the formulation for administration is about 9000 IU/ml to 10000 IU/ml.

In some embodiments, the formulation for administration comprises one or more zwitterionic compounds, including for example, amino acids like histidine, glycine, arginine. In some embodiments, the formulation for administration comprises a component with amphipathic characteristic having a minimum of one hydrophobic and one hydrophilic group, including for example polysorbate 80, octylpyranosid, dipeptides, and/or amphipathic peptides. In some embodiments, the formulation for administration comprises a non-reducing sugar or sugar alcohol or disaccharides, including for example, sorbitol, mannitol, sucrose, or trehalose. In some embodiments, the formulation for administration comprises a nontoxic water-soluble salt, including for example, sodium chloride, that results in a physiological osmolality. In some embodiments, the formulation for administration comprises a pH in a range from 6.0 to 8.0. In some embodiments, the formulation for administration comprises a pH of about 6.0, about 6.5, about 7, about 7.5 or about 8.0. In some embodiments, the formulation for administration comprises one or more bivalent cations that stabilize rVWF, including for example, Ca2+, Mg2+, Zn2+, Mn2+ and/or combinations thereof. In some embodiments, the formulation for administration comprises about 1 mM to about 50 mM glycine, about 1 mM to about 50 mM histidine, about zero to about 300 mM sodium chloride (e.g., less than 300 mM sodium), about 0.01 % to about 0.05% polysorbate 20 (or polysorbate 80), and about 0.5 % to about 20% (w/w) sucrose with a pH of about 7.0 and having a physiological osmolarity at the time point of administration.

In specific aspects, the rVWF is contained in a formulation containing a buffer, a sugar and/or a sugar alcohol (including without limitation trehalose and mannitol), a stabilizer (such as glycine), and a surfactant (such as polysorbate 80). In further embodiments, for formulations containing rFVIII, the formulation may further include sodium, histidine, calcium, and glutathione.

In some embodiments, the formulation for administration can be freeze dried. In some embodiments, the formulation for administration is stable and can be stored in liquid state at about 2°C to about 8°C, as well as at about 18°C to about 25°C. In some embodiments, the formulation for administration is stable and can be stored in liquid state at about 2°C to about 8°C. In some embodiments, the formulation for administration is stable and can be stored in liquid state at about 18°C to about 25°C.

To administer VWF compositions to human or test animals, in one aspect, the compositions comprises one or more pharmaceutically acceptable carriers. The phrases "pharmaceutically" or "pharmacologically" acceptable refer to molecular entities and compositions that are stable, inhibit protein degradation such as aggregation and cleavage products, and in addition do not produce allergic, or other adverse reactions when administered using routes well-known in the art, as described below. "Pharmaceutically acceptable carriers" include any and all clinically useful solvents, dispersion media, coatings, antibacterial and antifungal agents, isotonic and absorption delaying agents and the like, including those agents disclosed above.

The pharmaceutical formulations are administered orally, topically, transdermally, parenterally, by inhalation spray, vaginally, rectally, or by intracranial injection. The term parenteral as used herein includes subcutaneous injections, intravenous, intramuscular, intracisternal injection, or infusion techniques. Administration by intravenous, intradermal, intramuscular, intramammary, intraperitoneal, intrathecal, retrobulbar, and/or intrapulmonary injection at a particular site is contemplated as well. Generally, compositions are essentially free of pyrogens, as well as other impurities that could be harmful to the recipient.

Single or multiple administrations of rVWF are carried out with the dose levels and pattern being selected by the treating physician. For the prevention or treatment of disease, the appropriate dosage depends on the type of disease to be treated (*e.g.*, von Willebrand disease), the severity and course of the disease, whether drug is administered for preventive or therapeutic purposes, previous therapy, the patient's clinical history and response to the drug, and the discretion of the attending physician.

In one aspect, formulations are administered by an initial bolus followed by a continuous infusion to maintain therapeutic circulating levels of drug product. As another example, the inventive compound is administered as a one-time dose. Those of ordinary skill in the art will readily optimize effective dosages and administration regimens as determined by good medical practice and the clinical condition of the individual patient. The route of administration can be, but is not limited to, by intravenous, intraperitoneal, subcutaneous, or intramuscular administration. The frequency of dosing depends on the pharmacokinetic parameters of the agents and the route of administration. The optimal pharmaceutical formulation is determined by one skilled in the art depending upon the route of administration and desired dosage. See for example, Remington's Pharmaceutical Sciences, 18th Ed., 1990, Mack Publishing Co., Easton, Pa. 18042 pages 1435-1712, the disclosure of which is hereby incorporated by reference in its entirety for all purposes and in particular for all teachings related to formulations, routes of administration and dosages for pharmaceutical products. Such formulations influence the physical state, stability, rate of in vivo release, and rate of in vivo clearance of the administered agents. Depending on the route of administration, a suitable dose is calculated according to body weight, body surface area or organ size. Appropriate dosages may be ascertained through use of established assays for determining blood level dosages in conjunction with appropriate dose-response data. The final dosage regimen is determined by the attending physician, considering various factors which modify the action of drugs, e.g. the drug's specific activity, the severity of the damage and the responsiveness of the patient, the age, condition, body weight, sex and diet of the patient, the severity of any infection, time of administration and other clinical factors.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, Highly stabilized York, Gait, "Oligonucleotide Synthesis: A Practical Approach" 1984, IRL Press, London, Nelson and Cox (2000), Lehninger, Principles of Biochemistry 3rd Ed., W. H. Freeman Pub., Highly stabilized York, N.Y. and Berg et al. (2002) Biochemistry, 5th Ed., W. H. Freeman Pub., Highly stabilized York, N.Y., all of which are herein incorporated in their entirety by reference for all purposes.

Note that as used herein and in the appended claims, the singular forms "a," "an," and "the" include plural referents unless the context clearly dictates otherwise. Thus, for example, reference to "a polymerase" refers to one agent or mixtures of such agents, and reference to "the method" includes reference to equivalent steps and methods known to those skilled in the art, and so forth.

Unless defined otherwise, all technical and scientific terms used herein have the same meaning as commonly understood by one of ordinary skill in the art to which this invention belongs. All publications mentioned herein are incorporated herein by reference for the purpose of describing and disclosing devices, compositions, formulations and methodologies which are described in the publication and which might be used in connection with the presently described invention.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

In the above description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

Although the present invention is described primarily with reference to specific embodiments, it is also envisioned that other embodiments will become apparent to those skilled in the art upon reading the present disclosure, and it is intended that such embodiments be contained within the present inventive method.

### EXAMPLES

### EXAMPLE 1 - MANAGEMENT OF MENORRHAGIA IN A PHASE 3 OPEN-LABEL STUDY OF RECOMBINANT VON WILLEBRAND FACTOR (RVWF) IN PATIENTS WITH SEVERE VON WILLEBRAND DISEASE (VWD): A POST HOC ANALYSIS

### INTRODUCTION

The most common symptom in women with von Willebrand disease (VWD) is heavy menstrual bleeding (HMB) or menorrhagia, which affects up to 80% of women with VWD, with 20% of women with moderate or severe VWD requiring hysterectomy predominantly because of HMB.^{1,2}

Menorrhagia/HMB is commonly associated with symptomatic iron deficiency anemia, psychological stress, and reduced quality of life. It can adversely affect work, school, and daily activities, and result in increased health care costs.^{3,4}

Lack of diagnosis and effective treatment for menorrhagia is an unmet health need in women with VWD.³

Recombinant von Willebrand factor (rVWF, vonicog alfa, VEYVONDI^{™}; Baxalta Innovations GmbH, a Takeda company, Vienna, Austria) has demonstrated hemostatic efficacy and a positive benefit-risk profile when used for the on-demand treatment of bleeding episodes (BEs) in adult patients with severe VWD.^{5,6} However, limited information exists specifically on the management of menorrhagia with rVWF.

### OBJECTIVES

The goal of the study was to determine the efficacy and safety of on-demand rVWF treatment for menorrhagia episodes in patients with severe VWD.

### METHODS

A phase 3, multicenter, part-randomized study (NCT01410227; EudraCT 2010-024108-84) was conducted to assess the pharmacokinetics, safety, and hemostatic efficacy of rVWF in the treatment of BEs in adults with severe VWD.⁶

Inclusion criteria of the study included the following factors: male or female; 18-65 years of age; diagnosis of either type 3 VWD (VWF antigen ≤3 IU/dL); or severe non-type 3 VWD of the following types:
- Type 1 or type 2A (VWF ristocetin cofactor activity [VWF:RCo] <20 IU/dL);
- Type 2B (as diagnosed by genotype);
- Type 2N (factor VIII [FVIII] activity [FVIII:C] <10% and historically documented genetics);
- Type 2M;
and receipt of VWF concentrate to treat ≥1 BE in the past 12 months.

Exclusion criteria included history of hypersensitivity to any components of rVWF or rFVIII; history of VWF or FVIII inhibitors; immunologic disorders; or thromboembolic events.

### Study design

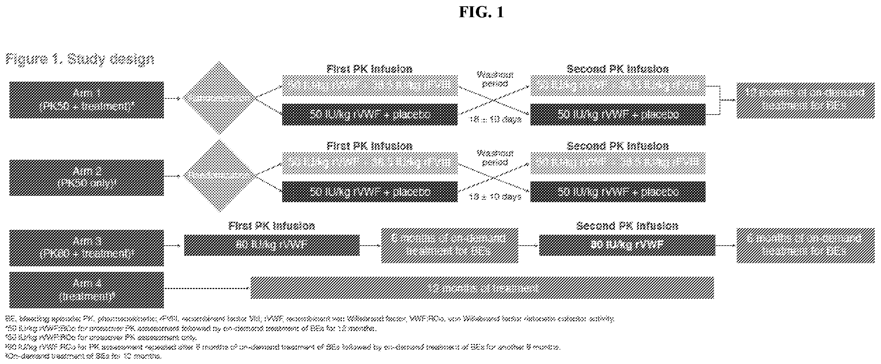

Patients were enrolled into 1 of 4 treatment cohorts to receive rVWF with or without recombinant FVIII (rFVIII; antihemophilic factor [recombinant] [ADVATE^{®}; Baxalta US Inc., a Takeda company, Lexington, MA, USA]) for pharmacokinetic assessment and/or treatment of BEs (Figure 1).⁶

### Treatments

In general, the initial dose (also referred to as the first dose) aimed for VWF full replacement with levels of VWF:RCo >0.6 IU/mL (60%) and of FVIII:C >0.4 IU/mL (40%).

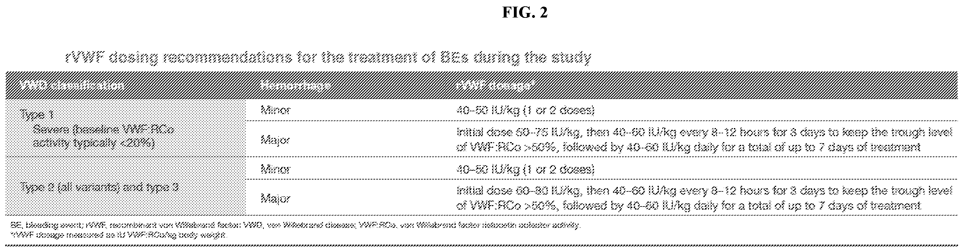

In major BEs, subsequent doses aimed to keep the trough level of VWF:RCo >50% for 3 days and then, as deemed necessary by the investigator, on subsequent days (Figure 2).

In moderate BEs, the aim was a VWF:RCo trough level >30% for as long as deemed necessary by the investigator. Treatment for minor BEs mostly consisted of 1 or 2 doses only (Figure 2).

### Post hoc data analyses of menorrhagia episodes

Post hoc data analyses were conducted on a subset of female patients treated with rVWF during the 12-month study period who reported ≥1 episode of menorrhagia. Menorrhagia episodes treated with rVWF only were included in the analyses.

Assessments of menorrhagia as BEs by the investigator included:
- Number and severity of BEs (minor, moderate, or severe)
- Total dose of rVWF and number of infusions per BE
- Time to resolution of each BE was taken from the date and time of the start of the first infusion and the date and time of BE resolution; only BEs for which the date and time were available were included in the analyses.

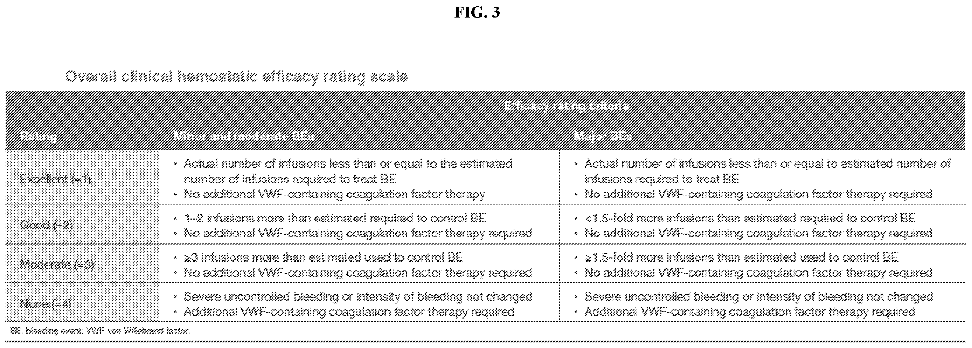

After resolution of the BE, overall clinical efficacy was determined by the investigator on the basis of the objective rating scale (Figure 3).

The following information was recorded by the patient, the patient's health care provider (for treatments away from the primary investigative site), the patient's legal representative (for home treatment), or by authorized, qualified personnel at the participating site (for hospital-based treatment):
- Location of BE (*e.g.,* joint, soft tissue, muscle); type of BE (*e.g.,* spontaneous, traumatic, unknown); and severity of BE (*e.g.*, minor, moderate, major)
- Dates and times of onset and resolution of BE
- Date and time of each infusion of rVWF-rFVIII or rVWF used to treat a BE
- Date and time of subjectively assessed hemostatic efficacy
- Type and number of analgesics required.

BEs were rated subjectively by the patient within 8 hours after the first infusion; the last available assessment within 8 hours after infusion was used in the analyses. For BEs treated with multiple infusions, the hemostatic efficacy rating of the first infusion was used in the analyses.

Concomitant medications taken for menorrhagia and menorrhagia-related anemia during the study were recorded.

Safety assessments included number, type, seriousness, and severity of adverse events (AEs) and causal relationship between AEs and study treatment.

Categorical variables were reported as n (%) and continuous variables were reported as median (range).

### RESULTS

### Patients

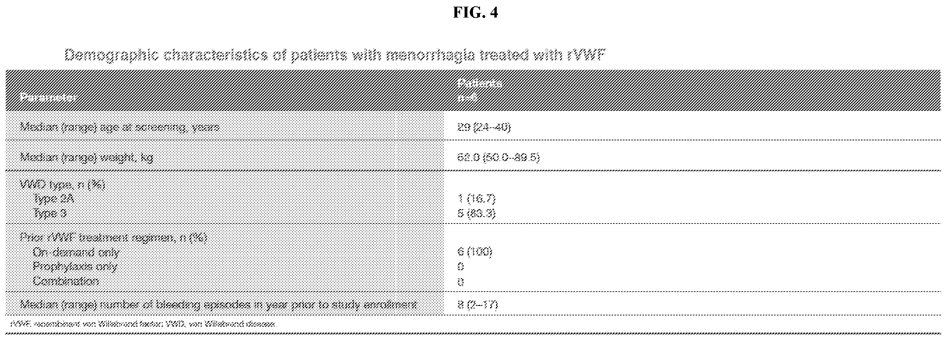

6 patients had 46 menorrhagia episodes (2-15 BEs per patient) that were treated with rVWF (Figure 4), 45 of which were treated with rVWF only (*e.g.*, not plasma-derived VWF) and were included in these analyses.

1 patient had 2 menorrhagia episodes, 1 of which was treated with both plasma-derived VWF and rVWF and, therefore, was excluded from the analysis.

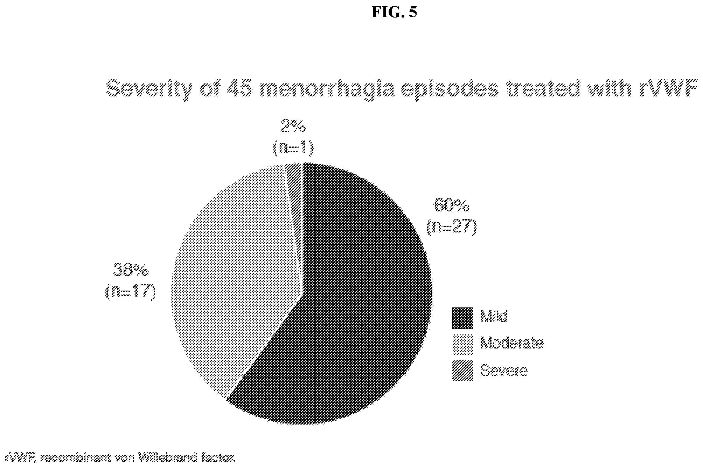

The majority of menorrhagia episodes were rated by the physicians or patients (home treatment) as mild (Figure 5).

### Factor consumption for menorrhagia and treatment outcomes

The rVWF dose required to control menorrhagia episodes increased with bleed severity and was higher in VWD type 3 than type 2A (Figure 6).

The number of rVWF infusions used to treat each menorrhagia episode generally increased with bleed severity (Figure 6), with 30 of 45 menorrhagia episodes requiring only 1 infusion.

For menorrhagia episodes required more than 1 infusion. rFVIII was co-administered in 12 of 15 (80%) first infusions, 4 of 15 (26.7%) second infusions, 1 of 6 (16.7%) third infusions, and 0 of 1 fourth infusions.

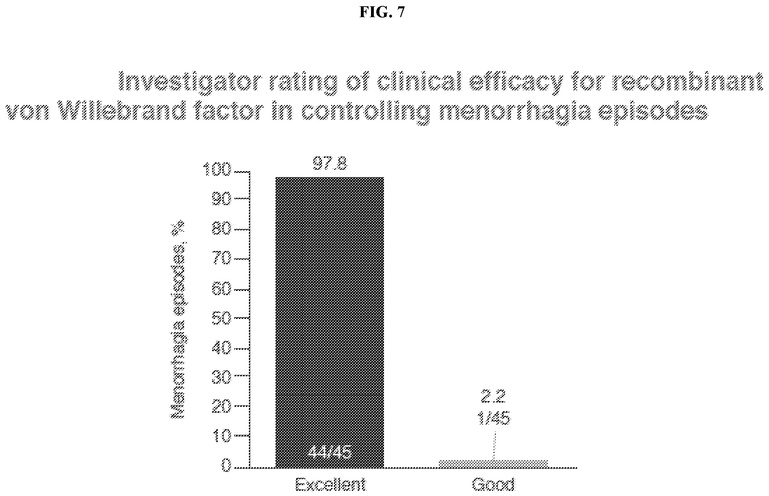

The median (range) time from the start of the first infusion to resolution of menorrhagia episodes (n=43) was 24.3 (0.3-202.7) hours. The hemostatic efficacy of rVWF for the treatment of menorrhagia episodes was mostly rated by the investigators as "excellent" (Figure 7). Patients rated the hemostatic efficacy of rVWF as "excellent" in 39 (90.7%) menorrhagia episodes, "good" in 3 (7.0%) episodes, and "moderate" in 1 (2.3%) episode.

### Concomitant medications and safety of study treatment

3 patients used tranexamic acid and 3 patients used oral contraceptives as concomitant treatments for menorrhagia.

All 6 patients had iron replacement therapy to treat anemia resulting from menorrhagia.

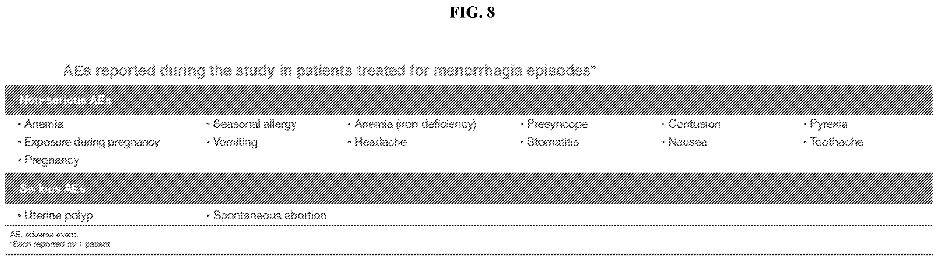

4 out of 6 patients reported a total number of 15 AEs (Figure 8).
- All AEs were considered unrelated to study treatment by the investigator.
- 1 AE of a uterine polyp was considered serious and severe.
- 1 AE of a spontaneous abortion was considered serious but mild in severity.
- 13 AEs were considered non-serious: 12 were mild and 1 was moderate in severity.

### Caveats

The post hoc analyses of data from this phase 3, part-randomized rVWF study involved a small number of patients with severe VWD treated for menorrhagia/HMB episodes in a 12-month study.

### CONCLUSIONS

A median of 1 infusion per HMB (heavy menstrual bleeding or menorrhagia) episode was required, and patients and investigators rated the clinical efficacy of treatment with rVWF as "excellent" for managing the majority of HMB episodes.

This analysis provides evidence showing the efficacy and safety of rVWF on an on-demand basis for the management of HMB episodes in women with severe VWD.

Further studies will be performed to confirm if rVWF treatment regimens may help manage consistently this debilitating condition in women with severe VWD.

### REFERENCES

1. Nichols WL, et al. Haemophilia 2008;14:171-232.
2. De Wee EM, et al. Thromb Haemost 2011;106:885-92.
3. Ragni MV, et al. Haemophilia 2016P2:397 -402.
4. Kadir RA, et al. Haemophilia 2010;16:832-9.
5. European Medicines Agency. VEYVONDI. Summary of Product Characteristics. https://www.ema.europa.eu/en/documents/product-information/veyvondi-epar-product-information_en.pdf. Accessed December 18, 2019.
6. Gill JC, et al. Blood 2015;126:2038-46.

### Items:

1. A method for treating a menorrhagia bleeding episode in a subject with severe von Willebrand Disease (VWD) comprising administering to the subject at least one dose of recombinant von Willebrand Factor (rVWF) ranging from about 30 IU/kg to about 90 IU/kg.
2. The method of treatment of item 1, wherein the menorrhagia bleeding episode is selected from the group consisting of a minor menorrhagia bleeding episode and a major menorrhagia bleeding episode.
3. The method of treatment of item 1 or 2, wherein the VWD is selected from a group consisting of Type 1 VWD, Type 2 VWD, and Type 3 VWD.
4. The method of treatment of any one of items 1 to 3, wherein a first dose of 30-60 IU/kg rVWF is administered to the subject, and the menorrhagia bleeding episode is a minor menorrhagia bleeding episode.
5. The method of treatment of item 4, further comprising administering to the subject a second dose of 30-60 IU/kg rVWF.
6. The method of treatment of any one of items 1 to 3, wherein a first dose of 40-50 IU/kg rVWF is administered to the subject, and the menorrhagia bleeding episode is a minor menorrhagia bleeding episode.
7. The method of treatment of item 6, further comprising administering to the subject a second dose of 40-50 IU/kg rVWF.
8. The method of treatment of any one of items 1 to 7, wherein the VWD is Type 1 VWD.
9. The method of treatment of any one of items 1 to 7, wherein the VWD is selected from the group consisting of Type 2A, Type 2B, Type 2N, Type 2M VWD, and Type 3 VWD.
10. The method of treatment of any one of items 1 to 3, wherein a first dose of 40-90 IU/kg rVWF is administered to the subject, and the menorrhagia bleeding episode is a major menorrhagia bleeding episode.
11. The method of treatment of item 10, wherein the VWD is Type 1 VWD.
12. The method of treatment of item 10, wherein the VWD is selected from the group consisting of Type 2A VWD, Type 2B VWD, Type 2N VWD, Type 2M VWD, and Type 3 VWD.
13. The method of treatment of any one of items 10 to 12, further comprising administering to the subject a plurality of doses of rVWF.
14. The method of treatment of any one of items 10 to 13, further comprising administering to the subject a plurality of doses comprising the first dose of 40-90 IU/kg rVWF and a plurality of second doses, wherein each of the second doses is administered every 8 to 12 hours for about 3 days.
15. The method of treatment of any one of items 10 to 13, further comprising administering to the subject a plurality of doses comprising the first dose of 40-90 IU/kg rVWF and a plurality of second doses, wherein each of the second doses is 40-60 IU/kg rVWF and administered every 8 to 12 hours for about 3 days.
16. The method of treatment of any one of items 10 to 13, further comprising administering to the subject a plurality of doses comprising the first dose of 40-90 IU/kg rVWF and a second dose of 40-60 IU/kg rVWF.
17. The method of treatment of any one of items 10 to 16, further comprising administering to the subject a third dose of 30-70 IU/kg rVWF daily for the remaining duration of the bleeding episode.
18. The method of treatment of any one of items 10 to 17, wherein the total duration of the method is no more than about 7 days.
19. The method of treatment of any one of items 1 to 3, wherein a first dose of rVWF administered to the subject is 50-80 IU/kg rVWF, and the menorrhagia bleeding episode is a major menorrhagia bleeding episode.
20. The method of treatment of item 19, wherein the VWD is Type 1 VWD.
21. The method of treatment of item 19 or 20, further comprising administering to the subject a plurality of doses of rVWF.
22. The method of treatment of any one of items 19 to 21, further comprising administering to the subject a plurality of doses comprising the first dose of 50-80 IU/kg rVWF and a plurality of second doses, wherein each of the second doses is administered every 8 to 12 hours for about 3 days.
23. The method of treatment of any one of items 19 to 21, further comprising administering to the subject a plurality of doses comprising the first dose of 50-80 IU/kg rVWF and a plurality of second doses, wherein each of the second doses is 40-60 IU/kg rVWF and administered every 8 to 12 hours for about 3 days.
24. The method of treatment of any one of items 19 to 21, further comprising administering to the subject a plurality of doses comprising a first dose of 50-80 IU/kg rVWF and a second dose of 40-60 IU/kg rVWF.
25. The method of treatment of any one of items 19 to 24, further comprising administering to the subject a third dose of 30-70 IU/kg rVWF daily for the remaining duration of the bleeding episode.
26. The method of treatment of any one of items 19 to 25, wherein the total duration of the method is no more than about 7 days.
27. The method of treatment of item 19, wherein the first dose of rVWF administered to the subject is 60-80 IU/kg, and the VWD is selected from the group consisting of Type 2A VWD, Type 2B VWD, Type 2N VWD, Type 2M VWD, and Type 3 VWD.
28. The method of treatment of item 19 or 27, further comprising administering to the subject a plurality of doses of rVWF.
29. The method of treatment of any one of items 19, 27 and 28, further comprising administering to the subject a plurality of doses comprising the first dose of 60-80 IU/kg rVWF and a plurality of second doses, wherein each of the second doses is administered every 8 to 12 hours for about 3 days.
30. The method of treatment of any one of items 19, 27 and 28, further comprising administering to the subject a plurality of doses comprising the first dose of 60-80 IU/kg rVWF and a plurality of second doses, wherein each of the second doses is 40-80 IU/kg rVWF and administered every 8 to 12 hours for about 3 days.
31. The method of treatment of any one of items 19, 27 and 28, further comprising administering to the subject a plurality of doses comprising a first dose of 60-80 IU/kg rVWF and a second dose of 40-80 IU/kg rVWF.
32. The method of treatment of any one of items 19 to 31, further comprising administering to the subject a third dose of 40-60 IU/kg rVWF daily for the remaining duration of the bleeding episode.
33. The method of treatment of any one of items 19 to 32, wherein the total duration of the method is no more than about 7 days.
34. The method of treatment of any one of items 1 to 33, further comprising administering recombinant Factor VIII (rFVIII) to the subject.
35. The method of treatment of item 34, wherein the rFVIII is administered concomitantly or sequentially with one or more doses of rVWF.
36. The method of treatment of item 34 or 35, wherein the rFVIII is administered concomitantly or sequentially with the first dose and/or the second dose of rVWF.
37. The method of treatment of any one of items 34 to 36, wherein the rFVIII is administered concomitantly or sequentially with each dose of rVWF.
38. The method of treatment of any one of items 1 to 37, wherein the rFVIII is administered to the subject at a dose of about 20 IU/kg to about 50 IU/kg.
39. The method of treatment of any one of items 1 to 38, wherein the rFVIII is administered to the subject such that said rVWF to FVIII ratio is selected from the group consisting of about 1.5:0.8; about 1.3:1; about 1.1:0.8; about 1.5:1; and about 1.1:1.2.
40. A method for treating a major menorrhagia bleeding episode in a subject with severe Type 1 von Willebrand Disease (VWD) comprising:
   a) administering a first dose of 50-75 IU/kg rVWF to the subject;
   b) administering a plurality of second doses of 40-60 IU/kg rVWF to the subject, wherein each of the doses is administered every 8-12 hours for 3 days such that a trough level of VWF:RCo is maintained at at least 0.30 IU/mL or a trough level of VWF:GPIbM or VWF:GPIbR is maintained that is equivalent to the trough level of VWF:RCo of at least 0.30 IU/mL; and
   c) administering a third dose of 40-60 IU/kg rVWF to the subject once a day for the remaining duration of the bleeding episode;
   wherein the total duration of steps (a)-(c) is no more than about 7 days.
41. The method of treatment of item 40, wherein the trough level of VWF:RCo is maintained at at least 0.40 IU/mL or the trough level of VWF:GPIbM or VWF:GPIbR is maintained that is equivalent to the trough level of VWF:RCo of at least 0.40 IU/mL.
42. The method of treatment of item 40 or 41, wherein the trough level of VWF:RCo is maintained at at least 0.50 IU/mL or the trough level of VWF:GPIbM or VWF:GPIbR is maintained that is equivalent to the trough level of VWF:RCo of at least 0.50 IU/mL.
43. The method of treatment of any one of items 40 to 42, wherein the total duration of steps (a)-(c) is no more than 6 days.
44. The method of treatment of any one of items 40 to 42, wherein the total duration of steps (a)-(c) is no more than 5 days.
45. The method of treatment of any one of items 40 to 42, wherein the total duration of steps (a)-(c) is no more than 4 days.
46. A method for treating a major menorrhagia bleeding episode in a subject with severe Type 2 or Type 3 von Willebrand Disease (VWD) comprising:
   a) administering a first dose of 60-80 IU/kg rVWF to the subject;
   b) administering a plurality of second doses of 40-60 IU/kg rVWF to the subject, wherein each of the doses is administered every 8-12 hours for 3 days such that a trough level of VWF:RCo is maintained at at least 0.30 IU/mL or a trough level of VWF:GPIbM or VWF:GPIbR is maintained that is equivalent to the trough level of VWF:RCo of at least 0.30 IU/mL; and
   c) administering a third dose of 40-60 IU/kg rVWF to the subject once a day for the remaining duration of the bleeding episode;
   wherein the total duration of steps (a)-(c) is no more than about 7 days.
47. The method of treatment of item 46, wherein the trough level of VWF:RCo is maintained at at least 0.40 IU/mL or the trough level of VWF:GPIbM or VWF:GPIbR that is equivalent to the trough level of VWF:RCo of at least 0.40 IU/mL is maintained.
48. The method of treatment of item 46 or 47, wherein the trough level of VWF:RCo is maintained at at least 0.50 IU/mL or the trough level of VWF:GPIbM or VWF:GPIbR is maintained that is equivalent to the trough level of VWF:RCo of at least 0.50 IU/mL.
49. The method of treatment of any one of items 46 to 48, wherein the total duration of steps (a)-(c) is no more than 6 days.
50. The method of treatment of any one of items 46 to 48, wherein the total duration of steps (a)-(c) is no more than 5 days.
51. The method of treatment of any one of items 46 to 48, wherein the total duration of steps (a)-(c) is no more than 4 days.
52. The method of treatment of any one of items 46 to 51, further comprising administering recombinant Factor VIII (rFVIII) to the subject.
53. The method of treatment of item 52, wherein the rFVIII is administered concomitantly or sequentially with the at least one dose of rVWF.
54. The method of treatment of any one of items 52 to 53, wherein the rFVIII is administered concomitantly or sequentially with the first dose and/or the plurality of second doses of rVWF.
55. The method of treatment of any one of items 52 to 54, wherein the rFVIII is administered concomitantly or sequentially with both the first dose and the plurality of second doses of rVWF.
56. The method of treatment of any one of items 52 to 55, wherein the dose of rFVIII administered to v subject is about 20 IU/kg to about 50 IU/kg.
57. The method of treatment of any one of items 52 to 56, wherein the rFVIII administered to the subject is at a rVWF to FVIII ratio selected from the group consisting of about 1.5:0.8; about 1.3:1; about 1.1:0.8; about 1.5:1; and about 1.1:1.2.
58. The method of treatment of any one of items 1 to 57, wherein the rVWF has a specific activity of between about 20 mU/µg to about 150 mU/µg.
59. The method of treatment of any one of items 1 to 58, wherein the rFVIII and rVWF administered to the subject has a rFVIII procoagulant activity (IU rFVIII:C) to rVWF Ristocetin cofactor activity (IU rVWF:RCo) ratio of between about 3:1 and about 1:5.
60. A method for treating a menorrhagia bleeding episode in a subject with severe von Willebrand Disease (VWD) comprising administering to the subject at least one dose of recombinant von Willebrand Factor (rVWF) ranging from about 30 IU/kg to about 90 IU/kg, wherein the duration and/or the severity of the bleeding episode suffered by the subject is reduced compared to the duration and/or the severity of the bleeding episode suffered by the subject when the rVWF is not administered.
61. The method of treating of any one of items 1 to 60, wherein one dose of rVWF is administered over the duration of the bleeding episode.
62. The method of treating of any one of items 1 to 61, wherein two doses of rVWF are administered over the duration of the bleeding episode.
63. The method of treating of any one of items 1 to 62, wherein three doses of rVWF are administered over the duration of the bleeding episode.
64. The method of treating of any one of items 1 to 63, wherein four doses of rVWF are administered over the duration of the bleeding episode.
65. The method of treating of any one of items 1 to 64, wherein five doses or more of rVWF are administered over the duration of the bleeding episode.
66. The method of treating of any one of items 1 to 65, wherein the rVWF administered to the subject over the duration of the bleeding episode is at a range of about 20 IU/kg to about 200 IU/kg rVWF.
67. The method of treating of any one of items 1 to 66, wherein the rVWF administered to the subject over the duration of the bleeding episode is at a range of about 20 IU/kg to about 100 IU/kg rVWF
68. The method of treatment of any one of items 1 to 67, wherein the rVWF has an amino acid sequence having at least 80%, 85%, 90%, 95%, 96%, 97%, 98%, 99%, or 100% identity to the sequence of SEQ ID NO:3.
69. The method of treatment of any one of items 1 to 68, wherein the rVWF is a fragment of an rVWF, wherein the rVWF has an amino acid sequence having at least 80%, 85%, 90%, 95%, 96%, 97%, 98%, 99%, or 100% identity to the sequence of SEQ ID NO:3.
70. The method of treatment of any one of items 1 to 69, wherein the rVWF is chemically modified using one or more techniques from the group consisting of ubiquitination, glycosylation, conjugation to therapeutic or diagnostic agents, labeling, covalent polymer attachment, introduction of non-hydrolyzable bonds, and insertion or substitution by chemical synthesis of one or more amino acids.

## Claims

1. Recombinant von Willebrand Factor (rVWF) for use in a method for treating a major or severe menorrhagia bleeding episode in a subject with severe Type 1 von Willebrand Disease (VWD) comprising:
a) administering a first dose of 50-75 IU/kg rVWF to the subject;
b) administering a plurality of second doses of 40-60 IU/kg rVWF to the subject, wherein each of the doses is administered every 8-12 hours for 3 days; and
c) administering a third dose of 40-60 IU/kg rVWF to the subject once a day for the remaining duration of the bleeding episode;
wherein the total duration of steps (a)-(c) is no more than about 7 days.

2. rVWF for use according to claim 1, wherein the trough level of VWF:RCo is maintained at at least 0.30 IU/mL or a trough level of VWF:GPIbM or VWF:GPIbR is maintained that is equivalent to the trough level of VWF:RCo of at least 0.30 IU/mL.

3. rVWF for use according to claim 1 or 2, wherein:
the trough level of VWF:RCo is maintained at at least 0.40 IU/mL or a trough level of VWF:GPIbM or VWF:GPIbR is maintained that is equivalent to the trough level of VWF:RCo of at least 0.40 IU/mL; and/or
the trough level of VWF:RCo is maintained at at least 0.50 IU/mL or a trough level of VWF:GPIbM or VWF:GPIbR is maintained that is equivalent to the trough level of VWF:RCo of at least 0.50 IU/mL.

4. rVWF for use according to any one of claims 1 to 3, wherein the total duration of steps (a)-(c) is no more than 6 days, no more than 5 days, or no more than 4 days.

5. rVWF for use according to any one of claims 1 to 4, wherein the rVWF has a specific activity of between about 20 mU/µg to about 150 mU/µg.

6. rVWF for use according to any one of claims 1 to 5, wherein:
one dose of rVWF is administered over the duration of the bleeding episode;
two doses of rVWF are administered over the duration of the bleeding episode;
three doses of rVWF are administered over the duration of the bleeding episode;
four doses of rVWF are administered over the duration of the bleeding episode; and/or
five doses or more of rVWF are administered over the duration of the bleeding episode.

7. rVWF for use according to any one of claims 1 to 6, wherein the rVWF administered to the subject over the duration of the bleeding episode is at a range of about 20 IU/kg to about 200 IU/kg rVWF.

8. rVWF for use according to any one of claims 1 to 7, wherein the rVWF administered to the subject over the duration of the bleeding episode is at a range of about 20 IU/kg to about 100 IU/kg rVWF

9. rVWF for use according to any one of claims 1 to 8, wherein the rVWF has an amino acid sequence having at least 80%, 85%, 90%, 95%, 96%, 97%, 98%, 99%, or 100% identity to the sequence of SEQ ID NO:3.

10. rVWF for use according to any one of claims 1 to 9, wherein the rVWF is a fragment of an rVWF, wherein the rVWF has an amino acid sequence having at least 80%, 85%, 90%, 95%, 96%, 97%, 98%, 99%, or 100% identity to the sequence of SEQ ID NO:3.

11. rVWF for use according to any one of claims 1 to 10, wherein the rVWF is chemically modified using one or more techniques from the group consisting of ubiquitination, glycosylation, conjugation to therapeutic or diagnostic agents, labeling, covalent polymer attachment, introduction of non-hydrolyzable bonds, and insertion or substitution by chemical synthesis of one or more amino acids.
